(19) Europäisches Patentamt
European Patent Office
Office européen des brevets

(11) EP 3 031 821 A1

# (12) EUROPEAN PATENT APPLICATION

(43) Date of publication:
**15.06.2016 Bulletin 2016/24**

(21) Application number: **14196847.9**

(22) Date of filing: **08.12.2014**

(51) Int Cl.:
***C07K 14/005*** *(2006.01)* ***C12N 7/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Life Science Inkubator**
**53175 Bonn (DE)**

(72) Inventors:
- **Franken, Sebastian**
**53175 Bonn (DE)**
- **Glassmann, Alexander**
**53175 Bonn (DE)**
- **Temme, Nadine**
**53175 Bonn (DE)**

(74) Representative: **von Renesse, Dorothea et al**
**König-Szynka-Tilmann-von Renesse**
**Patentanwälte Partnerschaft mbB**
**Mönchenwerther Str. 11**
**40545 Düsseldorf (DE)**

## (54) polyomavirus VLPs with a fusion protein

(57) The disclosure relates to a fusion protein comprising a VP1 binding protein and an exogenous peptide, wherein the exogenous peptide comprises a cargo-securing peptide (CSP) and/or an endosome translocating peptide (ETP) and to virus like particles (VLP) comprising the fusion protein for use as drug delivery system.

Also provided are polynucleotides encoding the fusion protein, suitable expression vectors, host cells, production methods for the fusion protein and the VLP comprising the fusion protein.

EP 3 031 821 A1

## Description

### FIELD OF THE INVENTION

The present invention relates to a fusion protein comprising a VP1 binding protein and an exogenous peptide, wherein the exogenous peptide comprises a cargo-securing peptide (CSP) and/or an endosomal translocating peptide (ETP), and to virus like particles (VLP) comprising the fusion protein for use as drug delivery system.

### BACKGROUND OF THE INVENTION

In the development of specific diagnostic or therapeutic procedures, the use of transfer systems (delivery system) that allow a possible cell-specific transfer of substances and nucleic acids such as, markers or agents, are of great importance. For this cell-specific transfer systems based on virus-like particles (VLP) have been developed. One of these systems is the VLP from the human polyomavirus John-Cunningham virus (JCV) as described in WO 97/19174 and EP 1270586 B1. Basis of this system is the ability of the VLP to package foreign cargo such as drugs or and nucleic acids instead of the viral DNA. As a VLP still has the ability to specifically recognize cells and to be internalized by the cells the VLP can be used to introduce a cargo of choice into specific cells. However, with the VLP systems established to date the yield of cargo entering the cells is not satisfactory.

In view of the prior art it is an object of the invention to provide a more efficient transfer system.

### SUMMARY OF THE INVENTION

According to a first aspect, the invention provides a fusion protein comprising a VP1 binding protein and an exogenous peptide, wherein the exogenous peptide comprises a cargo-securing peptide (CSP) and/or an endosomal translocating peptide (ETP).

The invention is *inter alia* based on the finding that the yield of cargo transfer provided by the VLP systems known in the art is reduced due to the degradation of the transported cargo by extracellular or intracellular factors, such as nucleases or proteases.

The degradation of the VLP cargo, however, can be reduced by a tighter packaging of the VLP capsids. The inventors have found that the addition of a cargo-securing peptide, in particular a cargo-binding peptide (CBP) to the VP2 protein leads to a tighter packaging of the particles and, thus, to a greater protection against degradation of the cargo.

Furthermore, the inventors have found that a large part of the VLPs entering a cell do not leave the endosomal pathway and are degraded in the endolysosome. Surprisingly, a fusion of a certain class of peptides to the VP2 or VP3 of a polyomavirus VLP leads to a destabilization of the endosomal membrane and, therefore, increases the number of VLPs released into the cytosol. This, in turn, increases the efficiency of the cargo transport. These peptides are referred to as endosomal translocating peptides (ETPs).

Thus, according to a second aspect of the invention, a VLP is provided which comprises a fusion protein according to the first aspect of the invention.

According to a third aspect of the invention, a pharmaceutical composition is provided which comprises at least one VLP according to the second aspect of the invention and at least one pharmaceutically acceptable carrier.

According to a fourth aspect of the invention, a polynucleotide is provided which comprises a nucleic acid sequence encoding a fusion protein according to the first aspect of the invention.

According to a fifth aspect of the invention, an expression vector is provided which comprises a polynucleotide according to the fourth aspect of the invention.

According to a sixth aspect of the invention, a host cell is provided which comprises the vector according to the fifth aspect of the invention.

Finally, according to a seventh aspect of the invention, a process of producing the VLP according to the second aspect is provided which comprises the steps of

a) introducing a polynucleotide according to the fourth aspect into a host cell;
b) culturing the transformed host cell in a medium under conditions leading to a protein expression with the nucleic acid as a template;
c) isolating the expression product; and
d) assembly of the expression product, optionally with further viral proteins in the VLP.

### BRIEF DESCRIPTION OF THE FIGURES

**Fig. 1** shows fluorescence microscopy images of COS7 cells treated with different VLPs. The fluorescence signal represents antibody labeled VLPs in the cells. The images from left to right correspond to COS7 cells containing VLPs formed by VP1, VP1 and VP2, VP1 and VP2-PENp (a VP2 penetratin fusion protein) and VP1 and VP2-HISp (His-tagged VP2).

**Fig. 2** shows fluorescence microscopy images of HeLa cells treated with different VLPs. The fluorescence signal represents antibody labeled VLPs in the cells. The images from left to right correspond to HeLa cells containing VLPs formed by VP1, VP1 and VP2-PENp and VP1 and VP2-HISp.

**Fig. 3** shows fluorescence microscopy images of TC670 cells treated with different VLPs. The fluorescence signal represents antibody labeled VLPs in the cells. The images from left to right correspond to top, middle and bottom slices of the same cells. The upper line of images are cells treated with VLPs formed by VP1VP2, and the lower line of images show cells treated with VLPs formed by VP1VP2-L2DD477p.

**Fig. 4** shows two diagrams (A and B) with the results of a DNA protection assay determined for different VLPs with and without cargo binding peptides according to the invention. The columns of the diagram represent a protection value in percent determined from the relation of molecule numbers quantified after DNAse incubation of DNA containing VLPs to those without a DNase treatment. In the figure "VP1" defines a VLP formed by VP1 only. "VP1_VP2" defines a VLP formed by VP1 and a wild type VP2, "VP1 VP3" defines a VLP formed by VP1 and a wild type VP3, "VP1/GFP-VP2" defines VLP formed by VP1 and VP1 with a VP2 fusion protein comprising a C-terminal protamine-1. The "(5:1)" and "(10:1)" define VP1-VP2-PENp stands for a VLP formed by VP1 and a VP2 fusion protein with a C-terminal penetratin peptide.

**Fig. 5** shows the result of a second DNA protection assay determined for different VLPs with and without cargo binding peptides according to the invention. In the figure "VP1" defines a VLP formed by VP1 only. "VP1-VP2-Prtm" defines a VLP formed by VP1 and a VP2 fusion protein with a C-terminal protamine-1 peptide, "VP1/VP1-VP2-Prtm" defines a mixture of pentamers formed by VP1 only and VP1 with a VP2 fusion protein comprising a C-terminal protamine-1 in the ratios 5:1 and 10:1. The columns of the diagram represent a protection value in percent determined from the relation of molecule numbers quantified after DNAse incubation of DNA containing VLPs to those without a DNAse treatment.

**Fig. 6** shows the result of a siRNA protection assay determined for different VLPs with and without cargo binding peptides according to the invention. In the diagram 1) "VP1" stands for a VLP formed by VP1 only, 2) "VP1-VP2" defines a VLP formed by VP1 and VP2, 3) "VP1-VP2-Prtm" stands for a VLP formed by VP1 and a VP2 fusion protein with a C-terminal protamine-1 peptide and 4) "VP1-VP2-PENp" stands for a VLP formed by VP1 and a VP2 fusion protein with a C-terminal penetratin peptide. The diagram gives a protection value in % determined from the relation of molecule numbers quantified after benzonase incubation of siRNA containing VLPs to those without a benzonase treatment.

**Fig. 7** shows the result of a transduction analysis of DNA transduction into COS7 cells using VLPs according to the invention. A plasmid comprising the luciferase gene is transfected into COS7 cells by different VLPs. The VLPs are formed by 1) VP1 (only VP1), 2) VP1_VP2-L2DD447p (VP1 and a VP2 fusion protein with a C-terminal HPV 33-L2-DD447 peptide), 3) VP1_VP2-TATp (VP1 and a VP2 fusion protein with a C-terminal TAT peptide), 4) VP1_VP2-PENp (VP1 and a VP2 fusion protein with a C-terminal pentratin peptide), 5) VP1_VP2-HISp (VP1 and a VP2 fusion protein with a His-tag). 6) Encapsulated Nanoluc vector without VLP carrier. The chemoluminescene signal generated by luciferase is measured for each transduction experiment and represented in relative light units (RLU).

**Fig. 8** shows the result of a transduction analysis of DNA transduction into TC620 cells using VLPs according to the invention. A plasmid comprising the luciferase gene is transfected into TC620 cells by different VLPs. The VLPs are formed by VP1 with VP2-HA, VP1 with VP2-Protamin, VP1-VP3-Protamin, a mixture of pentamers formed by VP1 only and VP1 with a VP2 fusion protein comprising a C-terminal protamine-1 in the ratios 5:1 or 10:1. The chemoluminescene signal generated by luciferase is measured for each transduction experiment and represented in relative light units (RLU).

**Fig. 9** shows the result of a siRNA protection test for a VLP formed by VP1_VP2coHA. VLPs containing Kif11_08 siRNA were incubated in blood plasma and the samples were taken over time. The number of siRNA molecules was determined in samples from the three time points and a control of siRNA incubated in blood plasma without VLP.

**Fig. 10** shows the results of a real-time cell adhesion assay with TC-620 cells transfected with different samples of the Kif11 08 siRNA which specifically interferes with cell division or control samples. The curves shown in the diagram relate cells treated with the following samples: 1) cell culture medium, 2) 5 nM Kif11 08 siRNA in cell culture medium, wherein Kif11 08 siRNA had been packaged into a VLP from VP1 and VP2-penetratin, treated with RNAse and extracted from the VLP after RNase treatment, 3) 5 nM Kif11 08 siRNA in cell culture medium. The X axis represents the time of the experiment in hours (h) and the Y-axis represents proliferation index.

**Fig. 11** shows the results of a real-time cell adhesion assay with TC-620 cells transduced with different samples of the Kif11 08 siRNA via VLP or control samples. The curves shown in the diagram relate cells treated with the following samples: 1) cell culture medium, 2) VLP delivery solution, 3) VLP control, 4) + 5) VP1-VP2-PENp VLP with Kif11_08 siRNA. The X axis represents the time of the experiment in hours (h) and the Y-axis represents proliferation index.

**Fig. 12** shows a 96-well plate of an exemplary of Hemagglutinin test. Wells with central dark spot represent a negative result, namely no agglutination, completely filled wells represent a positive agglutination result.

**Fig. 13** shows a transmission electron microscopy image of negatively stained VLPs.

## DETAILED DESCRIPTION OF THE INVENTION

### 1. Definitions

A "peptide" according to the present invention may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide according to the invention does not exceed a length of 500 amino acids, more preferably it does not exceed a length of 300 amino acids; even more preferably it is not longer than 250 amino acids. Thus, the term peptide includes oligopeptides, which usually refer to peptides with a length of 2 to 10 amino acids, and polypeptides which usually refer to peptides with a length of more than 10 amino acids. The term "protein" refers to a peptide with at least 60, at least 80, preferably at least 100 amino acids.

The term "fusion protein" according to the invention relates to proteins created through the joining of two or more genes that originally coded for separate proteins/peptides. The genes may be naturally occurring in the same organism or different organisms or may synthetic polynucleotides.

The term "exogenous" according to the invention relates to the property of a peptide or polynucleotide that it does not naturally occur in polyomaviruses.

The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et a/., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using thenobrief option) is used as the percent identity and is calculated as follows:

(Identical Residues x 100)/(Length of Alignment - Total Number of Gaps in Alignment)

For purposes of the present invention, the degree of sequence identity between two deoxyribonucleotide sequences is determined using the Needleman-Wunsch algorithm (Needleman and Wunsch, 1970, supra) as implemented in the Needle program of the EMBOSS package (EMBOSS: The European Molecular Biology Open Software Suite, Rice et a/., 2000, supra), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment - Total Number of Gaps in Alignment)

The term "isolated" means a substance in a form or environment which does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature.

The term "operably linked" means a configuration in which a control sequence is placed at an appropriate position relative to the coding sequence of a polynucleotide such that the control sequence directs the expression of the coding sequence.

The term "expression" includes any step involved in the production of a peptide including, but not limited to, transcription, post-transcriptional modification, translation, post-translational modification, and secretion.

The term "expression vector" means a linear or circular DNA molecule that comprises a polynucleotide encoding a peptide and is operably linked to additional nucleotides that provide for its expression.

The term "host cell" means any cell type that is susceptible to transformation, transfection, transduction, and the like, with a nucleic acid construct or expression vector comprising a polynucleotide of the present invention. The term "host cell" encompasses any progeny of a parent cell that is not identical to the parent cell due to mutations that occur during replication.

The term "comprise", as used herein, besides its literal meaning, also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The term "carrier" applied to pharmaceutical compositions of the invention refers to a diluent, excipient, or vehicle with which the VLP of the invention is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington The Science and Practice of Pharmacy," 21th edition, (David B. Troy ed., 2006, p. 745-775, p. 802-836 and p. 837 - 849).

As used herein, the term "pharmaceutical composition" refers to any composition comprising at least the VLP with or without cargo and at least one other ingredient, as well as any product which results, directly or indirectly, from combination, complexation, or aggregation of any two or more of the ingredients, from dissociation of one or more of the ingredients, or from other types of reactions or interactions of one or more of the ingredients. Accordingly, the term "pharmaceutical composition" as used herein may encompass, inter alia, any composition made by admixing a pharmaceutically active ingredient and one or more pharmaceutically acceptable carriers.

The term "low stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 25 percent formamide, following standard Southern blotting procedures for 12 to 24 hours. The carrier material is finally washed three times each for 15 minutes using 2x SSC, 0.2 percent SDS at 50 degrees centigrade.

The term "medium stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 35 percent formamide, following standard Southern blotting procedures for 12 to 24 hours. The carrier material is finally washed three times each for 15 minutes using 2x SSC, 0.2 % SDS at 55 °C.

The term "high stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 50 percent formamide, following standard Southern blotting procedures for 12 to 24 hours. The carrier material is finally washed three times each for 15 minutes using 2x SSC, 0.2 % SDS at 65 °C.

## 2. Fusion Protein

In a first aspect, the invention provides a fusion protein comprising a VP1 binding protein and an exogenous peptide, wherein the exogenous peptide comprises a cargo-securing peptide and/or an endosomal translocating peptide (ETP).

The term "VP1 binding protein" refers to any peptide that has the ability to bind to the major capsid protein VP1 of a polyomavirus, either synthetic or naturally occurring. In particular, the VP1 binding protein is a peptide comprising the VP1 interacting domain (VID) of a polyomavirus VP2/VP3 protein.

The capsids of all polyomaviruses have a similar structural set-up including the proteins VP1, VP2, VP3, and agnoprotein. The icosahedral virus capsid is formed by up to 72 VP1 pentamers. In the center of each of the pentamers, facing to the inside of the capsid, a VP2 or VP3 protein may be located. VP3 is identical to the C-terminal two-thirds of VP2. This shared region comprises *inter alia* the nuclear localization signal (NLS), the DNA-binding domain (DBD), and the VP1 interacting domain (VID).

"VP2" or "virus protein 2" according to the invention refers to a protein which is identical to or derived from the natural VP2 of the JC virus, having the amino acid sequence according to SEQ ID NO: 1. A protein derived from the natural VP2 of the JC virus preferably has an amino acid sequence homology or identity with the amino acid sequence according to SEQ ID NO: 1 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP2.

"VP3" or "virus protein 3" according to the invention refers to a protein which is identical to or derived from the natural VP3 of the JC virus, having the amino acid sequence according to SEQ ID NO: 2. A protein derived from the natural VP3 of the JC virus preferably has an amino acid sequence homology or identity with the amino acid sequence according to SEQ ID NO: 3 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP3.

The VID may be derived from a VP2 or VP3 or differently termed functional equivalent thereof from any known polyomavirus.

In a preferred embodiment of the invention, the VID is derived from human polyoma virus comprising Human polyomavirus 6 (HPyV6), Human polyomavirus 7 (HPyV7), Human polyomavirus 9 (HPyV9), BK polyomavirus (BKPyV), JC polyomavirus (JCPyV), Merkel Cell polyomavirus (MCPyV), KI polyomavirus (formerly known as Karolinska Institute polyomavirus, KIPyV), WU polyomavirus (formerly known as Washington University polyomavirus, (WUPyV), Trichodysplasia spinulosa-associated polyomavirus (TSV), human polyoma virus 10 (HPyV10), MW polyomavirus and MX polyomavirus. In a more preferred embodiment of the invention, the VID is derived from the. The VID from human polyoma virus JCV is identified by SEQ ID NO:3.

The VP1 binding protein according to the invention comprises the VP1 interacting domain of VP2 and allows a positioning of the fusion protein and, in particular, the exogenous peptide within a VLP derived from a polyomavirus. Preferably, the VP1 interacting domain has an identity of at least 90 %, preferably at least 95 %, more preferably at least 98 % to SEQ ID NO: 3. Thus, the VP1 binding protein preferably comprises at least a sequence with an identity of at least 90 %, preferably at least 95 %, more preferably at least 98 % to SEQ ID NO: 3.

The VP1 binding protein is preferably a full length polyomavirus VP2 or VP3. These proteins are naturally adapted for the interaction with the VP1. Accordingly, in one embodiment of the first aspect of the invention, the VP1 binding protein comprises an amino acid sequence that has an identity of at least 80 %, preferably at least 90 %, more preferably at least 95 % to SEQ ID NO: 1 or SEQ ID NO: 2.

However, any fragment or sub-structure of VP2 or VP3 may be sufficient for a tight interaction with VP1 as long as it contains a functional VP1 interacting domain of VP2/VP3. For example, the VP1 binding protein according to the invention may include or exclude the DNA-binding domain. For example, the VP1 binding protein may comprise the VID and the NLS of VP2. Further examples are a VP1 binding protein comprising the VID and the NLS of VP2, a VP1 binding protein comprising the VID and the DBD of VP2, and VP1 binding protein comprising the VID, DBD and the NLS of VP2.

The VP1 binding protein may be a modified version of VP2 or VP3, e.g. mutated by insertion, deletion, or amino-acid replacement with respect to SEQ ID NO: 1 or 2. However, the protein may only be modified to the point that the VP1 interacting domain is still functional, i.e. still binds to a polyomavirus VP1.

The exogenous peptide may be located at any position of the fusion protein, i.e. at the C-terminus, at the N-terminus, or at any position within the amino acid sequence of the fusion protein. The location of the exogenous peptide is preferably on the surface of the folded protein. The exogenous peptide is further preferably freely accessible when the fusion protein is bound to the VLP capsid. The skilled person knows how to determine positions within the amino acid sequences that fulfill these prerequisites. The structure predictions of VP2 or VP3 show that the N-terminus and the C-terminus are located on the surface of VP2 and VP3, and oriented to the inside of the polyoma virus when VP2 or VP3 is bound to a VP1 pentamer.

In one embodiment of the first aspect of the invention the exogenous peptide forms the C-terminus or the N-terminus of the fusion protein. A construct containing the exogenous peptide on the C-terminus or N-terminus of the protein has the further advantage of an easier construction of the polynucleotide encoding the fusion protein. The C-terminus is particularly preferred as the location for the exogenous peptide because it is the part of the protein that is the last to be translated. Thus, an exogenous peptide on the C-terminus has the lowest influence on protein folding. According to one embodiment of the first aspect, the endosomal translocating peptide, preferably the CPP, is located on the C-terminus of the protein. Alternatively, the endosomal translocating peptide, preferably the CPP, forms the N-terminus of the fusion protein. According to an alternative embodiment, the cargo-securing peptide, in particular the cargo binding peptide, forms the C-terminus of the fusion protein. Alternatively, the cargo binding peptide may form the C-terminus.

The exogenous peptide preferably has a percentage of basic amino acids of at least 25 %, more preferably of at least 30 %.

According to one embodiment of the first aspect of the invention, the exogenous peptide comprises a cargo-securing peptide. A cargo-securing peptide according to the invention is a peptide that affects the packaging of cargo in a VLP such that the cargo is better protected from the surrounding of the VLP in particular in the blood plasma or inside a cell.

Preferably the cargo-securing peptide is a cargo-binding peptide. Depending on the application of the VLP it may be used for transporting different types of cargo. Examples of cargo are single- or double-stranded DNA, single- or double-stranded RNA, peptides, hormones, lipids, carbohydrates, or other small organic compounds. Accordingly, the cargo-binding peptide may be specific for one or more of these possible cargos. A preferred cargo-binding peptide is a DNA-binding peptide. A further preferred cargo-binding peptide is an RNA-binding peptide.

As shown in the examples, a cargo-securing peptide fused to the VP2 or VP3 protein leads to an improved protection, i.e. less degradation, of DNA packaged into VLPs. Without being bound to theory, one explanation for this better protection of the DNA cargo is a tighter packaging of the VLP due to the improved interaction with the cargo. Accordingly, the cargo-securing peptide is in particular a cargo binding peptide. Wildtype polyomavirus VP2/VP3 already contain a DNA-binding domain located at the C-terminus. However, the addition of protamine-1 to the C-terminus VP2 or VP3 leads to an improved protection of the cargo with respect to the wild type VP2 or VP3 as shown in the examples.

The length of the cargo-binding peptide is in principle only limited by the requirement that it does not interfere with the folding of the fusion protein. However, the cargo-binding peptide preferably has a length in the range from 5 to 100 amino acids, more preferably in the range from 10 to 70 amino acids, most preferably in the range from 10 to 60 amino acids. In one embodiment, the length is in the range from 15 to 25 amino acids.

According to one embodiment of the first aspect of the invention, the amino acid sequence of the cargo-binding peptide has a percentage of basic amino acids of at least 40 %. Basic amino acids are positively charged. The positive charge facilitates a binding to negatively charged cargo, e.g. nucleotides. Preferably, the majority of the basic amino acids of the cargo-binding peptide are arginine residues. Accordingly, the percentage of arginine residues in the sequence of the cargo-binding peptide is at least 20 %, more preferably at least 25 %, most preferably at least 35 %. In a particularly preferred embodiment, the percentage of arginine is at least 40 %.

According to one embodiment of the first aspect of the invention the cargo-binding peptide comprises a structural motif $(R)_n$ wherein n is an integer of at least 2, at least 3, at least 4, at least 5.

Cargo-binding peptides according to the invention may be DNA-binding peptides, RNA-binding peptides, peptide-binding peptides, lipid-binding peptides, carbohydrate-binding peptides. Examples of such peptides are protamine-1 (PRM1), Snap tag, SAMp73, TFF2 and DOMON-like type 9 carbohydrate-binding module. Preferably, the CBP in the fusion protein according to the invention is protamine-1. Besides cargo-binding peptides the group of cargo-loading peptides also includes for example GFP or EGFP. As shown in the examples GFP bound to the C- or N-terminus of VP3 leads to an improved protection of the DNA cargo. According to one embodiment of the fusion protein according to the first aspect of the invention the cargo-securing peptide is neither GFP nor EGFP.

According to a further embodiment of the first aspect of the invention the amino acid sequence of the cargo-securing peptide has an identity of at least 80 %, preferably of at least 90 %, more preferably of at least 95 % to SEQ ID NO: 4 (Protamine-1) or SEQ ID NO: 5 (Protamine-1aa8-29). A sequence identity to SEQ ID NO: 4 is particularly preferred. As shown in the examples, protamine-1 bound to either VP2 or VP3 has a strong effect on the protection of the cargo transported by VLPs.

According to an alternative embodiment of the first aspect of the invention the exogenous peptide comprises an endosomal translocating peptide (ETP).

An "ETP" according to the invention is a peptide that has the ability to translocate itself and any cargo bound to it through the endosomal membrane. Preferred endosomal translocating peptides are in particular cell-penetrating peptides (CPP). Cell-penetrating peptides (CPPs) are short peptides that facilitate cellular uptake of various molecular cargo (from nano-size particles to small chemical molecules or large fragments of DNA). The cargo is associated with the peptides either through chemical linkage via covalent bonds or non-covalent interactions. The functions of the CPPs are to deliver the cargo into the cells. A process that commonly occurs through endocytosis with the cargo delivered to the endosomes of the living mammalian cells. However, other peptides not classified as CPPs have the same function providing means to translocate through the endosomal membrane. Such peptides are also included in the definition of ETPs. One example for such a peptide is a polyhistidine peptide. A polyhistidine peptide consists of at least six histidine (His) residues. It was shown that a polyhistidine peptide also has a destabilizing effect on membranes. According to one embodiment of the first aspect of the invention the ETP is not a $His_6$-tag.

CPPs typically have an amino acid composition that either contains a high relative abundance of positively charged amino acids such as lysine or arginine, or has sequences that contain alternating pattern of charged amino acids and non-polar/hydrophobic amino acids. These two types of structures are referred to as polycationic or amphiphatic, respectively. A third class of CPPs are hydrophobic peptides, containing only apolar residues with a low net charge or have hydrophobic amino acid groups that are crucial for cellular uptake.

The mechanism by which the CPPs translocate the plasma membrane and facility the delivery of molecular cargo to the cytoplasm or an organelle is not entirely understood. However, the theories of CPP translocation can be classified into three main entry mechanisms: direct penetration in the membrane, endocytosis-mediated entry, and translocation through the formation of a transitory structure.

The CPP is in theory not limited in length; however, the peptide must allow a correct folding of the fusion protein. The cargo-binding peptide preferably has a length in the range of 5 to 100 amino acids, more preferably in the range from 10 to 30 amino acids, most preferably in the range from 15 to 25 amino acids.

Preferably, the CPPs contain in addition to the basic amino acids also non-polar amino acids. In particular, the CPP has a percentage of non-polar amino acids of at least 25 %, preferably of at least 30 %, more preferably of at least 35 %. The groups of CPPs differ in their relative percentage of basic non-polar amino acids.

The first type of CPPs, the amphipathic CPPs consist of alternating basic and non-polar amino acids. The amphipathic form often generates a pore or channel through the membrane bilayer. Examples of amphipathic CPPs are the trans-activating transcriptional activator (TAT) from human immunodeficiency virus-1 (HIV-1) and penetratin, a peptide derived from the DNA-binding domain of antennapedia homeo protein. The second type of CPPs, the so-called polycationic CPPs include the HPV peptide L2. These CPPs comprise at least one cluster of basic amino acids adjacent to at least one cluster of hydrophobic amino acids. Both regions are required for full activity of the peptide. Without being bound to theory, scientific results suggest that the positive charge of the basic amino acid cluster mediates tight association with negatively charged lipids of the membranes and that subsequent insertion of the hydrophobic cluster into membranes induces a torsional stress which results in membrane disruption.

Amphipathic CPPs in particular have a percentage of basic amino acids in the range from 40 to 60 %, and a percentage of non-polar amino acids in the range from 28 to 39 %. The amphipathic CPPs preferably have a percentage of arginines in the range from 18 to 36 %, and a percentage of lysines in the range from 22 to 28 %.

The polycationic CPPs preferably have a percentage of arginines in the range from 26 to 30 %, and a percentage of lysines in the range from 3 to 8 %.

According to one embodiment of the first aspect of the invention, the amino acid sequence of the CPP comprises a structural motif $(R)_n$, wherein $_n$ is an integer of at least two, preferably of at least three, more preferably of at least four, and the sequence further comprises two or more adjacent non-polar amino acids. Polycationic CPP, such as HPV 33-L2, may have a sequence of four arginines and a sequence of three non-polar amino acids.

Preferred CPPs according to the invention are TAT, penetratin, and HPV 33-L2. TAT has an amino acid sequence as defined by SEQ ID NO: 6. Penetratin has an amino acid sequence as defined by SEQ ID NO: 7, and HPV 33-L2 has an amino acid sequence as defined by SEQ ID NO: 8. A further preferred CPP is a variant of HPV 33-L2, which is identified as HPV 33-L2-DD447 (SEQ ID NO: 9), differs from HPV 33-L2 by a replacement of the N-terminal phenylalanine and isoleucine by two aspartates. This variant was shown to have a stronger cell-penetrating effect (Kemper et al., 2006). Further examples of CPPs according to the invention are SynB1 (SEQ ID NO: 50), SynB3 (SEQ ID NO: 51), PTD-4 (SEQ ID NO: 52), PTD-5 (SEQ ID NO: 53), FHV Coat-(35-49) (SEQ ID NO: 54), BMV Gag-(7-25) (SEQ ID NO: 55), HTLV-II Rex-(4-16) (SEQ ID NO: 56), D-Tat (SEQ ID NO: 57), R9-Tat (SEQ ID NO: 58).

Thus, according to one embodiment of the first aspect of the invention, the amino acid sequence of the CPP has an identity of at least 80 %, preferably of at least 90 %, more preferably of at least 95 %, most preferably of at least 98 % to SEQ ID NO: 6. Alternatively, the amino acid sequence of the CPP has an identity of at least 80 %, preferably of at least 90 %, more preferably of at least 95 %, most preferably of at least 98 % to SEQ ID NO: 7. The sequence of the CPP may also have an identity of at least 80 %, preferably of at least 90 %, more preferably of at least 95 %, most preferably of at least 98 % to SEQ ID NO: 5. Moreover, the amino acid sequence of the CPP may have an identity of at least 80 %, preferably of at least 90 %, more preferably of at least 95 %, most preferably of at least 98 % to SEQ ID NO: 9.

According to one embodiment of the first aspect of the invention, the fusion protein comprises at least one exogenous cargo-binding peptide, and at least one exogenous CPP. That way, the fusion protein may provide to a VLP, which is used as a transport system for a specific cargo into a cell, a tighter packaging, an improved protection of the cargo, and, in addition, an improved ability to leave the endosomal pathway and arrive at the cytoplasm. Consequently, the combination of an ETP, in particular a CPP, and a cargo-securing peptide, in particular a cargo-binding peptide together in one fusion protein with the ability to bind to the major structural protein VP-1 strongly increases the yield of cargo entering the cytoplasm of a cell and thus increases the efficiency of a VLP mediated transport into a cell.

In a fusion protein comprising both a cargo-binding peptide and a CPP, the two peptides may be located at opposite termini of the fusion protein. Thus, either the cargo-binding peptide forms the N-terminus of the fusion protein, and the CPP forms the C-terminus of the fusion protein, or the cargo-binding peptide forms the C-terminus of the fusion protein and the CPP forms the N-terminus of the fusion protein. Both termini of VP2 and VP3 are presented to the surface of the protein and to the inside of the VLP when the VP2/VP3 is attached to VP1. VP2 and VP3 both contain a DNA-binding sequence on the C-terminus of the peptide. Thus, preferably, the exogenous cargo-binding peptide forms the C-terminus of the fusion protein and the CPP forms the N-terminus of the fusion protein.

According to one embodiment of the first aspect of the invention, one exogenous peptide comprises the CPP and the cargo-binding peptide and forms the N- or C-terminus of the protein. A localization of the two peptides on the C-terminus is preferred. The localization on the C-terminus is advantageous in cases in which the exogenous peptide may interfere with the protein folding. As protein folding already occurs co-translationally, there will be less interference by an exogenous peptide bound to the C-terminus which is only translated in the end.

According to one embodiment of the first aspect of the invention a fusion protein is provided, wherein the VP1 binding protein is VP2 and the cargo-securing peptide is protamine-1.

According to one embodiment of the first aspect of the invention a fusion protein is provided, wherein the VP1 binding protein is VP3 and the cargo-securing peptide is protamine-1.

According to one embodiment of the first aspect of the invention a fusion protein is provided, wherein the VP1 binding protein is VP2 and the CPP is penetratin.

According to one embodiment of the first aspect of the invention a fusion protein is provided, wherein the VP1 binding protein is VP3 and the CPP is penetratin.

According to one embodiment of the first aspect of the invention a fusion protein is provided, wherein the VP1 binding protein is VP2 and the CPP is TAT.

According to one embodiment of the first aspect of the invention a fusion protein is provided, wherein the VP1 binding protein is VP3 and the CPP is TAT.

According to one embodiment of the first aspect of the invention a fusion protein is provided, wherein the VP1 binding protein is VP2 and the CPP is HPV 33-L2.

According to one embodiment of the first aspect of the invention a fusion protein is provided, wherein the VP1 binding protein is VP3 and the CPP is HPV 33-L2.

According to one embodiment of the first aspect of the invention a fusion protein is provided, wherein the sequence of the VP1 binding protein is at least 95 % identical to SEQ ID NO: 1, and the sequence of the exogenous peptide is identical to SEQ ID NO: 4, and the exogenous peptide forms the C-terminus of the fusion protein. In particular, the fusion protein has sequence according to SEQ ID NO: 10.

According to one embodiment of the first aspect of the invention a fusion protein is provided, wherein the sequence of the VP1 binding protein is at least 95 % identical to SEQ ID NO: 2, and the sequence of the endogenous peptide is identical to SEQ ID NO: 4, and the exogenous peptide forms the N-terminus of the fusion protein. In particular, the fusion protein has sequence according to SEQ ID NO: 17.

According to a further embodiment of the first aspect of the invention a fusion protein is provided, wherein the sequence of the VP1 binding protein is at least 95 % identical to SEQ ID NO: 1, and the sequence of the endogenous peptide is identical to SEQ ID NO: 6, and the exogenous peptide forms the C-terminus of the fusion protein. In particular, the fusion protein has sequence according to SEQ ID NO: 12.

According to a further embodiment of the first aspect of the invention a fusion protein is provided, wherein the sequence of the VP1 binding protein is at least 95 % identical to SEQ ID NO: 2, and the sequence of the exogenous peptide is identical to SEQ ID NO: 6, and the exogenous peptide forms the N-terminus of the fusion protein. In particular, the fusion protein has sequence according to SEQ ID NO: 19.

According to a further embodiment of the first aspect of the invention a fusion protein is provided, wherein the sequence of the endogenous peptide is at least 95 % identical to SEQ ID NO:1, and the sequence of the exogenous peptide is identical to SEQ ID NO: 7, and the exogenous peptide forms the C-terminus of the protein In particular, the fusion protein has sequence according to SEQ ID NO: 13.

According to a further embodiment of the first aspect of the invention a fusion protein is provided, wherein the sequence of the VP1 binding protein is at least 95 % identical to SEQ ID NO: 2, and the sequence of the exogenous peptide is identical to SEQ ID NO: 7, and the exogenous peptide forms the N-terminus of the protein In particular, the fusion protein has sequence according to SEQ ID NO: 20.

According to a further embodiment of the first aspect of the invention a fusion protein is provided, wherein the sequence of the VP1 binding protein is at least 95 % identical to SEQ ID NO: 1, and the sequence of the exogenous peptide is identical to SEQ ID NO: 8, and form the C-terminus of the protein. In particular, the fusion protein has sequence according to SEQ ID NO: 14.

According to a further embodiment of the first aspect of the invention a fusion protein is provided, wherein the sequence of the VP1 binding protein is at least 95 % identical to SEQ ID NO: 2, and the sequence of the exogenous peptide is identical to SEQ ID NO: 8, and the exogenous peptide forms the N-terminus of the protein In particular, the fusion protein has sequence according to SEQ ID NO: 21.

According to a further embodiment of the first aspect of the invention a fusion protein is provided, wherein the sequence of the VP1 binding protein is at least 95 % identical to SEQ ID NO: 1, and the sequence of the exogenous peptide is identical to SEQ ID NO: 9, and the exogenous peptide forms the C-terminus of the protein In particular, the fusion protein has sequence according to SEQ ID NO: 15.

According to a further embodiment of the first aspect of the invention a fusion protein is provided, wherein the sequence of the VP1 binding protein is at least 95 % identical to SEQ ID NO: 2, and the sequence of the exogenous peptide is identical to SEQ ID NO: 9, and the exogenous peptide forms the N-terminus of the protein. In particular, the fusion protein has a sequence according to SEQ ID NO: 22.

3. Virus-Like Particle

The fusion protein according to the first aspect of the invention is particularly useful in the context of a virus-like particle (VLP) derived from a polyomavirus.

Thus, according to a second aspect of the invention, a VLP is provided which comprises a fusion protein according to the first aspect of the invention.

Non-limiting examples for viruses of the polyoma family are: B-lymphotropic polyomavirus (formerly known as African green monkey polyomavirus, AGMPyV) (LPyV), Baboon polyomavirus 1 (SA12), Bat polyomavirus (formerly known as Myotis polyomavirus, MyPyV; BatPyV) BK polyomavirus (BKPyV), Bornean orang-utan polyomavirus (OraPyV1), Bovine polyomavirus (BPyV), California sea lion polyomavirus (SLPyV), Hamster polyomavirus (HaPyV), JC polyomavirus (JCPyV), Merkel Cell polyomavirus (MCPyV), Murine pneumotropic virus (formerly known as Kilham strain of polyomavirus, Kilham virus, K virus; MPtV), Murine polyomavirus (MPyV), Simian virus 40 (formerly known as Simian vacuolating virus 40; SV40), Squirrel monkey polyomavirus (SqPyV), Sumatran orang-utan polyomavirus (OraPyV2), Trichodysplasia spinuolsa-associated polyomavirus (TSPyV), Human polyomavirus 6 (HPyV6), Human polyomavirus 7 (HPyV7), KI polyomavirus (formerly known as Karolinska Institute polyomavirus, KIPyV), WU polyomavirus (formerly known as Washington University polyomavirus, (WUPyV), Avian polyomavirus (formerly known as Budgerigar Fledgling disease polyomavirus, BFPyV, APyV), Canary polyomavirus (CaPyV), Crow polyomavirus (CPyV), Finch polyomavirus (FPyV), Goose Hemorrhagic polyomavirus (GHPyV), Athymic rat polyomavirus (RatPyV), Baboon polyomavirus 2 (BPyV2), Cynomolgus polyomavirus (CyPV), Gorilla gorilla gorilla polyomavirus 1 (GggPyV1), Human polyomavirus 9 (HPyV9), Trichodysplasia spinulosa-associated polyomavirus (TSV), Mastomys polyomavirus (multimammate mouse - Mastomys species), Pan troglodytes verus polyomavirus 1a (PtvPyV1a), Pan troglodytes verus polyomavirus 2c (PtvPyV2c), Rabbit kidney vacuolating virus (RKV).

Preferably the VLP is derived from a human polyoma virus comprising Human polyomavirus 6 (HPyV6), Human polyomavirus 7 (HPyV7), Human polyomavirus 9 (HPyV9), BK polyomavirus (BKPyV), JC polyomavirus (JCPyV), Merkel Cell polyomavirus (MCPyV), KI polyomavirus (formerly known as Karolinska Institute polyomavirus, KIPyV), WU polyomavirus (formerly known as Washington University polyomavirus, (WUPyV), Trichodysplasia spinulosa-associated polyomavirus (TSV), human polyoma virus 10 (HPyV10), MW polyomavirus and MX polyomavirus. In a more preferred embodiment of the invention, the VLP is derived from the human polyoma virus JCV.

VLPs are multi-protein structures that mimic the organization and conformation of authentic native viruses but lack the viral genome.

The virus-like particle according to the invention is preferably derived from human polyomavirus. In the context of the invention, the term "from human polyomavirus" refers to a VLP with structural proteins that can be isolated or extracted from polyomaviruses or which can be generated by recombinant expression of a polyoma structural protein or a modified form of said structural protein.

A VLP derived from a polyomavirus according to the second aspect of the invention, comprises a fusion protein according to the first aspect of the invention. The VLP further comprises one or more copies of the major capsid protein VP1. The VLP is preferably composed of a capsid built from multiple copies of VP1. VP1 assembles into pentameric structures, thus, preferably, the VLP is composed of several VP1 pentamers, in particular up to 72 VP1 pentamers. The VLP capsid may optionally comprise further proteins or other molecules. The structural proteins or molecules assembling the VLP in addition to the fusion protein according to the first aspect of the invention can either be identical to native polyomavirus proteins or it can be modified in order to optimize the VLP characteristics.

"VP1" or "virus protein 1" according to the invention refers to a protein which is identical to or derived from the natural VP1 of the JC virus, having the amino acid sequence according to SEQ ID NO: 24. A protein derived from the natural VP1 of the JC virus preferably has an amino acid sequence homology or identity with the amino acid sequence according to SEQ ID NO: 24 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP1. The terms "VP1 derived from the natural VP1 of the JC virus" and "VP1 derived from JC virus" in particular also include VP1 which is identical to the natural VP1 of the JC virus. The term "VP1" according to the invention also encompasses fractions and derivatives of the natural VP1, which are capable of assembling into VLP. Preferably, said fractions and derivatives of VP1 at least comprise amino acids 32:316 of the amino acid sequence according to SEQ ID NO: 24 or a derivative thereof, having a homology or identity with the amino acid sequence from amino acid position 32:316 of SEQ ID NO: 9 of at least 80 %, of at last 85 %, of at least 90 %, of at least 95 %, of at least 97 %, of at least 98 %, or of at least 99 %.

The virus capsid built from preferably 72 copies of VP1 pentamers is shaped like a hollow sphere. The VP2 and VP3 proteins have the ability to bind to the center of a VP1 pentamer by means of the VP1 interaction domain, so that the VP2 or VP3 protein faces to the inside of the VLP capsid. Thus, according to one embodiment of the second aspect of the invention, the fusion protein is located on the inside of the VLP capsid.

A localization of the fusion protein on the inside of the VLP capsid facilitates the assembly of the VLP around a specific cargo recognized by the cargo-binding domain of the fusion protein. Moreover, binding of the fusion protein to both the VP1 and the cargo strengthens the packaging of the VLP.

Localization on the inside on the VLP is also advantageous for the CPPs of the fusion protein. In this position, the CPPs are hidden within the VLP and cannot act on a membrane before being modified in the endosome. The VLP derived from polyomavirus enters the cell by endocytosis. The endocytic pathway of mammalian cells consists of distinct membrane compartments which internalize molecules from the plasma membrane. The principle components of the endocytic pathway are: early endosomes, late endosomes, and lysosomes. Early endosomes are the first station of the endocytic pathway, and are often located in the periphery of the cell receiving most types of vesicles coming from the cell's surface. They have a characteristic tubular-vesicular structure, and a mildly acidic pH. The late endosomes receive internalized material on the way to the lysosomes usually from early endosomes and the endocytic pathway. These late endosomes are acidic with a pH of about 5.5. It is assumed that the low pH of the late endosomes leads to partial uncoating of the capsids. Due to this partial uncoating, the CPPs in the fusion protein are presented to the outside and lead to a destabilization of the endosomal membrane, so that the VLP and, accordingly, also the transported cargo, is released into the cytosol of the cell.

A VP1 interacting domain is derived from the same polyomavirus as the VP1 forming the VLP is particularly preferred.

According to one embodiment of the second aspect of the invention, the VLP further comprises a VP1 fusion protein with first and a second peptide,

- wherein the first peptide is VP1 or a fragment thereof and
- the second peptide comprises a targeting region and a first and a second interaction region,
- the second peptide is located on the surface of the fusion protein,
- the second peptide comprises at least two interaction pairs, wherein an interaction pair is formed by an amino acid of the first interaction region and an amino acid of the second interaction region,
- the interaction region between the amino acid of an interaction pair is covalent or non-covalent, and
- at least one interaction pair is a covalent interaction pair in which the amino acids are covalently bound.

The second peptide of the VP1 fusion protein, i.e. the targeting peptide, is based on a particular secondary structure resembling a hair pin known from single-stranded polynucleotides especially in RNA molecules. When folded into its secondary structure, the peptide preferably comprises two paired regions of the amino acid sequence, the first and second interaction region and an unpaired loop comprising the targeting region.

The targeting region of the second peptide comprises an amino acid sequence - the targeting sequence - that interacts with a target of interest, in particular a cellular receptor. The secondary structure of the second peptide may also be described as a stem loop comprising a stem region and a loop region. Accordingly, the two interaction regions of the peptide preferably form the stem and the targeting region forms the loop. When located on the surface of the VP1 fusion protein, the stem, i.e. the first and second interaction region of the second peptide, lead to a sufficient spacing between the surface of the protein and the targeting region so that an interaction with a targeting recognizing means where in particular a cellular receptor is possible without steric hindrance.

The folding of the structure is based on the following theoretic principle. During protein folding, the amino acids on the first and second and second interaction region get into proximity. When two complementary amino acids of the two interaction regions get in proximity to each other, they will transiently bind to each other, i.e. interact non-covalently, and, thus, form a non-covalent interaction pair. The more non-covalent interaction pairs are formed at a time, the higher is the binding strength and the longer the transient interaction of the two interaction regions. The interaction pairs of the second peptide are preferably set up such that the formation of these non-covalent interaction pairs brings the amino acids of the covalent interaction pair, in particular cysteines, into proximity to each other for a sufficient time so as to allow formation of a covalent bond, e.g. a disulfide-bridge. The formation of the covalent interaction pair leads to a further stabilization of the interaction of the first and second interaction region of the second peptide. Accordingly, the final secondary structure of the second peptide with a loop including the targeting region and a stem formed by the first and second interaction region is formed. The loop can be regarded as a circular peptide connected by a covalent interaction pair. It was shown for a variety of signaling/targeting peptides that a circular shape of the peptide improves its recognition by the specific receptor.

Thus, according to one embodiment in the primary structure of the second peptide the amino acid sequence of the targeting region is located between the amino acid sequences of the first and second interaction region. A location of the amino acid sequence of the targeting region between the first and second interaction region allows obtaining a targeting region that is located in the loop of the folded second peptide.

The amino acid sequence of the targeting region may overlap with the amino acid sequences of the first and/or second interaction region. In particular, the amino acids forming the covalent interaction pair may be part of the targeting region.

The amino acid sequence of the targeting region may be any sequence that is recognized or binds to a target molecule, in particular a cellular receptor. Non-limiting examples of such peptides are Lyp-1 (SEQ ID NO: 76), RGD, RGR, HER2 binding peptide (SEQ ID NO: 77), CREKA peptide (SEQ ID NO: 78), NGR peptide, CPP-2 (SEQ ID NO: 79), CPP-44 (SEQ ID NO: 80), F3 (SEQ ID NO: 81), RMS-P3 (SEQ ID NO: 82), F56 (SEQ ID NO: 83), LTVSPWY-peptide (SEQ ID NO: 84), and WNLPWYYSVSPT-peptide (SEQ ID NO: 85).

Thus, according to one embodiment, the targeting region comprises a sequence selected from the group consisting of SEQ ID NO: 76, RGD, RGR, SEQ ID NO: 77, SEQ ID NO: 78, NGR, SEQ ID NO: 79, SEQ ID NO: 80, SEQ ID NO: 81, SEQ ID NO: 82, SEQ ID NO: 83, SEQ ID NO: 84, or SEQ ID NO: 85. Preferably the amino acid sequence of the targeting region comprises SEQ ID NO: 76.

Lyp-1 is a tumor homing peptide that selectively binds the tumor-associated lymphatic vessels and tumor cells in certain tumors. The nine amino acid long peptide specifically recognizes the receptor P32. The RGD-peptide and NGR-peptide are a tri-peptides composed of L-arginine-glycine-L-aspartic acid and L-asparagine-glycine-L-arginine, respectively. The sequences are common elements in cellular recognition. RGD peptides are implicated in cellular attachment via integrins. The HER2 binding peptide specifically targets the Human Epidermal Growth Receptor 2 (HER2). The CREKA peptide is a tumor homing peptide identified in phage display libraries consisting of the sequence Cys-Arg-Glu-Lys-Ala (see Simberg D, et al. Biomimetic amplification of nanoparticle homing to tumors. Proc Natl Acad Sci USA. 2007 Jan 16;104(3):932-6). CPP-2 and CPP-44 are tumor homing peptides described in Kondo et al. Tumourlineage-homing cell-penetrating peptides as anticancer molecular delivery systems. Nat Commun. 2012 Jul 17;3:951. F3 comprises amino acid sequences 17-48 of High Mobility Group Nucleosomal Binding Protein 2 (HMGN2) and was identified in a phage display cDNA library screen for peptides capable of homing to tumors, especially to their vascular endothelium (see (see Christian et al., Nucleolin expressed at the cell surface is a marker of endothelial cells in angiogenic blood vessels. J Cell Biol. 2003 Nov 24;163(4):871-8). RMS-P3 is a furin targeted peptide suitable for targeting Rhabdomyosarcoma (RMS) cells (see Hajdin K, et al. Furin targeted drug delivery for treatment of rhabdomyosarcoma in a mouse model. PLoS One. 2010 May 3;5(5)). F56 specifically binds to VEGF receptor Flt-1 (see Herringson and Altin, Effective tumor targeting and enhanced anti-tumor effect of liposomes engrafted with peptides specific for tumor lymphatics and vasculature. Int J Pharm. 2011 Jun 15;411(1-2):206-14). LTVSPWY-peptide and WNLPWYYSVSPT-peptide specifically bind to breast cancer cells (see Shadidi and Sioud, Identification of novel carrier peptides for the specific delivery of therapeutics into cancer cells, FASEB J. 2003 Feb;17(2):256-8).

According to one embodiment, the loop between the first and second interaction region which comprises the targeting region has a number of amino acids in the range from 3 to 50 amino acids. The number of amino acids of the loop is counted from the covalent interaction pair "closing" the loop and consequently includes the amino acids of the covalent interaction pair. Accordingly, if the number of amino acids in the loop is 2 the loop only includes the covalent interaction pair. Thus, the minimal number of amino acids in the loop is 3. The maximum length of the loop is in principle limited by the influence of the peptide on the folding of the VP1 fusion protein and the tendency for aggregation with higher length. Thus, the maximum number of amino acids in the loop is preferably 25, more preferably 20, most preferably 15 amino acids. According to a particularly preferred embodiment, the number of amino acids in the loop is in the range from 5 to 15 amino acids.

The covalent interaction pair may be formed by any two amino acids, the side chains of which may form a covalent bond. These may be in particular cysteines or seleno cysteines which form disulfide bridges. According to one embodiment, the covalent interaction pair is formed by a cysteine in the first interaction region and by a cysteine in the second interaction region. A VP1 fusion protein may comprise more than one covalent interaction pair. For example, the VP1 fusion protein may comprise 7 or less, 6 or less, 5, or less, 4 or less, 3 or less, 2 or less interaction pairs. The covalent interaction pairs may be located in sequence or spaced apart. Preferably, the VP1 fusion protein comprises one covalent interaction pair.

According to one embodiment, at least two interaction pairs are non-covalent interaction pairs in which the amino acids interact non-covalently. Preferably, the second peptide comprises at least 3 non-covalent interaction pairs, more preferably at least 4 non-covalent interaction pairs. In principal, the higher the number of interaction pairs, the stronger the interaction of the first and second interaction region of the second peptide. The number of non-covalent interaction pairs also depends on the type of interaction of the amino acids. The non-covalent interaction may be by hydrogen bridges, van der Waal forces, hydrophobic interactions or acid-base interactions.

Preferably, at least a part of the non-covalent interaction pairs are acid-base interaction pairs formed by an acidic amino acid in one interaction region and a basic amino acid in the other interaction region. At a neutral pH, these amino acids are charged negatively and positively, respectively. The contrary charges of the amino acids lead to an attraction of these amino acids and consequently of the interaction regions. Moreover, the contrary charges provide a tight binding of binding.

According to one embodiment, the second peptide comprises 2 to 20 acid-base interaction pairs, preferably 2 to 10 acid-base interaction pairs, more preferably 2 to 6 acid-base interaction pairs, most preferably 3 to 5 acid-base interaction pairs. The higher the number of acid-base interaction pairs, the higher the attraction of the first and second interaction region. However, a number more than 20 acid-base interaction pairs will be problematic for the folding of the VP1 fusion protein. A number of more than 10 acid-base interaction pairs renders cloning more problematic as very long primers have to be used. Moreover, it is assumed that the use of more than 6 acid-base interaction pairs does not further significantly increase the interaction of the first and second interaction region. With regard to ease of cloning and optimal strength of interaction of the non-covalent interaction pairs, a number of 3 to 5 acid-base interaction pairs are preferred. In a particularly preferred embodiment the second peptide comprises 4 acid-base interaction pairs.

The charged amino acids, i.e. the acidic and basic amino acids of the first and second interaction region may be directly in sequence or contain a spacer of one or more non-charged amino acids. According to one embodiment of the invention, spacing of the charged amino acids within the amino acid sequence of the first interaction region is 0 or 1 amino acid. Likewise the spacing of the charged amino acids in the second interaction region is preferably 0 or 1. More preferably the spacing of charged amino acids in the first and/or second interaction is 0. Thus, the charged amino acids in the first (or second)interaction region are directly connected.

Basic amino acids according to the invention can be arginine, lysine or histidine. Acidic amino acids according to the invention can be glutamic acid or aspartic acid.

The first and second interaction region may comprise both acidic and basic amino acids, only acidic amino acids or only basic amino acids. The basic and acid amino acids in one interaction region may be alternating or form clusters. Non-limiting examples of alternating sequences are: EERR, ERER, EERREE, RREERR. Examples of clusters are EEERRR, DDERKK, DDDRR. However, it is preferred that one of the interaction regions comprises a majority of acidic amino acids and the other, consequently, a majority of basic amino acids. For example the first inter action region comprises the mainly basic sequence RRRRE and the second interaction region comprises the mainly acidic sequence EEEER.

According to a preferred embodiment, the non-covalent interaction pairs are acid-base interaction pairs and formed by an acidic amino acid in the first interaction region and basic amino acid in the second region. The first interaction region may comprise at least 2, at least 3, at least 4, at least 5, at least 6 acidic amino acids. Also, the second interaction region may comprise at least 2, at least 3, at least 4, at least 5, at least 6 basic amino acids. Preferably, the first interaction region comprises at least 4 acidic amino acids and the second interaction region comprises at least 4 basic amino acids. More preferably, the first interaction region comprises at least 4 consecutive acidic amino acids and the second interaction region comprises at least 4 consecutive basic amino acids.

According to one embodiment, the majority of the basic amino acids are arginine. In an alternative embodiment, the majority of the basic amino acids are lysines. Preferably, all basic amino acids in the interaction region are arginine acids.

In a preferred embodiment, the first interaction region comprises four consecutive arginines. According to one embodiment, the majority of the acidic amino acids are glutamic acid. According to an alternative embodiment, the majority of acidic amino acids are aspartic acids. Preferably, all acidic amino acids in the second peptide are glutamic acids. In particular the first interaction region comprises the sequence EEEE and the second interaction region comprises the sequence RRRR.

The spacing region between the covalent interaction pair or pairs and the non-covalent interaction pair or pairs has an influence on the formation of the hair pin-like structure of the second peptide. If the number of amino acids forming the spacer is too high, the effect of bringing the amino acids of the covalent interaction pair proximity by means of the binding of the one or more non-covalent interaction pairs may be lost. In contrast, a too short distance may be problematic for steric reasons. For example, the size of the side chains of the acidic and basic amino acids is bigger than the size of the side chain of cysteines. Accordingly, if the cysteines are directly adjacent to the charged amino acids in the second peptide a disulfide bridge might not form. Thus, according to one embodiment, the number of amino acids in the first and second interaction region between the at least one covalent interaction pair and the closest non-covalent interaction pair is in the range from 1 to 6, preferably 1 to 4, more preferably 1 to 3. Most preferably, the spacers in both interaction regions between the at least one covalent interaction pair and the closest non-covalent interaction pair is 2 amino acids.

In addition, the type of amino acids forming the spacer between the at least one covalent interaction pair and the closest non-covalent interaction pair influences the formation of the covalent bond. For the spacers, polar uncharged amino acids, with short side chains are preferred such as glycine, serine or alanine. More preferably the amino acids of the spacers between the at least one covalent interaction pair and the closest non-covalent interaction pair are glycine and serine. According to a particularly preferred embodiment, the spacers between the covalent interaction pair and the non-covalent interaction pair consist of one glycine and one serine.

Preferably, the second peptide is introduced into a region of the first peptide that is not essential for folding so that the second peptide does not interfere with the folding of the first peptide. Moreover, it is preferred that the second peptide is introduced into a region of the first peptide that is located on the surface of the first peptide when folded. The skilled person knows how to determine suitable positions within an amino acid sequence. Suitable positions are preferably determined from crystal structures of the protein or related proteins. Preferably, the second peptide is located in a loop of the first peptide of the fusion protein. More preferably in a loop on the surface of the first peptide.

Preferably, the first peptide of the VP1 fusion protein is a VP1 from JCV. According to X-ray crystallography analysis the folded VP1 (e.g. PDB entry 3NXD) contains three loops on the surface of the protein. Two of these loops, the DE-loop (aa 120-137) and the HI-loop (aa 262-272) are known to be eligible for the introduction of exogenous peptide structures as an exogenous structure introduced into the loop is accessible and in general does not interfere with folding of the VP1 protein. Thus, according to one embodiment, the second peptide of the VP1 fusion protein is located in the DE-loop or the HI-loop of VP1. Preferably, the second peptide is located between amino acid 120 and 137 (DE-loop) or 262 and 272 (HI-loop) of VP1. More preferably between amino acid 129 and 132 (DE-loop) or 265 and 268 (HI-loop) of VP1.

According to one embodiment of the second aspect of the invention, the VLP comprises no cargo. In the context of this invention, a VLP without cargo is also referred to as "empty VLP".

Surprisingly, it was found that an empty VLP exhibits cytotoxic properties when introduced into a cell (cf. Example 10). A VLP without cargo preferably comprises a fusion protein according to the first aspect of the invention with an exogenous peptide comprising a CPP. As defined above, the CPP leads to an increased yield of VLPs reaching the cytoplasm of the cells. Thus, an empty VLP with a fusion protein comprising CPP exhibits an increased cytotoxic effect. Due to the cytotoxic effect empty VLPs may, for example, be used as chemotherapeutic agents.

The cytotoxic effect is preferably cell specific. A high cell specificity of the cytotoxic effect has the advantage that only specific cell types can be targeted to be highly cell specific in order not to harm any healthy cells. Thus, an empty VLP preferably comprises targeting means for specific cells on which the cytotoxic effect should be acted on. This targeting means may, for example, be a VP1 protein comprising an exogenous peptide with a targeting region and a first and a second interaction region, as defined above.

According to an alternative embodiment of the second aspect of the invention, the VLP comprises a cargo. Non limiting examples of cargo are single-stranded or double-stranded DNA, single-stranded or double-stranded RNA, peptides, hormones, lipids, carbohydrates, or other small organic compounds or mixtures thereof. Preferably, the cargo is a substance that produces an effect in a eukaryotic cell, in particular a mammalian cell. A preferred type of cargo is a substance that is pharmaceutically active. Preferably, the cargo is a molecule able to activate the RNA pathway. More preferably, the cargo is siRNA.

According to one embodiment the cargo is double-stranded DNA and the VP1-binding protein comprises the VID, the NLS and the DBD of VP2. According to an alternative embodiment, the cargo is double-stranded DNA VP1-binding protein comprises the VID, the NLS and the DBD of VP2.

According to one embodiment, the VLP is for use as a medicament. In particular, the VLP is for use in the treatment of tumor diseases. In this regard, the VLP provides a vehicle for the cargo, preferably a pharmaceutically active ingredient, to enter the cells of an organism. Thus, according to one embodiment the VLP is for use as a drug delivery system. For this purpose the VLP is loaded by a drug of interest. The loading of the drug is in particular performed by disassembly of the VLP into pentamers and reassembly in the presence of the cargo. The VLP drug delivery system is then used to deliver the loaded drug to a specific target.

In one embodiment, the cargo is a pharmaceutically active substance that is not applicable by itself to a patient or leads to strong side effects. An example of a group of such substances is chemotherapeutic substances. According to a further embodiment, the cargo is a diagnostic agent. Preferably, the diagnostic agent is a substance used in imaging methods. More preferably, the diagnostic agent is a dye, in particular a fluorescent dye. Thus, according to one embodiment of the second aspect, the VLP is for use in a diagnostic method. Examples of diagnostic methods are the diagnosis of tumors and metastasis.

The treatment or diagnosis of a patient with a VLP according to the second aspect of the invention comprises the transfer of the cargo into a cell of an organism. Preferably, the organism is a mammal, more preferably, a human.

According to one embodiment of the second aspect of the invention, the VLP comprises more than one copy of the fusion protein according to the first aspect of the invention. The icosahedral capsid of a polyomavirus VLP in principal consists of 72 copies of a VP1 pentamer. The VP2 and VP3 proteins bind to the center of a VP1 pentamer. Thus, the polyomavirus VLP may contain up to 72 copies of the fusion protein according to the first aspect of the invention. The higher the number of copies of fusion proteins comprising a cargo-binding peptide according to the invention, the stronger the cargo-binding, and, thus, the tighter the packaging of the VLP. Moreover, a higher number of copies of a CPP according to the invention leads to a stronger destabilization of the endosomal membrane, and, thus, to an improved release of the VLPs into the cytosol.

Therefore, the VLP, according to the second aspect of the invention, comprises preferably at least two copies of the fusion protein according to the first aspect of the invention, more preferably at least five copies. On the other hand presence of a high number of the VP2 or VP3 fusion proteins in the VLP may have a negative effect on formation of virus like particles. Thus, preferably the number of copies of the fusion protein according to the first aspect of the invention of 20 or less, preferably 15 or less more preferably 10 or less.

According to one embodiment of the second aspect of the invention, the VLP comprises two or more different fusion proteins according to the first aspect. In particular, the VLP may comprise one type of fusion protein with a CPP one type of fusion protein with a cargo-binding peptide. For example, the VLP may comprise at least one copy of a full length VP2 with a C-terminal cargo-binding peptide, and a at least one copy of full length VP2 with a C-terminal CPP, or the VLP may comprise at least one copy of a full length VP2 with a C-terminal cargo-binding peptide and at least one copy of full length VP3 with a C-terminal CPP. In particular, the VLP may comprise at least one copy of a fusion protein of SEQ ID NO: 17 and at least one copy of a fusion protein of SEQ ID NO: 15.

4. Pharmaceutical composition

According to a third aspect, the invention provides a pharmaceutical composition that comprises at least one fusion protein according to the first aspect of the invention and at least one pharmaceutically acceptable excipient. According to one embodiment of the third aspect the pharmaceutical composition comprises a VLP according to the second aspect of the invention and at least one pharmaceutically acceptable carrier. Examples of preferred carriers are PBS, Tris buffer and aqueous solutions.

5. Polynucleotide

According to a fourth aspect, the invention provides an isolated polynucleotide that comprises a nucleic acid sequence encoding a fusion protein according to the first aspect of the invention.

The techniques used to isolate or clone a polynucleotide encoding a peptide are known in the art and include isolation from genomic DNA, preparation from cDNA, or a combination thereof. The cloning of the polynucleotides from such genomic DNA can be effected, e.g., by using the well-known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used.

The isolated polynucleotides preferably comprise a first part encoding the VP1 binding protein and second part encoding the exogenous peptide. The first part of the polynucleotide encoding the VP1 binding protein preferably has a degree of sequence identity to the sequence coding for the VP1 interacting domain of VP2/VP3 from JCV SEQ ID NO: 27 of at least 80 percent, at least 85 percent, at least 90 percent, at least 95 percent, at least 96 percent, at least 97 percent, at least 98 percent, at least 99 percent, or 100 percent. In one embodiment of the fourth aspect of the invention, the first part of the polynucleotide encoding the VP1 binding protein hybridizes under very low stringency conditions, low stringency conditions, medium stringency conditions, medium-high stringency conditions, high stringency conditions, or very high stringency conditions with SEQ ID NO: 27.

Preferably, the first part of the polynucleotide encoding the VP1 binding protein has a degree of sequence identity to the JCV-VP2 coding sequence SEQ ID NO: 25 of at least 80 percent, at least 85 percent, at least 90 percent, at least 95 percent, at least 96 percent, at least 97 percent, at least 98 percent, at least 99 percent, or 100 percent. In one embodiment of the fourth aspect of the invention the first part of the polynucleotide encoding the VP1 binding protein hybridizes under very low stringency conditions, low stringency conditions, medium stringency conditions, medium-high stringency conditions, high stringency conditions, or very high stringency conditions with SEQ ID NO: 25.

Alternatively, first part of the polynucleotide encoding the VP1 binding protein preferably has a degree of sequence identity to the JCV-VP3 coding sequence SEQ ID NO: 26 of at least 80 percent, at least 85 percent, at least 90 percent, at least 95 percent, at least 96 percent, at least 97 percent, at least 98 percent, at least 99 percent, or 100 percent. Preferably, the first part of the polynucleotide encoding the VP1 binding protein hybridizes under very low stringency conditions, low stringency conditions, medium stringency conditions, medium-high stringency conditions, high stringency conditions, or very high stringency conditions with SEQ ID NO: 26.

According to one embodiment of the fourth aspect of the invention, the second part of the polynucleotide encoding the exogenous peptide preferably has a degree of sequence identity to SEQ ID NO: 28 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the exogenous peptide preferably hybridizes under very low stringency conditions, low stringency conditions, medium stringency conditions, medium-high stringency conditions, high stringency conditions, or very high stringency conditions with SEQ ID NO: 28.

According to a further embodiment of the fourth aspect of the invention, the second part of the polynucleotide encoding the exogenous peptide may have a degree of sequence identity to SEQ ID NO: 30 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the exogenous peptide hybridizes under very low stringency conditions, low stringency conditions, medium stringency conditions, medium-high stringency conditions, high stringency conditions, or very high stringency conditions with SEQ ID NO: 30.

According to one embodiment of the fourth aspect of the invention, the second part of the polynucleotide encoding the exogenous peptide preferably has a degree of sequence identity to SEQ ID NO: 31 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the exogenous peptide hybridizes under very low stringency conditions, low stringency conditions, medium stringency conditions, medium-high stringency conditions, high stringency conditions, or very high stringency conditions with SEQ ID NO: 31.

According to a further embodiment of the fourth aspect of the invention, the second part of the polynucleotide encoding the exogenous peptide may have a degree of sequence identity to SEQ ID NO: 32 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the exogenous peptide hybridizes under very low stringency conditions, low stringency conditions, medium stringency conditions, medium-high stringency conditions, high stringency conditions, or very high stringency conditions with SEQ ID NO: 32.

According to a further embodiment of the fourth aspect of the invention, the second part of the polynucleotide encoding the exogenous peptide may have a degree of sequence identity to SEQ ID NO: 33 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the exogenous peptide hybridizes under very low stringency conditions, low stringency conditions, medium stringency conditions, medium-high stringency conditions, high stringency conditions, or very high stringency conditions with SEQ ID NO: 33.

In particular the polynucleotide according to the invention may have a degree of sequence identity to SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 of at least 80 percent, at least 85 percent, at least 90 percent, at least 95 percent, at least 96 percent, at least 97 percent, at least 98 percent, at least 99 percent, or 100 percent. More preferably, the polynucleotide encoding the fusion protein hybridizes under very low stringency conditions, low stringency conditions, medium stringency conditions, medium-high stringency conditions, high stringency conditions, or very high stringency conditions with SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46.

## 6. Expression vector

In a fifth aspect, the invention also relates to expression vectors comprising a polynucleotide according to the fourth aspect of the invention. The expression vector further preferably comprises control elements such as a promoter, and transcriptional and translational stop signals. The polynucleotide of according to the fourth aspect and of the control elements may be joined together to produce a recombinant expression vector that may include one or more restriction sites to allow for insertion or substitution of the polynucleotide encoding the polypeptide at such sites. The polynucleotide may be inserted into an appropriate expression vector for expression. In creating the expression vector, the coding sequence is located in the expression vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide of the fourth aspect of the invention. The choice of the expression vector will typically depend on the compatibility of the expression vector with the host cell into which the expression vector is to be introduced. The expression vectors may be a linear or closed circular plasmid.

The expression vector may be adapted for cell-based or cell-free expression. The expression vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

Alternatively, the vector may be one that, when introduced into the host cell, is integrated into the genome and replicated together with the chromosome(s) into which it has been integrated. For integration into the host cell genome, the expression vector may rely on any other element of the expression vector for integration into the genome by homologous or non-homologous recombination. Alternatively, the vector may contain additional polynucleotides for directing integration by homologous recombination into the genome of the host cell at a precise location in the chromosome.

The vectors of the present invention preferably contain one or more (e.g., several) selectable markers that permit easy selection of transformed, transfected, transduced, or the like, cells. A selectable marker is a gene the product of which provides for biocide or viral resistance, resistance to heavy metals, prototrophy to auxotrophs, and the like.

The procedures used to ligate the elements described above to construct the recombinant expression vectors of the present invention are well known to one skilled in the art (see, e.g., Sambrook et al, 1989, supra).

## 7. Host Cells

According to a sixth aspect, the invention provides a host cell, comprising the expression vector according to the fifth aspect of the invention. The expression vector according to the fifth aspect is introduced into a host cell so that the expression vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will, to a large extent, depend upon the gene encoding the polypeptide and its source.

The host cell may be any cell useful in the recombinant production of a polypeptide of the present invention, e.g., a prokaryote or a eukaryote.

The prokaryotic host cell may be any Gram positive bacterium or a Gram negative bacterium. Gram positive bacteria include, but not limited to, Bacillus, Streptococcus, Streptomyces, Staphylococcus, Enterococcus, Lactobacillus, Lactococcus, Clostridium, Geobacillus, and Oceanobacillus. Gram negative bacteria include, but not limited to, E. coli, Pseudomonas, Salmonella, Campylobacter, Helicobacter, Flavobacterium, Fusobacterium, Ilyobacter, Neisseria, and Ureaplasma. Preferably, the host cell is E.coli.

The host cell may also be a eukaryote, such as a mammalian, insect, plant, or fungal cell. Preferably, the host cell is an insect cell, still more preferably a lepidopteran cell, and, most preferably, a cell selected from the group consisting of Sf9, Sf21, Express SF+, and BTITn-5B1-4 ("TN High Five").

## 8. Production Method

According to a seventh aspect, the invention provides a process of producing the VLP according the second aspect of the invention.

The process at least comprises the steps of protein expression of the fusion protein, according to the first aspect of the invention, with a polynucleotide according to the fourth aspect of the invention as a template, purifying the fusion protein and assembling several copies of the fusion protein together with several copies of VP1 to form a VLP.

For expression of fusion protein cell-based or cell-free *(in vitro*) expression systems may be used. Common cell based systems are bacteria, such as *E.coli, B. subtilis,* yeast, such as S. *cerevisiae* or eukaryotic cell lines, such as baculovirus infected Sf9 cells mammalian cells like CHO or HeLa.

Cell-free *(In vitro)* protein expression is the production of recombinant proteins in solution using biomolecular translation machinery extracted from cells. Cell-free protein production can be accomplished with several kinds and species of cell extract. Extracts used for cell-free protein expression are made from systems known to support high level protein synthesis. For example cell-free extracts capable are made from *E. coli,* rabbit reticulocyte lysates (RRL), wheat germ extracts, or insects cell (such as SF9 or SF21) lysates.

Preferably, a cell-based expression system is used. Thus, according to one embodiment of the sixth aspect of the invention the process of producing the VLP according the second aspect of the invention comprises at least the steps of:

a) introducing a polynucleotide, according to the fourth aspect, into a host cell;
b) culturing the transformed host cell in a medium under conditions leading to a protein expression with the polynucleotide according to the as a template;
c) isolating the expression product; and
d) assembly of a VLP with the expression product and VP1.

Suitable host cells and expression vectors are described above. The use of baculo viruses together with insect cells, is preferred. More preferably the insect cell line is Sf9 or High Five.

Methods of cultivation of host cells in a nutrient medium suitable for production of the fusion protein are well known in the art. For example, the host cell may be cultivated by shake flask cultivation, small-scale or large-scale fermentation (including continuous, batch, fed-batch, or solid state fermentations) in laboratory or industrial fermentors performed in a suitable medium and under conditions allowing the fusion protein to be expressed. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). Depending on the host/vector system used, the fusion protein may or may not be secreted into the nutrient medium. In case it is secreted, the polypeptide can be recovered directly from the medium. Otherwise, the cells are separated from the culture medium and lysed.

Methods of cell lysis are known in the art. Non-limiting examples of the methods of cell lysis are mechanical disruption, liquid homogenization, sonication, freeze-thaw procedure or mortar and pestle.

The fusion protein may be recovered using methods known in the art. For example, the fusion protein may be recovered from the nutrient medium by conventional procedures including, but not limited to, centrifugation, filtration, extraction, spray-drying, evaporation, or precipitation.

The fusion protein may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides.

Expressed VP1 assembles into VLPs upon expression. The VLP may be disassembled by treatment with DTT and EDTA. Under these conditions, the VLP disassembles into VP1 pentamers. The VP1 pentamers can be reassembled into icosahedral VLPs by dialysis against a $Ca^{2+}$ buffer. In order to produce a VLP with the fusion protein, the fusion protein may be incubated with the VP1 pentamers.

The fusion protein according to the first aspect can, for example, be incorporated into the capsid envelope by co-expression of the respective fusion protein and VP1 in a suitable host cell, e.g.an eukaryotic cell. In a preferred embodiment of the invention, the fusion protein is co-expressed with a VP1 or a fusion protein comprising the VP1. In particular, the fusion protein and the VP1 are coexpressed in Sf9 cells.

Active substances can be incorporated into the interior of the capsid envelope by, for example, dissociation of the capsid envelope and subsequent re-association in the presence of the active substance or by osmotic shock of the VLP in the presence of the active substance.

## EXAMPLES

### EXAMPLE 1: Production of VLPs with VP2/VP3 fusion proteins

#### I. Cloning of VP1 and VP2 fusion protein coding nucleotides into pFastBacDual.VP1VP2coHA vector

Oligonucleotides (Life technologies) used in the cloning:

L2 sense: (SEQ ID NO: 59)
CCATATTTTTTTACAGATGTCCGTGTGGCGGCCTGAGCGGCCGCTTTC

L2 antisense: (SEQ ID NO: 60)
AAAACGTTTACGCCTGCGACGTAAAATAAAGTCGACAGCGTAATCTGGAAC

L2DD447 antisense: (SEQ ID NO: 61)
AAAACGTTTACGCCTGCGACGTAAATCATCGTCGACAGCGTAATCTGGAAC

Tat sense: (SEQ ID NO: 62)
CGCCGTCCACCCCAAAAGCGCAAG GGCTGAGCGGCCGCTTTC

Tat antisense: (SEQ ID NO: 63)
ACGTTGGCGACGCTTCTTGCGCCCGTCGACAGCGTAATCTGGAAC

Penetratin sense: (SEQ ID NO: 64)
AATCGACGAATGAAATGG AAA AAATGAGCGGCCGCTTTC

Penetratin antisense: (SEQ ID NO: 65)
TTGAAACCAGATTTTGATTTGTCGGTCGACAGCGTAATCTGGAAC

HIS sense: (SEQ ID NO: 66) CATCACCATTGAGCGGCCGCTTTC

HIS antisense: (SEQ ID NO: 67) GTGATGATGGTCGACAGCGTAATCTGGAAC

pFastBacDual.VP1_VP2coHA (SEQ ID NO: 68)

Before the PCR the primers were phosphorylated 20 min at 37°C and 10 min at 75°C in the following reaction set up:

10 μl primer (10 μM)
2 μl buffer A
2 μl 10 mM dATP
10 U T4 polynucleotidkinase (Fermentas)
ad 20 μl bidest

The following PCR mixture was prepared:

31 μl bidest
10 μl 5x buffer Q5 (NEB)
5 μl dNTPs
1 μl pFastBacDual.VP1_VP2coHA (50 ng)
1 μl sense primer
1 μl antisense primer
1 μl Q5 polymerase

For PCR, the following temperature profile was used: An initial activation at 98 °C for 30 sec followed by 35 repetitions of the following cycle steps 1 to 3: 1) Denaturation: 98 °C for 10 seconds; 2) Annealing: 60 °C for 30 seconds; and 3) Extension: 72 °C for 5 min. After the temperature cycling, the samples were again kept at 72 °C for 10 min until the samples were retrieved.

PCR samples were separated by agarose gel electrophoresis and fragments of about 7400 bp were eluted using the QiaEx Kit (Qiagen) with the modification that the elution was performed with 30 μl of 70°C buffer 4 (NEB). The eluted samples were subsequently incubated with 2 μl Dpnl (digest of methylated template plasmid DNA) for 2h at 37°C followed by an additional 20 min at 80°C for inactivation of the Dpnl. Afterwards the PCR fragments were religated to using the T4 ligase.

The plasmids were then transformed into competent DH5α bacteria by thermal shock and selection of recombinant clones by growth on Ampicillin agar plates.

The DNA of the recombinant clones was prepared and analysed using restriction. analysis with Sail. Clones with correct restriction pattern were sequenced.

**II. Generation of recombinant baculovirus**

The baculoviruses were generated using the "Bac-to-Bac" system (Invitrogen). For details of the protocol it is referred to the "Bac-to-Bac" manual. The protocol includes the following steps a to f:

***a) Transformation of DH10 bacteria with corresponding pFastBacDual construct***

***b) Isolation and PCR analysis of recombinant bacmid***

Oligonucleotides:

| | | |
|---|---|---|
| Puc/M13-BACfor | FW | CCCAGTCACGACGTTGTAAAACG (SEQ ID NO. 69) |
| Puc/M13-BACrev | RW | AGCGGATAACAATTTCACACAGG (SEQ ID NO. 70) |

***c) Transfection of Sf9 with recombinant bacmid***

***d) Production of recombinant baculovirus***

***e) Western blot analysis of protein expression*** Western Blot (VP1 and VP2/3 fusion protein) was performed using mouse monoclonal VP1 antibody (254C7E4) and mouse monoclonal HA antibody (12CA5) for detection of VP2/VP3coHA

***f) qPCR analysis of P3 supernatants (number of bacmids)***

The following qPCR mixture was prepared:

10 µl 2x DynNAmo HS SYBR Green Mix (Thermo)
7 µl bidest
1 µl P3 fw primer TGACATGCTGCCCTGCTACT (SEQ ID NO: 71)
1 µl P3 rw primer GCAAGTCAGGTCCTCGTTCAG (SEQ ID NO: 72)
1 µl template

The template was either a recombinant Baculovirus, a standard (purified bacmid) or bidest $H_2O$.

For qPCR, the following temperature profile was used: An initial activation at 95 °C for 10 min followed by 35 repetitions of the following cycle steps 1 to 3: 1) Denaturation: 95 °C for 10 seconds; 2) Annealing: 60 °C for 20 seconds; and 3) Extension: 72 °C for 30 sec. After the temperature cycling, the samples were again heated to 95 °C.

**III. Expression of recombinant proteins in Sf9 and production of EPN**

***a) Protein expression***

Sf9 were grown to a cell density of 2 x $10^7$ in serum-free TC100 medium and infected with the recombinant baculovirus with a multiplicity of infection (MOI) of 5. After infection the cells were grown for 5 to 7 days at 27°C producing the corresponding protein encoded by the baculovirus. The produced protein is secreted into the expression medium. In the cell supernatant the secreted proteins self-assemble into VLPs.

***b) Purification of VLPs from the supernatant***

Sf9 cells were separated from the supernatant by centrifugation for 5 min at 500 x g. Cells were discarded and the supernatant was centrifuged a second time for 90 min at 5000 x g in order to remove larger impurities.

The VLPs were then separated by ultracentrifugation. For this, 15 ml of clarified supernatant was loaded on 3 ml 40% sucrose.

Ultracentrifugation was performed in a Sorwall MX150 for 4h at 100000 x g and 4°C. In the centrifugation tube a pellet formed by the VLPs which was harvested. The VLP containing pellet was resuspended in Tris buffer (10 mM Tris, 150 mM NaCl, pH 7,5) The protein concentration of the resuspended VLPs was determined and adjusted to 0,5 µg/µl by the addition of Tris-Buffer.

**IV. Analysis**

***a) Analysis of the Particle assembly***

1 .Hemagglutination assay (HA) to analyze VLP's activity_

The quality and quantity of EPN preparation was visualized by hemagglutination of red blood cells. VLPs attach to molecules present on the surface of red blood cells (RBCs). A consequence of this is that at certain concentrations, the VLP suspension may agglutinate the RBCs, thus preventing the RBCs from settling out of suspension. For visualization 96 well U-bottom plates are used. The hemagglutination assay was performed in 96-well U-bottom plates (Greiner). In the wells of the plate settling out of the red blood cells can be recognized by a dark red central spot formed by the red blood cells at the bottom of the tube.

Human "O" type red blood cells were briefly centrifuged at 500 x g for 10 min. Red blood cells were then washed twice and resuspended in Alsever's buffer (27 mM sodium citrate, 70 mM NaCl, 100 mM glucose, 2.6 mM citric acid). Serial twofold dilutions of VLPs were prepared in Alsever's buffer in a volume of 50 µl. 50 µl of red blood cells were added into each well and mixed. Plates were incubated for 2h at 4°C. An example of a Hemagglutination assay test plate is shown in Fig. 10

2. Transmission Electron microscopy

For electron microscopy VLP preparations were loaded on 400 mesh copper grids with carbon film (Science Services) and negatively stained with uranyl acetate (2%). Pictures were taken with a ZEISS 922 TEM. An exemplary image of a VLP sample is shown in **Fig. 11.**

**b) Expression and Purity**

Presence of recombinant viral proteins was again monitored by Western Blot analysis and the purity of VLP preparations by protein staining using InstantBlue dye (Fa. Expedeon).

**c) Analysis of the incorporation of VP2 fusion proteins into VP1 pentamers**

5 μg of the VLP to be tested were dissociated in 100 μl Dissociation buffer (10 mM Tris-HCl, 150 mM NaCl, 5 mM DTT, 10 mM EDTA) by incubation for one hour at 25°C and 600 rpm shaking. Dynabeads®MagneticBeads (LifeTechnologies) were resuspended for 10 min. 0.3 μg Dynabeads in 10 μl per sample were transferred into a reaction tube. The tubes were put into the magnetic part and the supernatants of the Dynabeads were discarded. 100 μl PBS/Tween 0.02% were added together with 0.5 μg of precipitating antibody (mouse monoclonal HA antibody 12CA5). The same experimental set up without antibody served as negative control for each antibody. The reaction mixture was tested incubated for 10 min at room temperature (RT). The tubes were put into the magnetic part and supernatants were discarded. The magnetic beads were washed with 100 μl PBS/Tween 0.02%. The VLP samples were added to the reaction tube in a volume of 100 μl and incubated at least 10 min at RT. The tubes were put into the magnetic part and supernatants were discarded. The beads were washed three times with PBS/Tween 0.02%. The beads-antibody-VP2/3coHA complexes were resuspended in 100 μl PBS and transferred into new reaction tubes. The reaction tubes were put into the magnetic part and supernatants were discarded. Beads-antibody-VP2/3coHA complexes were resuspended in 2x SDS loading buffer and heated for 5 min at 5 min at 95°C. The tubes were put into the magnetic part and supernatants were harvested. VP1 co-immunoprecipitation was tested by Western blot analysis using mouse monoclonal VP1 antibody 254C7E4.

**d) Analysis of the incorporation of VP2/VP3 fusion proteins**

To investigate the incorporation of minor capsid proteins in VLPs, 300 μg of EPN preparations were loaded onto 3 ml of a sucrose step gradient (10-50%, 5 steps a 600 μl). Gradients were centrifuged at 36.000 rpm and 4°C for 35 min in a S50ST rotor. After ultracentrifugation, 300 μl fractions were harvested from the top of each gradient. Subsequently, 30 μl of each fraction were tested either for the presence of VP1 or VP2/VP3coHA by Western blot analysis.

**EXAMPLE 2: Intracellular localization of VLPs with VP2/ETP fusion proteins**

**I. Localization of VLPs in COS7 cells**

***a) Production and assembly of VLPs***

The following four types of VLPs are expressed and assembled according to the protocol of example 1:

1) A VLP only consisting of VP1,
2) A VLP consisting of VP1 and VP2,
3) A VLP consisting of VP1 VLP and a VP2 fusion protein with a C-terminal penetratin peptide (SEQ ID NO: 13), and
4) A VLP consisting of VP1 VLP and a VP2 fusion protein with a C-terminal His-tag (SEQ ID NO: 16).

***b) Internalization of VLPs***

COS7 cells were inoculated into the culture medium DMEM+ (10% FCS, 1% penicillin/streptomycin) and grown on cover slips in a 24 well plaid at 37°C. Upon reaching a cell density 5 x $10^4$ cells/well, 10 μg of a VLP is added to the cell culture medium and the cells are further incubated for 24 hours at 37°C. After the incubation, the cells are washed three times with 1x PBS and fixed with 150 μl methanol at a temperature of -20 °C.

***c) Antibody labelling of the VLPs in the cells***

The cells were then washed two times with 1 ml 1x PBS.

After the second wash-step, 1x Roti-Immunobloc (Fa. Roth) was added in a volume of 1 ml to each well. The cells were incubated with the blocking buffer for 30 minutes at room temperature (RT).

Primary antibody

A mouse anti-VP1 antibody (254C7E4) or a rabbit polyclonal anti-VP1 antibody at a concentration of 0.5 μg/ml was diluted 1:100 in 1x Roti-Immunobloc. 50 μl of the antibody solution was added to the cells, and incubated for one hour at room temperature (RT). The cells were then washed three times with 1x PBS/Tween 0.1%. For washing about 1 ml of the PBS/Tween mixture is added to the cells and removed again from the cells.

Secondary antibody

An anti-mouse, anti-rabbit Ig-Alexa546 (Life Technologies) was diluted 1:1000 in 1xroti-immunobloc and 50 μl of the antibody solution are added to the cells. The cells are incubated with the secondary antibody for one hour at room temperature.

***d) Fluorescent microscopy***

Afterwards, the cells are washed again three times with 1x PBS/0.1 % (v/v) Tween. The cells on cover slips were then mounted on a slide using ProlongGold anti-fade reagent + DAPI (from Molecular Probes) to counterstain cell nuclei. The preparations were finally analyzed with the Nikon Eclipse TS 100-F microscope and pictures were taken with the corresponding NIS software.

The results are shown in **Fig. 1. Fig. 1** consists of four panels representing the experimental results obtained with the four types of virus-like particles as defined above. Virus-like particles of types 1 to 4 are shown from the left to the right.

In the images, the cells are visible as light grey structures before a black background. Light grey or white structures within the cells represent areas with a high concentration of VLP. Darker areas contain low concentrations of VLP. The VLP concentration is proportional to the intensity of the signal. There is an obvious difference between the pictures of the two left panels and the two right panels.

In the two left panels representing the results of VLPs with only VP1 or VP1 and wild type VP2, a variety of bright spots are visible. In contrast, light grey areas, that means areas with a high signal, are more evenly distributed in the cells or in the two right panels which represent the results of VP2 fusion proteins. Moreover, in the two right panes very few bright spots are found.

An explanation of this result is that the VLPs consisting of VP1 only or VP1 and wild type VP2 are mainly located within the endosomes. The high concentration of VLPs within the endosomes leads to fluorescence spots of very high intensity. Thus, the majority of the proteins is not able to leave the endosomal pathway.

On the other hand, the VLPs with VP2 fusion proteins VP2-penetratin (VP2-PENp) or his-tagged VP2 (VP2-HISp) and accordingly the fluorescence signals of these VLPs are evenly distributed in the cell because the VLPs are able to leave the endosomal pathway and a lower percentage of the VLPs is trapped in the endosomal pathway.

**II. Localization of VLPs in HeLa cells**

The internalization of VLPs in HeLa cells was tested using the same protocol as described under Example 1 I. for COS7 cells. The following VLP constructs were used:

1) A VLP only consisting of VP1,
2) A VLP consisting of VP1 and a VP2 fusion protein with C-terminal penetratin peptide (SEQ ID NO: 13), and
3) A VLP consisting of VP1 and a VP2 fusion protein with C-terminal His-tag (SEQ ID NO: 16).

The results are shown in **Fig. 2** which consists of three panels representing from left to right the experimental results obtained with the VLP constructs 1) to 3) as defined above. In the images, the cells are visible as light grey structures before a black background. Light grey or white structures within the cells represent areas with a high concentration of VLP. Darker areas contain low concentrations of VLP. The VLP concentration is proportional to the intensity of the signal.

Again, light grey areas are more evenly distributed in the cells or in the central and right panel which represent the results of VP2- fusion proteins while in the HeLa cells infected with VLPs with only (left panel) several bright spots are visible. Accordingly, also in HeLa cells the VLPs consisting of VP1 only are mainly located within the endosomes and the VLPs with VP1 and the VP2-fusion proteins (VP2-PENp or VP2-HISp) spread out in the cells.

### III. Localization of VLPs in TC620 cells

The experiment of Example 2 I. and II. was repeated with TC620 cells and two VLP constructs: 1) VP1 and 2) VP1 and VP2-L2DD447 fusion protein.

In contrast to the protocol described in Example 2 I. the cells were fixed with 4% PFA, and stained for membranes with wheat germ agglutinin (WGA). Pictures were taken with a Zeiss LSM from the top to the bottom of the cells.

Fig. 3 shows images of sections of cells infected with the VP1-VLP in the upper row and cells infected with VP1/VP2-L2DD447-VLP in the bottom row. The images from left to right top, middle and bottom sections.

The section images of the cells infected with VP1/VP2-L2DD447-VLP show a much higher fluorescent signal throughout the cell as compared to the cells infected with VP1. Accordingly, a higher number of VP1/VP2-L2DD447-VLPs

### EXAMPLE 3: Packaging of DNA using VLP with VP2/VP3 fusion proteins.

VLPs formed by the following proteins were used for the packaging test: wild type VP1 (VP1), wild type VP1 and VP2 (VP1_VP2), wild type VP1 and VP3 (VP1_VP3), wild type VP1 and VP2 with an N-terminal GFP fusion tag (VP1_GFP-VP2), wild type VP1 and VP3 with an N-terminal GFP fusion (VP1_GFP-VP3), VP1 wild type and VP2 with a C-terminal GFP (VP1_VP2-GFP), wild type VP1 and VP3 with a C-terminal GFP (VP1_VP3-GFP), wild type VP1 and VP2 with a C-terminal protamine-1 (VP1_VP2-PRTM), wild type VP1 and VP3 with a C-terminal protamine-1 (VP1_VP3-PRTM).

#### *a) VLP production*

VLPs from the above identified proteins were produced according to the protocol of example 1.

#### *b) Packaging of the DNA*

DNA is packaged into the VLPs by a dissociation of the VLPs and reassociation in the presence of the DNA.

25 μg of the VLP are solved in 100 μl TRIS buffer (10 mM Tris-HCl, 150 mM NaCl, 5 mM DTT, 10 mM EDTA) and incubated for one hour at 25°C and 600 rpm shaking. Afterwards, 5 μg DNA (pGI4.51, #42) in water were added to the VLP solution and the protein/DNA mixture was incubated for one hour at room temperature to allow a binding of the VLP proteins to the DNA.

For reassociation, the VLP DNA mixture was transferred to 100 μl dialysis cassettes (Thermo Scientific) and the dialysis cassette was placed into a TRIS buffer as defined above with additional 1 mM $CaCl_2$. At a temperature of 4 °C, the dialysis was allowed to happen for 72 hours. After this time period, the buffer was exchanged against a Glutathion buffer.

<u>Composition of the Glutathion buffer:</u>

10 mM TRIS, pH 7.5
150 mM NaCl
1 mM $CaCl_2$
4.5 mM GSSG (glutathione ox., Firma Roth)
0.5 mM GSH (glutathion red., Firma Roth)

The dialysis cassettes were stored in 100 ml of this buffer for 24 hours at 4 °C.

#### *c) DNAse treatment of VLPs*

After reassociation, VLP samples were divided in halves and one half was treated with DNase I (Fermentas). For DNase treatment, 50 units DNAseI and 6 mM $MgCl_2$ were added to the sample and incubated for 1 hour at 37 °C in a micro test tube (Eppendorf).

#### *d) Recovery of packaged DNA*

For recovery, at first the VLPs are digested. Thus, 40 μl of packaging samples with or without DNaseI treatment were incubated in 500 μl LP buffer and 50 μl 10% SDS (w/v) for one hour at 56 °C.

Composition of the LP buffer:

10 mM TRIS, pH 8.2,
400 mM NaCl
2 mM EDTA
0.3 mg/ml proteinase K.

After one hour of incubation, 500 µl roti-phenol (Fa. Roth) were added, mixed thoroughly and centrifuged at 11,000 g for 10 minutes. After centrifugation, the water-soluble upper phase was transferred into a new micro test tube and mixed with 500 µl chloroform. The resulting mixture was incubated for one hour at 4 °C wherein the tube was constantly rolled. After incubation, the mixture was centrifuged again for 10 minutes at 11,000 g. Again, the water-soluble upper face was transferred to a new tube and mixed with 500 µl 2-propanol at a temperature of -20 °C and 50 µl of a 5 M NaCl solution. The resulting mixture was incubated for one hour at -20 °C without shaking. The mixture was then centrifuged for 30 minutes at 4 °C and 11,000 g. After centrifugation, the liquid was decanted leaving a pellet at the bottom of the tube. The pellet was washed with 500 µl 70% (v/v) ethanol, dried and finally resuspended in 35 µl bi-distillated $H_2O$. The resuspended DNA was then stored at - 20 °C.

***e) Quantification of the recovered DNA by q-PCR analysis.***

PCR was performed in BR clear 96-well PCR plates (Greiner).
The following PCR mixture was used:

10 µl 2x DyNAmo HS SYBR Green Mix (Thermo Scientific)
7 µl $H_2O_{bid}$
1 µl FW primer
1 µl RW primer
1 µl DNA.

DNA Quantification FW primer
CTTGGCAATCCEGTACTGTT (SEQ ID NO: 73).
DNA Quantification RW primer
ATATGGCGTCGGTAAAGGC (SEQ ID NO: 74).

The DNA in the mix was either one of the DNA samples recovered under step 4. In the negative control ("NTC") instead of the DNA sample $H_2O_{bid}$ was added to the PCR mixture.
As a standard for determining the DNA concentration, the plasmid pGI4.51 was measured in different concentrations in the range from $10^3$ to $10^7$ molecules.
For PCR, the following temperature profile was used: An initial activation at 95 °C for 10 minutes followed by 40 repetitions of the following cycle steps 1 to 3: 1) Denaturation: 95 °C for 10 seconds; 2) Annealing: 60 °C for 20 seconds; and 3) Extension: 72 °C for 30 seconds. After the temperature cycling, the samples were again heated for at 95 °C for 10 seconds, cooled to 55 °C for 5 seconds heated again to 95 °C until the samples were retrieved.
Using the standard the signal obtained in the PCR reactions could be related to a specific number of DNA molecules using the CFX manager. Comparing the samples of a specific VLP treated and untreated with DNase a DNA protection value in percent was obtained. Accordingly, a protection value in percent can be obtained for each VLP construct. In particular the DNA protection is defined by

$$\text{DNA protection} = N_{treated}/N_{untreated} * 100$$

with
$N_{untreated}$ = Number of DNA molecules not treated with DNase
$N_{treated}$ = Number of DNA molecules treated with DNase
Thus, it represents the percentage of DNA protected in by a VLP construct. The results are represented for each of the VLP constructs in **Fig. 4.**

**EXAMPLE 4: Packaging of DNA using VLP with VP2/VP3 fusion proteins, influence of the concentration of the fusion protein**

A second DNA packaging test was performed with the following constructs: a VLP build from VP1 as negative control and VLPs build from VP1-VP2-protamine-1 with different concentrations of the VP2-protamine-1 fusion protein.

The protocol described in Example 3 was repeated with VP1 and VP1-VP2-protamine-1. In addition two more VLP constructs were created by mixing the disassociated VP1 and VP1-VP2-protamine-1 pentamers in step b) in a ratio of 5:1 and 10:1.

DNA protection results are shown in **Fig. 5.** VP1-VP2-protamine-1 and both constructs with a reduced number of VP2-protamine-1 (VP1:VP1-VP2-protamine-1 5:1 and 10:1) exhibit high DNA protection values. Interestingly, in both cases of reduced VP2-protamine the percentage of protected DNA is higher than for the VP1-VP2-protamine-1 construct.

**EXAMPLE 5: Packaging of siRNA using VLP with VP2/VP3 fusion proteins.**

VLPs formed by the following capsid proteins were used for the packaging test:

1) VP1,
2) VP1-VP2,
3) VP1-VP2-protamine-1, and
4) VP1-VP2-pentratin.

***a) VLP production***

VLPs from the above identified proteins were produced according to the protocol of example 1.

***b) Packaging of the siRNA***

siRNA is packaged into the VLPs by a dissociation of the VLPs and reassociation in the presence of the siRNA.

75 μg (5 pmol) of the VLP are solved in 150 μl TRIS buffer in a micro test tube (Eppendorf) and incubated for one hour at 25°C and 600 rpm shaking.

After the one hour incubation, 10 pmol Kif1_8 siRNA (SEQ ID NO: 49) were added to the VLP solution and the protein/siRNA mixture was incubated for another hour at room temperature to allow a binding of the VLP proteins to the siRNA.

For reassociation, the VLP/siRNA mixture was transferred to 100 μl dialysis cassettes (Thermo Scientific) and the dialysis cassette was placed into 2 l of TRIS buffer as defined above with additional 1 mM $CaCl_2$ and incubated at a temperature of 4 °C. After 72 hours of dialysis the buffer was exchanged against a Glutathion buffer.

The dialysis cassettes were stored in 100 ml of this buffer for 24 hours at 4 °C.

***c) Benzoase treatment of VLPs***

After reassociation, VLP samples were divided in halves, and one half was treated with Benzoase (Novagen). For Benzoase treatment, 25 units Benzoase and 6 mM $MgCl_2$ were added to the sample and incubated for 1 hour at 37 °C in a micro test tube (Eppendorf).

***d) Recovery of packaged siRNA***

For recovery of the siRNA, at first the VLPs are digested. Thus, 40 μl of packaging samples with or without Benzoase treatment were incubated in 500 μl LP buffer and 50 μl 10% SDS (w/v) for one hour at 56 °C.

After one hour of incubation, 500 μl Roti-Phenol (Fa Roth) were added, mixed thoroughly, and centrifuged at 11,000 g for 10 minutes. After centrifugation, the water-soluble upper phase was transferred into a new micro test tube and mixed with 500 μl chloroform. The resulting mixture was incubated for one hour at 4 °C, wherein the tube was constantly rolled. After incubation, the mixture was centrifuged again for 10 minutes at 11,000 g. Again, the water-soluble upper face was transferred to a test tube and mixed with 500 μl 2-propanol at a temperature of -20 °C and 50 μl of a 5 M NaCl solution. The resulting mixture was incubated for one hour at -20 °C without shaking. The mixture was then centrifuged for 30 minutes at 4 °C and 11,000 g. The liquid was decanted leaving a white pellet at the bottom of the tube. The pellet was washed with 500 μl 70% (v/v) ethanol, dried and finally resuspended in 35 μl bi-distillated $H_2O$. The resuspended siRNA was stored at -20° Celsius. Before storage at -20 °C 1 μl of RNasin (Promega) was added.

***e) Quantification of the recovered siRNA by RT and qPCR analysis.***

For quantification the siRNA is first transcribed into cDNA by reverse transcriptase (RT) and afterwards quantified by quantitative (real time) qPCR.

The following mixture was prepared for reverse transcriptase reaction and applied in to the wells of a 96 well plate (Fa. Greiner):

| | |
|---|---|
| 5x HiSpec Buffer | 4 μl |
| 10x Nucleics Mix | 2 μl |
| RNase-free water | 7 μl |
| Reverse Transcriptase Mix | 2 μl |
| Template RNA | 5 μl (= 350 ng standard siRNA) |

In addition to the recovered siRNA also the following controls were used:

1. 10 pmol siRNA digested with benzonase in 100 μl Tris buffer
2. 10 pmol siRNA extracted by phenol-chloroform-extraction and digested with benzonase 100 μl Tris-Puffer
3. empty VLPs to exclude a reaction with insect cell DNA

The 96 well plate was then incubated to 60 min at 37 °C for reverse transcription and then 5 min at 95 °C to inactivate the reverse transcriptase and separate the RNA and DNA strands.

PCR was performed in BR clear 96-well PCR plates (Greiner).

The following PCR mixture was used:

| | |
|---|---|
| 2x Quantitect SYBR Green PCR Master Mix | 12.5 μl |
| 10 x miSript Universal Primer | 2 μl |
| siRNA-specific fw Primer (5 μM) (SEQ ID NO: 75) | 2 μl |
| RNase-free water | 7.5 μl |
| 1 μl cDNA (from RT reaction) | 1 μl. |

For PCR, the following temperature profile was used: An initial activation at 95 °C for 15 minutes followed by 40 repetitions of the following cycle steps 1 to 3: 1) Denaturation: 95 °C for 15 seconds; 2) Annealing: 55 °C for 30 seconds; and 3) Extension: 70 °C for 30 seconds. After the temperature cycling the samples were again heated for at 95 °C for 10 seconds, cooled to 55 °C for 5 seconds heated again to 95 °C until the samples were retrieved.

The RNA protection in % is calculated as described in Example 2 for DNA protection. The results are shown in Fig. 6. From the Fig. 6 it is evident that VLPs build from VP1 and VP2fusion proteins of with protamine-1, or penetratin provide a better RNA protection than VLPs build VP1 and wild type VP2 or VP1 alone.

**EXAMPLE 6: Transduction of cells mediated by VLPs with VP2/VP3-CPP fusion proteins**

The assay is based on the principle that DNA encoding for luciferase is transduced into cells using the VLPs as transport system. Luciferase-dependent chemo luminescence in the cells is measured afterwards. The higher the chemo luminescent signal, the more DNA is introduced into the cells. Two different types of DNA were used as cargo:

a. pGl4.15
b. pNL1.1_CMV

The two DNA plasmids can be used in different luciferase assay systems, namely, the luciferase assay system of Promega with pGL4.51 and Nano-GLO™ Luciferase assay for pNL1.1_CMV.

1. VP1
2. VP1_VP2-L2DD447p
3. VP1_VP2-TATp
4. VP1_VP2-PENp
5. VP1_VP2-HISp
6. Nanoluc-Vector

***a) Packaging of DNA into VLPs***

The DNA was packaged into the different VLPs, according to the protocol of example 3.

***b) DNAse treatment on packaged DNA***

The DNAse treatment was carried out, according to the protocol of example 3.

***c) Transduction of cells with DNA containing VPLs***

COS7 cells were grown in 24 well plates. For this, cells were inoculated into 500 μl of DMEM+ medium (10% FCS, 1% penicillin/streptomycin). The cells were incubated at 37 °C shaking. Upon reaching a density 5 x $10^4$ cells per well, 25 μg VLP with or without DNasel (see step 2) were added to the cells and the cells were further incubated for 48 hours at 37°C.

After incubation, the DMEM+ medium was decanted and cells were washed two times with 1x PBS.

***d) A measurement of the luciferase expression in the cellular extracts.***

The transduced COS7 cells were incubated with 100 μl 1x Lysis buffer for 10 minutes at room temperature.

Composition of the Luciferase Cell Culture Lysis Reagent (1X)

25mM Tris-phosphate (pH 7.8)
2mM DTT
2mM 1,2-diaminocyclohexane-N,N,N,N-tetraacetic acid
10% glycerol
1% Triton® X-100

The cell lysates were spun in a centrifuge for 1 minute at 11.000 g and afterwards 20 μl of the supernatant were transferred into a well of a white 96 well plate (Nunc). At this stage, in case of the Nano-Glo Luciferase Assay, 20 μl of Nanoluc reagent (0.5 ml combi tip) were added and incubated for 3 minutes at room temperature. Afterwards, the signal strength in relative lights units (RLU) was determined. Alternatively, in case of the luciferase assay system (Glo-Max®, Promega), 100 μl substrate Luciferin were added to the 20 μl lysate, incubated for 10 sec and the signal strength in relative lights units (RLU) was determined.

**Fig. 7** shows a graph with the signal intensities determined for each of the samples represented in relative light units (RLUs). The first sample NT is a negative control, wherein no DNA was transferred into the cells and thus represents a measure of the background signal. The signal is slightly below 100 RLU.

Using only the Nanoluc vector without a VLP transportation system (6) results in a similar signal. Thus, no DNA is transferred into the cells. The transduction experiment with VLPs consisting of only VP1 also resulted in a signal strength of about 100 RLU for both the DNase treated and the untreated sample.

In contrast, all VP2-ETP fusion proteins led to a drastic increase of DNA transduction into the COS7 cells. While the DNase treated samples of the VLPs with the VP2 fusion proteins VP2-L2DD447p (2), VP2-TATp (3), VP2-PENp (4), VP2-HISp (5) had a signal in the range of 100 relative light units when treated with DNase. However, the DNase-untreated samples led to a signal strength in the range of 100,000 RLU for VP2-L2DD447p (2) and VP2-TATp (3). The signal strength of the penetratin fusion protein VP2-PENp (4) was 10,000 RLU and 3,000 RLU were determined for the his-tagged VP2 (5). These results show that VP2 fusion proteins with a peptide from the class of ETPs leads to a drastic increase in the transduction of COS7 cells.

**EXAMPLE 7: Transduction of cells mediated by VLPs with VP2/VP3 fusion proteins**

The Experiment according to example 6 was repeated with VLPs derived from the following constructs: VP1-VP2, VP1-VP2-protamine-1, VP1-VP2-L2DD447 and VP1-VP2-pentratin and VP1-VP2/VP1-VP2-protamine-1 in a ratio of 5:1. In contrast to Example 6 no control experiments with digested DNA were carried out. As negative controls served again cells that were not transduced or only transduced with the plasmid.

The results of the experiment are shown in **Fig. 8. Fig. 8** shows a graph with the signal intensities determined for each of the samples represented in relative light units (RLUs). The two negative controls have a similar signal in the below 100 RLU. The VLP from VP1 and VP2 wildtype lead to a signal just above 100 RLU: VLP containing the VP2-protamine-1, VP2-L2DD447 or VP1-VP2-pentratin fusion protein show a significantly increased RLU value and thus DNA transduction. Similar to the result in Example 6, the VLPs with a reduced number of VP2-protamine-1 (VP1-VP2/VP1-VP2-protamine in a ratio of 5:1) exhibit a higher RLU signal than the VLPs derived from VP1-VP2-protamine-1.

**EXAMPLE 8: siRNA protection by VLPs blood plasma treatment.**

VLPs with VP1 and VP2-PENp were produced according to the protocol of Example 1. The siRNA protection test including siRNA packaging, detection and quantification was performed according Example 5. In Fig. 9 A the number of molecules detected in the sample without benzonase treatment (1) and the sample with benozonase treatment (2) are shown. The number of molecules per milliliter ($N_{mol}$/μl) are in both cases almost identical. Thus the siRNA protection in this case is almost 100 %.

In a parallel experiment, the benzonase treatment step of Example 5 is replaced by an incubation of the RNA in 500 μl blood plasma at 37°C. During incubation samples were retrieved at the following time points: 15 min, 30 min, 60 min and 120 min. As a control, 10 pmol of unpackaged siRNA were analyzed for 30 min in 500 μl blood plasma at 37°C. The result is shown in **Fig. 9 B.** Accordingly, the signal strength representing the siRNA concentration decreases within the first 30 min from $10^{12}$ RLU auf $10^{8}$ RLU, but stays constant from then on to the last measured time point at 120 min. In contrast, no signal could be detected in the control experiment.

**EXAMPLE 9: Assessment of functionality of the protected siRNA**

**Steps**

1) Packaging of siRNA using VLP
2) Benzonase treatment packaged siRNA
3) Phenol/chloroform extraction of nucleic acids
4) Proliferation assay and transfection of siRNA

Steps 1 to 3 were carried out as described in example 5 (steps b) to d)). The siRNA was Kif11_8 siRNA (Qiagen). The VLPs were produced as described in example 1 with VP1 and VP2-Penetratin.

**Step 4**

TC-620 cells were diluted to a concentration of 2.0 x $10^{5}$ cells/ml in DMEM-HG FCS medium.

100 μl culture medium was applied into the wells a microtiter plate that incorporates a sensor electrode array (E-plate, ACEA Biosciences). After 15 min 50 μl the TC-620 cell suspension was added.

The proliferation of the cells is measured in the xCelligence equipment (ACEA Biosciences) for 20 h under humidified conditions at 37°C and 5% $CO_2$. The system measures the proliferation based on the following principle: The presence of the cells on top of the electrodes will affect the local ionic environment at the electrode/solution interface, leading to an increase in the electrode impedance. The more cells are attached on the electrodes, the larger the increases in electrode impedance. In addition, the impedance depends on the quality of the cell interaction with the electrodes. For example, increased cell adhesion or spreading will lead to a larger change in electrode impedance. The output is a proliferation index based on the electrode impedance.

For siRNA transfection a RNAi-Max/Opti-MEM® mastermix was prepared by mixing of 1 ml OptiMEM (Gibco) with 10 μl RNAi-Max (Life Technologies).

Three test samples were prepared:

1) Media control: 50 μl Opti-MEM® (Life Technologies)
2) VLP extracted siRNA: 2 pmol of siRNA from step 3, diluted in 50 μl OptiMEM,
3) Untreated siRNA: 2 pmol of untreated If11_8 siRNA diluted in 50 μl OptiMEM,

50 μl of the RNAi-Max/Opti-MEM® mastermix were added to each the test samples and incubated for 15 min at room temperature.

The xCelligence equipment is paused and 50 μl of the test sample/mastermix solution is applied to the wells of the E-Plates.

The assay was run for additional 96 h.

**Results:**

Fig. 10 shows the result of this experiment. The curves represent the proliferation rate of the EPN target cell TC-620.The X axis represents the time of the experiment in hours (h) and the Y-axis represents proliferation index.

Curve 1 relates to sample 1 ("Media control") and displays standard proliferation index curve representing a normal proliferation rate of the target cell. In the curve 1 the proliferation index constantly rises before and after addition sample befor reaching a plateau at about 85 h.

In contrast in curves 2 and 3, corresponding the siRNA treated samples, the proliferation index already reaches a plateau within about 10 h of addition of the siRNA Afterwards the value of the proliferation index constantly decreases. The reason is that the siRNA targets Kif11/Eg5 which is involved in cell division processes. In comparison siRNA protected against nucleases by VLP with a fusion protein according to the invention show nearly a comparable efficiency - just a small delay in the knock down kinetics - as the untreated one if the siRNA. This experiment shows the efficient functional protection of the siRNA by EPN against external influences.

**EXAMPLE 10: Transduction of siRNA into cells and test of functionality**

**Steps:**

1) Packaging of siRNA using VLP
2) Proliferation assay and transduction of siRNA by VLP

**Step** 1

Kif11_8 siRNA was packaged in VLPs from VP1 and VP2-Penetratin as described in example 5 step b).

**Step 2**

TC-620 cells were diluted to a concentration of 2.0 x $10^5$ cells/ml in DMEM-HG FCS medium.

100 μl culture medium was applied into the wells a microtiter plate that incorporates a sensor electrode array (E-plate, ACEA Biosciences). After 15 min 50 μl of the TC-620 cell suspension was added.

The proliferation of the cells is measured in the xCelligence equipment (ACEA Biosciences) for 20 h under humidified conditions at 37°C and 5% $CO_2$.

After 20 h the following samples were added to different wells of the E-plate:

1) 50 μl OptiMEM (media control)
2) 50 μl Reassociation Buffer
3) Empty VLP in 50 μl Reassociation Buffer
4) VLP with siRNA in 50 μl Reassociation Buffer
5) VLP with siRNA in 50 μl Reassociation Buffer

The assay was run for an additional 114 h.

**Results**

Fig. 11 shows the result of this experiment. The curves represent the proliferation rate of the TC-620 in the E-plates. Curve 1 (media control) displays the normal proliferation rate of the target cell shown 20 hours before and 114 hours after the compound application. Curve 2 (Reassociation buffer) shows a slightly reduced increase in the proliferation index after about 80 h compared the media control sample (curve 1). Accordingly the VLP delivery solution influences the proliferation negatively. The empty VLP (curve 3) shows a reduced proliferation rate already after about 50 h and thus provides a weak cytostatic effect. The encapsulated siRNA against Kif11/Eg5, a proliferation associated protein, demonstrate in two independent preparation the delivery of the siRNA VLP containing VP1 and VP2-PENp (curves 4 and 5). This leads to a cytostatic effect after 48 h hours and a cytotoxic one after 94 hours. At this time point, the cells begin to die, entering the apoptotic pathway which is induced by the down regulation of Kif11/Eg5. This experiment illustrates the delivery efficacy of VLP according to the invention (VP1-VP2-PENp) using the example of the active component class of siRNA’s.

## Claims

1. A fusion protein comprising a VP1 binding protein and an exogenous peptide, wherein the exogenous peptide comprises a cargo-securing peptide (CSP) and/or an endosome translocating peptide (ETP).

2. The fusion protein according to claim 1, wherein the CSP is not GFP and/or the ETP is not a His-Tag.

3. The fusion protein according to claim 1 or 2, wherein the VP1 binding protein is VP2 or VP3.

4. The fusion protein according to claim 1 to 3, wherein the VP1 binding protein has a sequence identity of at least 80 %, preferably at least 90 %, more preferably of at least 95 % to SEQ ID NO: 1 (VP2) or SEQ ID NO: 2 (VP3).

5. The fusion protein according to any of claims 1 to 4, wherein the exogenous peptide forms the C-terminus and/or the N-terminus of the fusion protein.

6. The fusion protein according to any of claims 1 to 5, wherein the amino acid sequence of the CSP or ETP has a percentage of basic amino acids of at least 25, preferably at least 30.

7. The fusion protein according to any of claims 1 to 5, wherein the CSP is a cargo binding peptide (CBP) and preferably has a percentage of arginine residues of at least 25, preferably at least 30, more preferably at least 35, most preferably at least 40.

8. The fusion protein according to claim 7, wherein the amino acid sequence of the CBP has an identity of at least 80 %, preferably at least 90 %, more preferably of at least 95 % to SEQ ID NO: 4, or SEQ ID NO: 5.

9. The fusion protein according to any of claims 1 to 5, wherein the ETP is a cell penetrating peptide (CPP) and preferably the amino acid sequence of the CPP has a percentage of nonpolar amino acids of at least 25, preferably of at least 30, more preferably of at least 35.

10. The fusion protein according to any of claims 9, wherein the amino acid sequence of the CPP has an identity of at least 80 %, preferably at least 90 %, more preferably of at least 95 % to SEQ ID NO: 6, SEQ ID NO: 7, SEQ ID NO:

8, or SEQ ID NO: 9.

**11.** The fusion protein according to any of claims 1 to 5, wherein the fusion protein comprises at least one exogenous CBP, according to claim 6 or 7, and at least one exogenous CPP, according to claim 8 or 9.

**12.** A virus-like particle from a polyomavirus, comprising a fusion protein according to any of claims 1 to 11.

**13.** The VLP according to claim 12, further comprising a VP1 fusion protein with first and a second peptide,

- wherein the first peptide is VP1 or a fragment thereof and
- the second peptide comprises a targeting region and a first and a second interaction region,
- the second peptide is located on the surface of the fusion protein,
- the second peptide comprises at least two interaction pairs, wherein an interaction pair is formed by an amino acid of the first interaction region and an amino acid of the second interaction region,
- the interaction region between the amino acid of an interaction pair is covalent or non-covalent, and
- at least one interaction pair is a covalent interaction pair in which the amino acids are covalently bound.

**14.** The VLP according to claim 12 or 13, wherein VLP comprises no cargo.

**15.** The VLP according to claim 12 or 13, wherein VLP comprises a cargo selected from single-stranded or double-stranded DNA or RNA, preferably siRNA, oligopeptides, polypeptides, hormones, lipids, carbohydrates, other small organic compounds or mixtures thereof.

**16.** The VLP according any of claims 15 for use as in treatment or diagnosis of a disease.

**17.** The VLP according to claim 15 or 16, for use as drug delivery system.

**18.** A pharmaceutical composition comprising at least one fusion protein according to any one of claims 1 to 11.

**19.** The pharmaceutical composition according to claim 16, comprising at least one VLP according to any one of claims 12 to 15, and at least one pharmaceutically acceptable carrier.

**20.** A polynucleotide comprising a nucleic acid sequence encoding a fusion protein according to any claims 1 to 11.

**21.** A vector comprising a polynucleotide according to claim 20.

**22.** A host cell comprising the vector according to claim 21.

**23.** A process of producing the VLP according to any of claims 12 to 15, comprising the steps of:

a) introducing a polynucleotide according to claim 20 into a host cell;
b) culturing the transformed cell in a medium under conditions leading to a protein expression with the nucleic acid as a template;
c) isolating the expression product; and
d) assembly of a VLP with the expression product VP1.

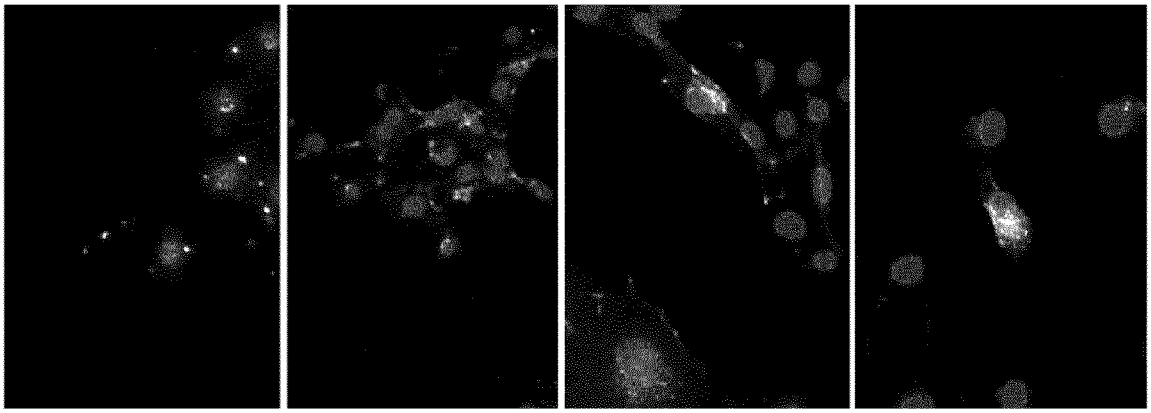

Fig. 1

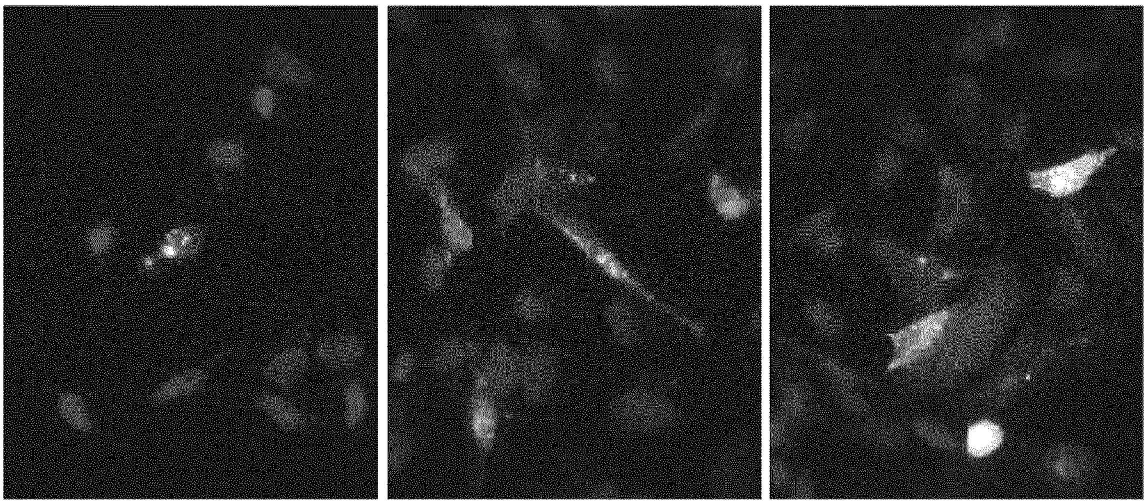

Fig. 2

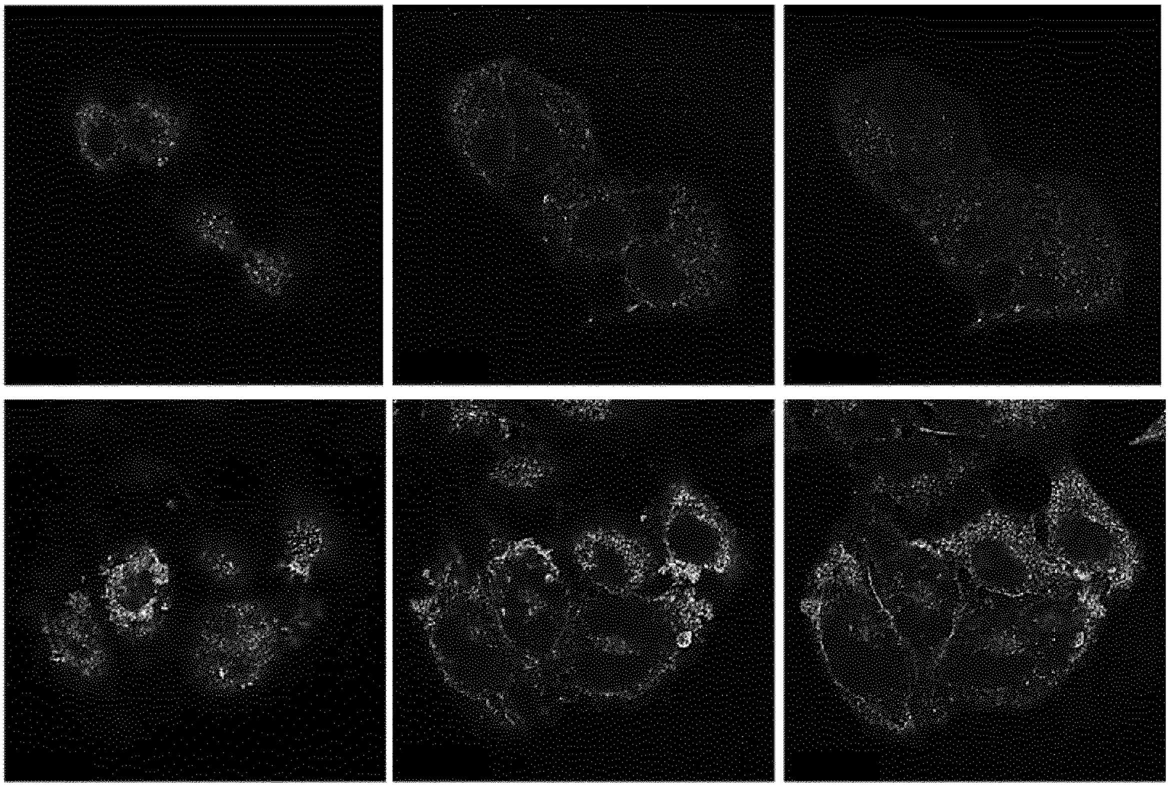

Fig. 3

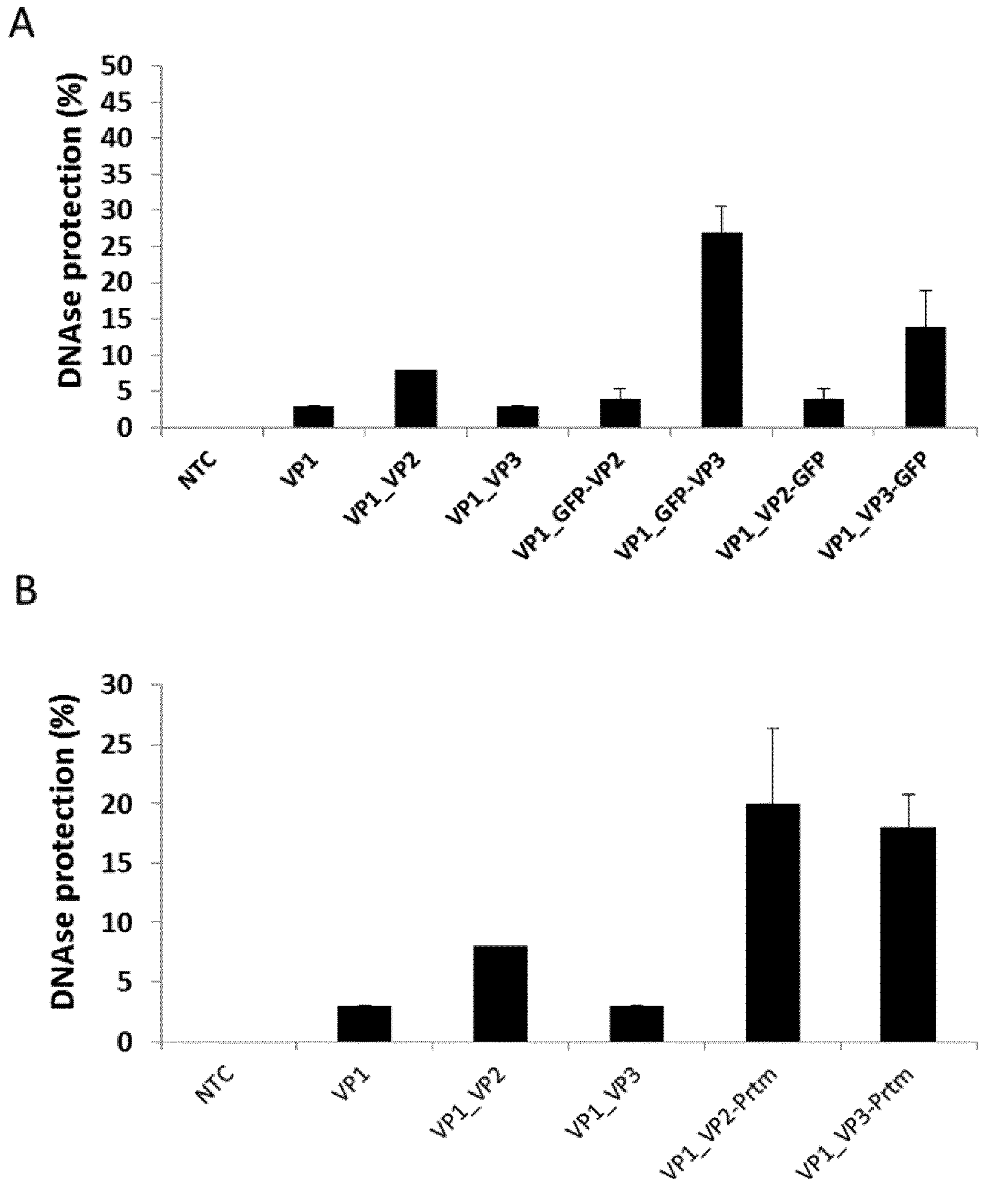
A
DNAse protection (%)
50
45
40
35
30
25
20
15
10
5
0
NTC
VP1
VP1_VP2
VP1_VP3
VP1_GFP-VP2
VP1_GFP-VP3
VP1_VP2-GFP
VP1_VP3-GFP
B
DNAse protection (%)
30
25
20
15
10
5
0
NTC
VP1
VP1_VP2
VP1_VP3
VP1_VP2-Prtm
VP1_VP3-Prtm

Fig. 4

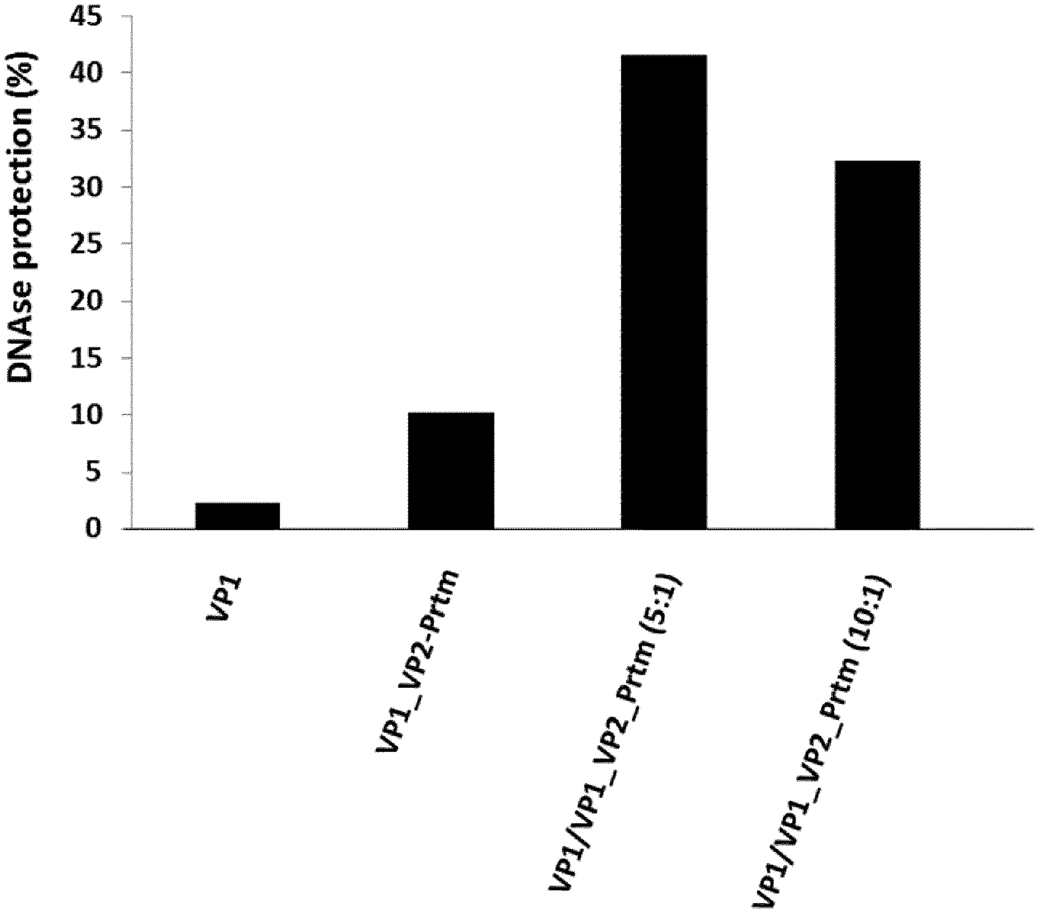
DNAse protection (%)
45
40
35
30
25
20
15
10
5
0
VP1
VP1_VP2-Prtm
VP1/VP1_VP2_Prtm (5:1)
VP1/VP1_VP2_Prtm (10:1)

Fig. 5

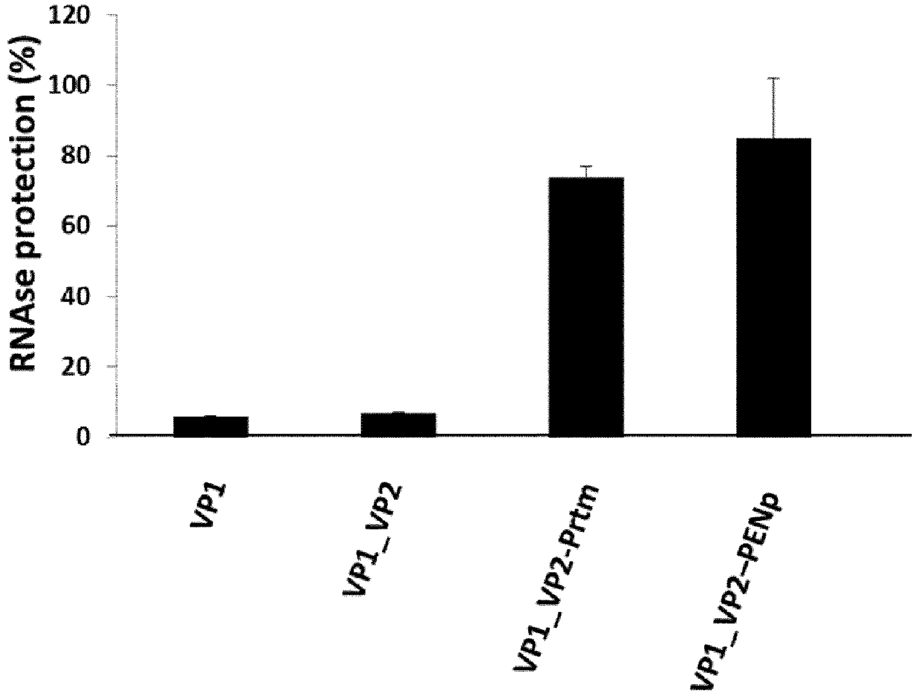
RNAse protection (%)
120
100
80
60
40
20
0
VP1
VP1_VP2
VP1_VP2-Prtm
VP1_VP2-PENp

Fig. 6

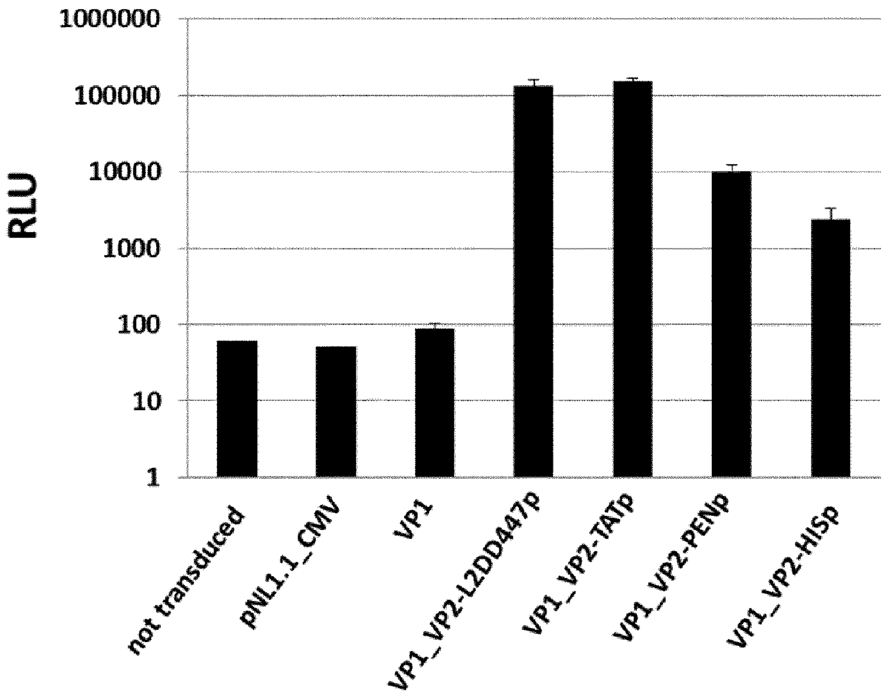
RLU
1000000
100000
10000
1000
100
10
1
not transduced
pNL1.1_CMV
VP1
VP1_VP2-L2DD447p
VP1_VP2-TATp
VP1_VP2-PENp
VP1_VP2-HISp

Fig. 7

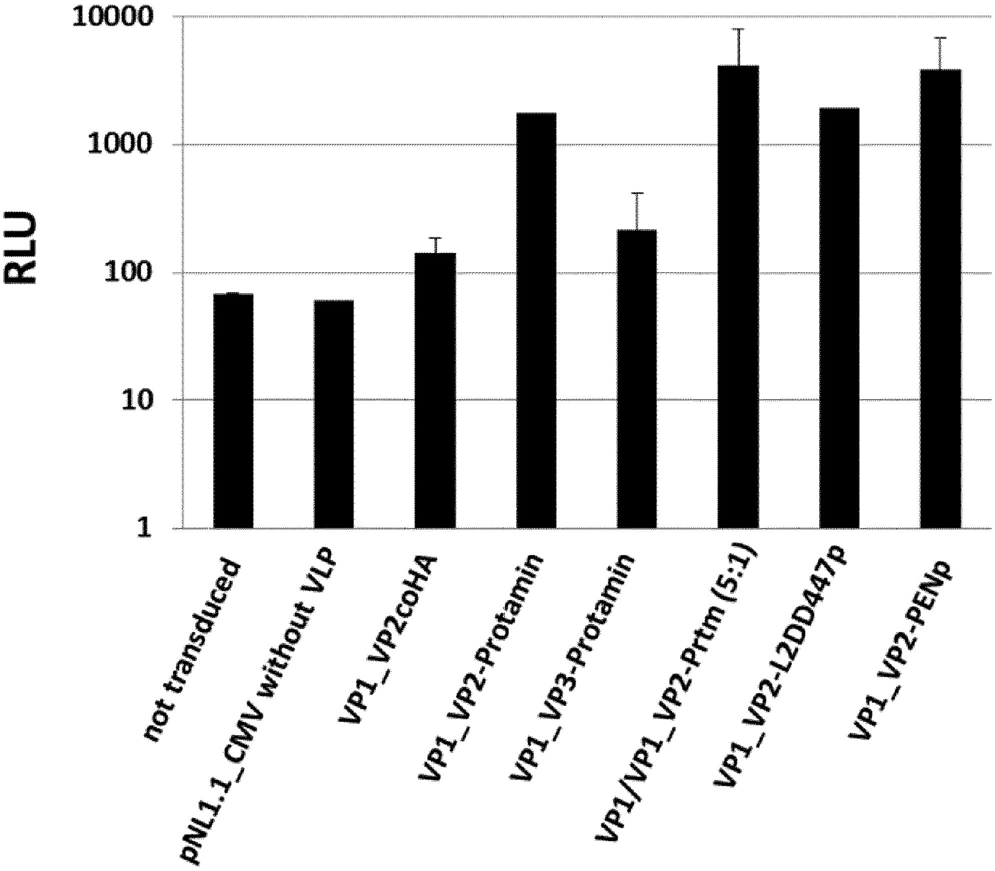
10000
1000
100
10
1
RLU
not transduced
pNL1.1_CMV without VLP
VP1_VP2coHA
VP1_VP2-Protamin
VP1_VP3-Protamin
VP1/VP1_VP2-Prtm (5:1)
VP1_VP2-L2DD447p
VP1_VP2-PENp

Fig. 8

**A**

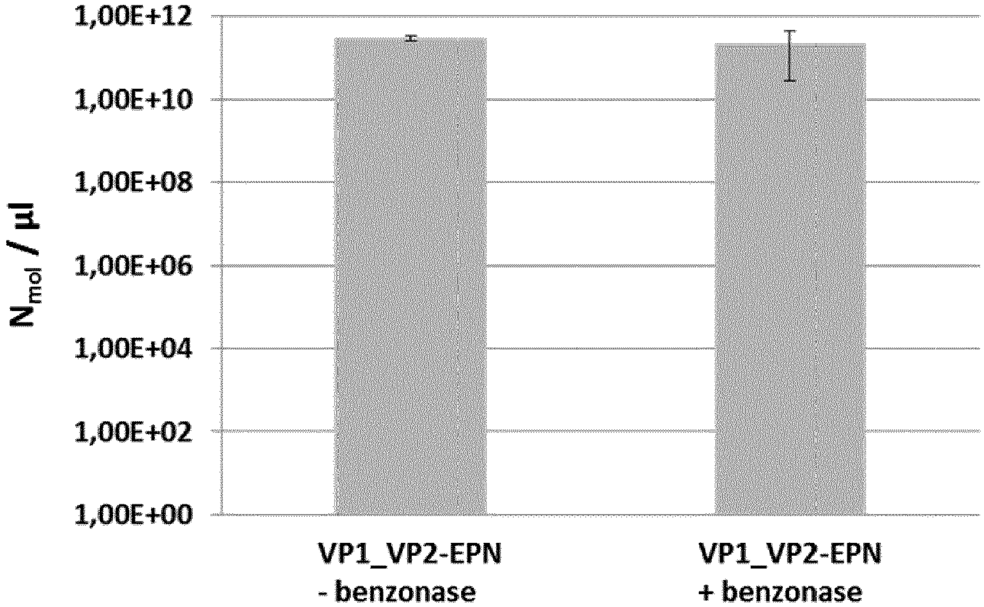
1,00E+12
1,00E+10
1,00E+08
1,00E+06
1,00E+04
1,00E+02
1,00E+00
$N_{mol}$ / µl
VP1_VP2-EPN
- benzonase
VP1_VP2-EPN
+ benzonase

**B**

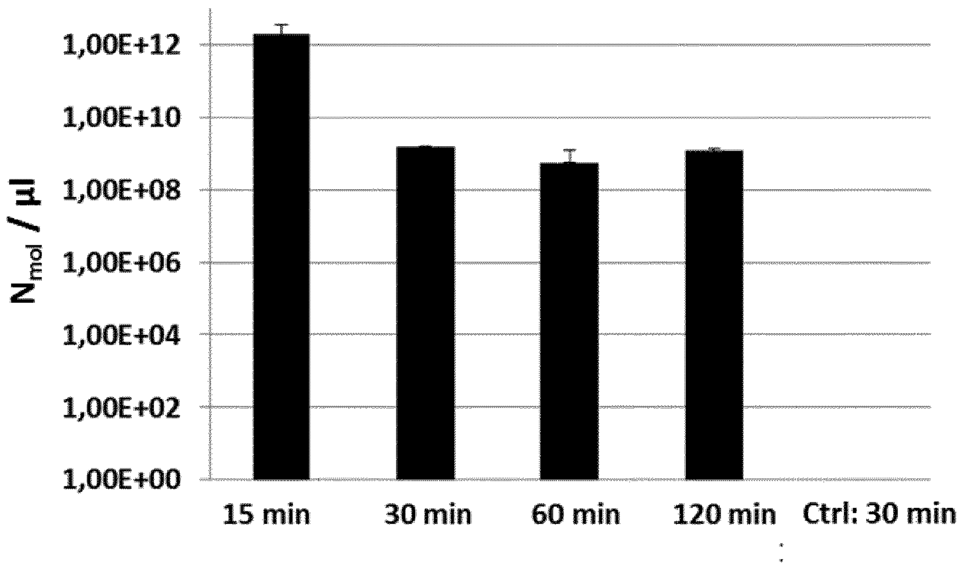
1,00E+12
1,00E+10
1,00E+08
1,00E+06
1,00E+04
1,00E+02
1,00E+00
$N_{mol}$ / µl
15 min
30 min
60 min
120 min
Ctrl: 30 min

Fig. 9

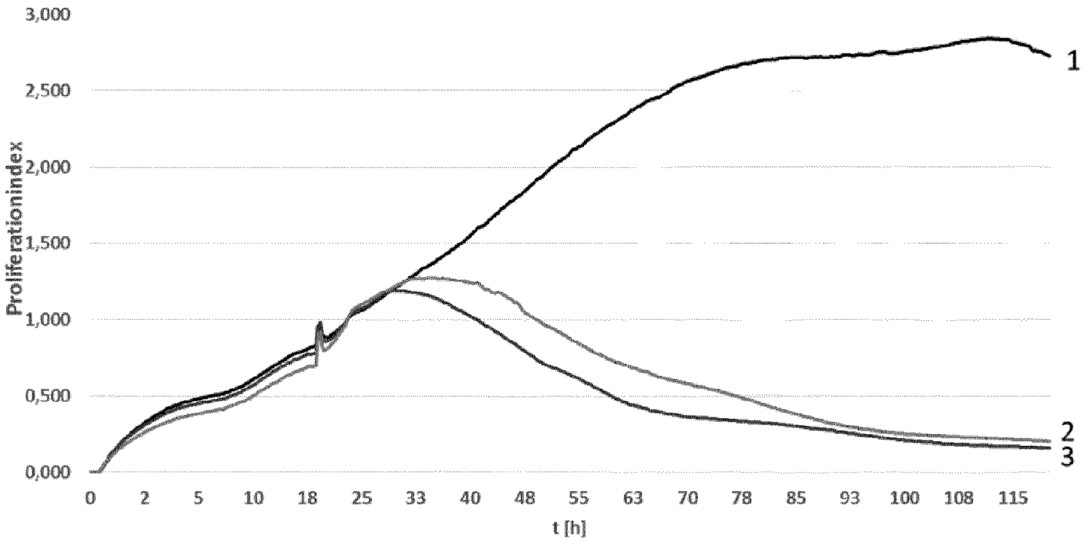
3,000
2,500
2,000
1,500
1,000
0,500
0,000
Proliferationindex
0
2
5
10
18
25
33
40
48
55
63
70
78
85
93
100
108
115
t [h]
1
2
3

Fig. 10

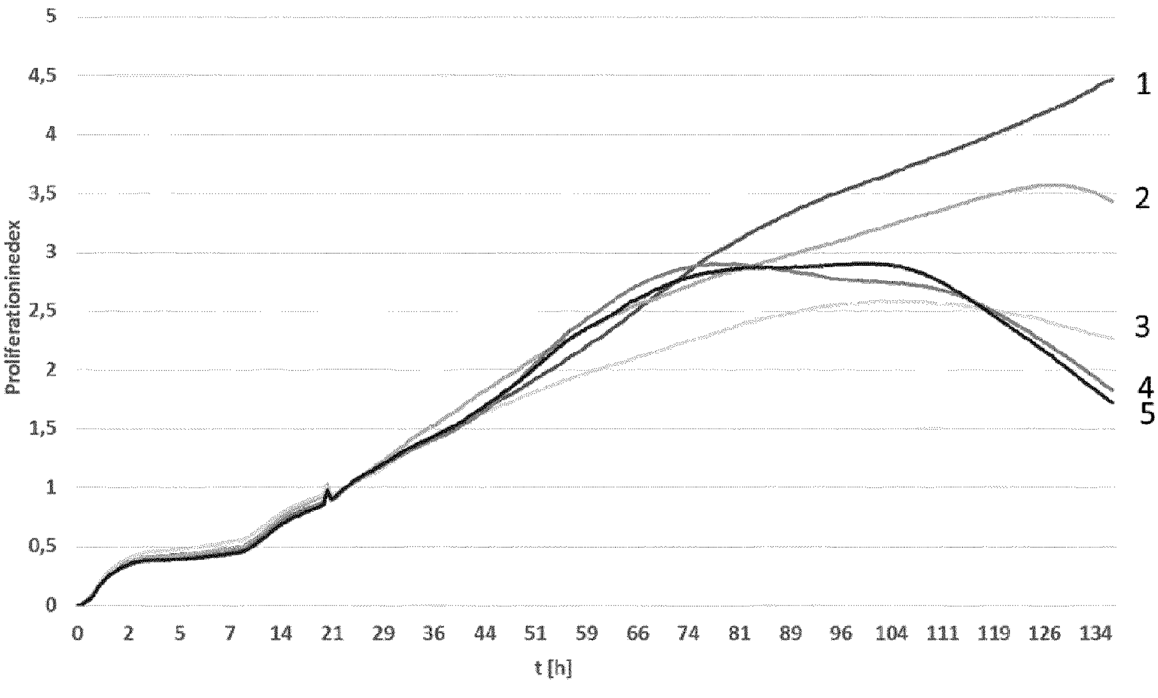
5
4,5
4
3,5
3
2,5
2
1,5
1
0,5
0
Proliferationinedex
0
2
5
7
14
21
29
36
44
51
59
66
74
81
89
96
104
111
119
126
134
t [h]
1
2
3
4
5

Fig. 11

| | 1 | 2 - 12 |
|---|---|---|
| A | 2 µg | ⊕Ctrl 51 aus EPN- 4-027<br>2E8/ml Ery's vom 13.05.14/eingestellt 15.05.14 |
| B | 2 µg | ⊕Ctrl 51 Citrat gefällt aus EPN-3-132<br>2E8/ml Ery's vom 13.05.14/eingestellt 15.05.14 |
| C | 2 µg | 5 PBS aus EPN- 1-185<br>2E8/ml Ery's vom 13.05.14/eingestellt 15.05.14 |
| D | 2 µg | 165 aus EPN- 3-189<br>2E8/ml Ery's vom 13.05.14/eingestellt 15.05.14 |
| E | 2 µg | 5/168 aus EPN- 1-186<br>2E8/ml Ery's vom 13.05.14/eingestellt 15.05.14 |
| F | 2 µg | 5/176 aus EPN- 1-186<br>2E8/ml Ery's vom 13.05.14/eingestellt 15.05.14 |
| G | | ⊖Ctrl<br>2E8/ml Ery's vom 13.05.14/eingestellt 15.05.14 |
| H | | Leer |

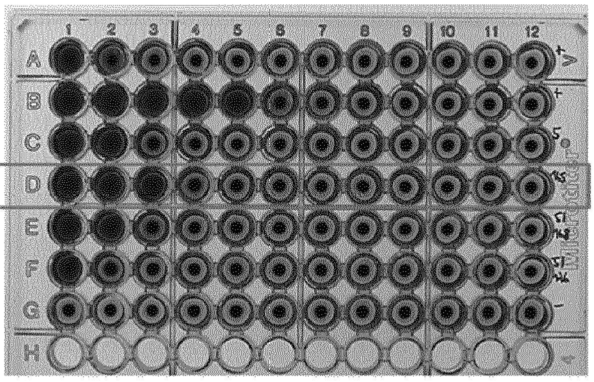

Fig. 12

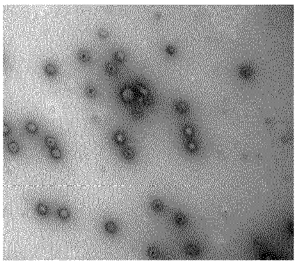

Fig. 13

Europäisches Patentamt
European Patent Office
Office européen des brevets

# EUROPEAN SEARCH REPORT

Application Number
EP 14 19 6847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TEUNISSEN ERIK A ET AL: "Production and biomedical applications of virus-like particles derived from polyomaviruses", JOURNAL OF CONTROLLED RELEASE, vol. 172, no. 1, 31 August 2013 (2013-08-31), pages 305-321, XP028772934, ISSN: 0168-3659, DOI: 10.1016/J.JCONREL.2013.08.026 * paragraph [0005] - paragraph [0007] * ----- | 1-7, 12-23 | INV. C07K14/005 C12N7/04 |
| X | BOURA E ET AL: "Polyomavirus EGFP-pseudocapsids: Analysis of model particles for introduction of proteins and peptides into mammalian cells", FEBS LETTERS, ELSEVIER, AMSTERDAM, NL, vol. 579, no. 29, 5 December 2005 (2005-12-05), pages 6549-6558, XP027697502, ISSN: 0014-5793 [retrieved on 2005-12-05] | 1-7,12, 14-23 | |
| Y | * the whole document * ----- | 13-17 | TECHNICAL FIELDS SEARCHED (IPC) |
| X | TEGERSTEDT K ET AL: "MURINE POLYOMAVIRUS VIRUS-LIKE PARTICLES (VLPS) AS VECTORS FOR GENE AND IMMUNE THERAPY AND VACCINES AGAINST VIRAL INFECTIONS AND CANCER", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 25, no. 4, July 2005 (2005-07), pages 2601-2608, XP009068573, ISSN: 0250-7005 * page 2606, right-hand column, last paragraph - page 2607, last paragraph * ----- -/-- | 1-5,12, 18-23 | C07K C12N |

The present search report has been drawn up for all claims

| Place of search | Date of completion of the search | Examiner |
|---|---|---|
| The Hague | 26 May 2015 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

Europäisches Patentamt
European Patent Office
Office européen des brevets

# EUROPEAN SEARCH REPORT

Application Number
EP 14 19 6847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ABBING A ET AL: "Efficient intracellular delivery of a protein and a low molecular weight substance via recombinant polyomavirus-like particles", JOURNAL OF BIOLOGICAL CHEMISTRY, AMERICAN SOCIETY FOR BIOCHEMISTRY AND MOLECULAR BIOLOGY, US, vol. 279, no. 26, 25 June 2004 (2004-06-25), pages 27410-27421, XP002354397, ISSN: 0021-9258, DOI: 10.1074/JBC.M313612200 * the whole document * ----- | 1,3,4, 12,18-23 | |
| Y | GLEITER STEFAN ET AL: "Coupling of antibodies via protein Z on modified polyoma virus-like particles", PROTEIN SCIENCE, WILEY, US, vol. 10, no. 2, February 2001 (2001-02), pages 434-444, XP002488028, ISSN: 0961-8368, DOI: 10.1110/PS.31101 * the whole document * ----- | 13-17 | TECHNICAL FIELDS SEARCHED (IPC) |
| Y | GLEITER S ET AL: "Changing the surface of a virus shell fusion of an enzyme to polyoma VP1", PROTEIN SCIENCE, WILEY, US, vol. 8, no. 12, December 1999 (1999-12), pages 2562-2569, XP002354398, ISSN: 0961-8368 * the whole document * ----- | 13-17 | |
| A,D | EP 1 270 586 B1 (JENAPHARM GMBH [DE] FORSCHUNGSZENTRUM FUER MEDIZIN [DE]) 29 November 2006 (2006-11-29) * the whole document * ----- -/-- | 1-23 | |

1

The present search report has been drawn up for all claims

| Place of search | Date of completion of the search | Examiner |
|---|---|---|
| The Hague | 26 May 2015 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 6847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 636 746 A1 (LIFE SCIENCE INKUBATOR [DE]) 11 September 2013 (2013-09-11)<br>* the whole document *<br>----- | 1,12, 18-23 | |
| A | EP 2 774 991 A1 (LIFE SCIENCE INKUBATOR BETR S GMBH & CO KG [DE]) 10 September 2014 (2014-09-10)<br>* the whole document *<br>----- | 1,12,18, 23 | |
| A | US 2010/322862 A1 (RUOSLAHTI ERKKI [US] ET AL) 23 December 2010 (2010-12-23)<br>* paragraphs [0029] - [0033], [0101] - [0146]; claims 1,2, 98-106,124--156,158 *<br>----- | 1 | |
| A | JASON DEROUCHEY ET AL: "A Comparison of DNA Compaction by Arginine and Lysine Peptides: A Physical Basis for Arginine Rich Protamines",<br>BIOCHEMISTRY,<br>vol. 52, no. 17,<br>30 April 2013 (2013-04-30), pages 3000-3009, XP055191551,<br>ISSN: 0006-2960, DOI: 10.1021/bi4001408<br>* the whole document *<br>----- | 1,6-8 | |
| A | BECHARA CHÉRINE ET AL: "Cell-penetrating peptides: 20years later, where do we stand?",<br>FEBS LETTERS, ELSEVIER, AMSTERDAM, NL,<br>vol. 587, no. 12, 10 May 2013 (2013-05-10), pages 1693-1702, XP028562950,<br>ISSN: 0014-5793, DOI: 10.1016/J.FEBSLET.2013.04.031<br>* paragraphs [2.2.1], [2.2.3]; table 1 *<br>-----<br>-/-- | 1,9,10 | TECHNICAL FIELDS SEARCHED (IPC) |

The present search report has been drawn up for all claims

1

| Place of search | Date of completion of the search | Examiner |
|---|---|---|
| The Hague | 26 May 2015 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 6847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YANG PAN ET AL: "Development of a microRNA delivery system based on bacteriophage?MS2 virus-like particles", FEBS JOURNAL, vol. 279, no. 7, 23 April 2012 (2012-04-23), pages 1198-1208, XP055159553, ISSN: 1742-464X, DOI: 10.1111/j.1742-4658.2012.08512.x * the whole document * ----- | 1,12 | |
| A | WEI B ET AL: "Development of an antisense RNA delivery system using conjugates of the MS2 bacteriophage capsids and HIV-1 TAT cell penetrating peptide", BIOMEDICINE AND PHARMACOTHERAPY, MAY 2009, ELSEVIER, FR, vol. 63, no. 4, 7 September 2008 (2008-09-07), pages 313-318, XP026019278, ISSN: 0753-3322, DOI: 10.1016/J.BIOPHA.2008.07.086 [retrieved on 2008-09-07] * the whole document * ----- | 1,9,10, 12,18 | TECHNICAL FIELDS SEARCHED (IPC) |
| A | JÄRVER P ET AL: "In vivo biodistribution and efficacy of peptide mediated delivery", TRENDS IN PHARMACOLOGICAL SCIENCES, 2010 NOVEMBER, ELSEVIER, HAYWARTH, GB, vol. 31, no. 11, 7 September 2010 (2010-09-07), pages 528-535, XP027431686, ISSN: 0165-6147, DOI: 10.1016/J.TIPS.2010.07.006 [retrieved on 2010-09-07] * tables 1,2 * ----- | 1 | |
| | -/-- | | |

1

The present search report has been drawn up for all claims

| Place of search | Date of completion of the search | Examiner |
|---|---|---|
| The Hague | 26 May 2015 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 6847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DONG-XU MA ET AL: "Distinct transduction modes of arginine-rich cell-penetrating peptides for cargo delivery into tumor cells", INTERNATIONAL JOURNAL OF PHARMACEUTICS, ELSEVIER BV, NL, vol. 419, no. 1, 2 August 2011 (2011-08-02), pages 200-208, XP028306630, ISSN: 0378-5173, DOI: 10.1016/J.IJPHARM.2011.08.001 [retrieved on 2011-08-09] * the whole document * ----- | 1,6-8 | |
| A | JAGU S ET AL: "Vaccination with multimeric L2 fusion protein and L1 VLP or capsomeres to broaden protection against HPV infection", VACCINE, ELSEVIER LTD, GB, vol. 28, no. 28, 17 June 2010 (2010-06-17), pages 4478-4486, XP027078067, ISSN: 0264-410X, DOI: 10.1016/J.VACCINE.2010.04.039 [retrieved on 2010-06-04] * the whole document * ----- | 1 | TECHNICAL FIELDS SEARCHED (IPC) |
| A | WO 02/16424 A2 (XZILLION GMBH & CO KG [DE]; KAPPEL ANDREAS [DE]; WINDHAB NORBERT [DE];) 28 February 2002 (2002-02-28) * claims * ----- | 1 | |

The present search report has been drawn up for all claims

1

| Place of search | Date of completion of the search | Examiner |
|---|---|---|
| The Hague | 26 May 2015 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 14 19 6847

This annex lists the patent family members relating to the patent documents cited in the above-mentioned European search report. The members are as contained in the European Patent Office EDP file on The European Patent Office is in no way liable for these particulars which are merely given for the purpose of information.

26-05-2015

| Patent document cited in search report | Publication date | Patent family member(s) | Publication date |
|---|---|---|---|
| EP 1270586 B1 | 29-11-2006 | AT 346859 T | 15-12-2006 |
| | | DE 10131145 A1 | 06-03-2003 |
| | | EP 1270586 A2 | 02-01-2003 |
| | | ES 2275781 T3 | 16-06-2007 |
| | | JP 2003061693 A | 04-03-2003 |
| | | NO 20022898 A | 30-12-2002 |
| | | US 2003044961 A1 | 06-03-2003 |
| EP 2636746 A1 | 11-09-2013 | AU 2013230232 A1 | 02-10-2014 |
| | | CA 2865564 A1 | 12-09-2013 |
| | | CN 104395475 A | 04-03-2015 |
| | | EP 2636746 A1 | 11-09-2013 |
| | | EP 2823048 A1 | 14-01-2015 |
| | | KR 20150003186 A | 08-01-2015 |
| | | US 2015045417 A1 | 12-02-2015 |
| | | WO 2013131644 A1 | 12-09-2013 |
| EP 2774991 A1 | 10-09-2014 | NONE | |
| US 2010322862 A1 | 23-12-2010 | CA 2766634 A1 | 13-01-2011 |
| | | CN 102869384 A | 09-01-2013 |
| | | EP 2445536 A1 | 02-05-2012 |
| | | JP 2012530787 A | 06-12-2012 |
| | | JP 2015044865 A | 12-03-2015 |
| | | KR 20120048563 A | 15-05-2012 |
| | | US 2010322862 A1 | 23-12-2010 |
| | | WO 2011005540 A1 | 13-01-2011 |
| WO 0216424 A2 | 28-02-2002 | AU 9177401 A | 04-03-2002 |
| | | CA 2420251 A1 | 28-02-2002 |
| | | DE 10041126 A1 | 04-04-2002 |
| | | EP 1313852 A2 | 28-05-2003 |
| | | US 2004072180 A1 | 15-04-2004 |
| | | WO 0216424 A2 | 28-02-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9719174 A
- EP 1270586 B1


### Non-patent literature cited in the description


- **NEEDLEMAN ; WUNSCH.** *J. Mol. Biol.,* 1970, vol. 48, 443-453
- **RICE.** EMBOSS: The European Molecular Biology Open Software Suite. *Trends Genet.,* 2000, vol. 16, 276-277
- **RICE.** *EMBOSS: The European Molecular Biology Open Software Suite,* 2000
- Remington The Science and Practice of Pharmacy. 2006, 745-775, 802-836, 837-849
- **SIMBERG D et al.** Biomimetic amplification of nanoparticle homing to tumors. *Proc Natl Acad Sci USA.,* 16 January 2007, vol. 104 (3), 932-6
- **KONDO et al.** Tumourlineage-homing cell-penetrating peptides as anticancer molecular delivery systems. *Nat Commun,* 17 July 2012, vol. 3, 951
- **CHRISTIAN et al.** Nucleolin expressed at the cell surface is a marker of endothelial cells in angiogenic blood vessels. *J Cell Biol.,* 24 November 2003, vol. 163 (4), 871-8
- **HAJDIN K et al.** Furin targeted drug delivery for treatment of rhabdomyosarcoma in a mouse model. *PLoS One,* 03 May 2010, vol. 5 (5
- **HERRINGSON ; ALTIN.** Effective tumor targeting and enhanced anti-tumor effect of liposomes engrafted with peptides specific for tumor lymphatics and vasculature. *Int J Pharm,* 15 June 2011, vol. 411 (1-2), 206-14
- **SHADIDI ; SIOUD.** Identification of novel carrier peptides for the specific delivery of therapeutics into cancer cells. *FASEB J.,* February 2003, vol. 17 (2), 256-8
- **INNIS et al.** PCR: A Guide to Methods and Application. Academic Press, 1991
- Protein Purification. VCH Publishers, 1989

SEQUENCE LISTING

<110> Life Science Inkubator Betriebs GmbH & Co. KG

<120> VLP with a fusion protein

<130> 55 322 K

<160> 89

<170> PatentIn version 3.5

<210> 1
<211> 344
<212> PRT
<213> JC-Polyomavirus

<400> 1

```
Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140

Ile His Tyr Leu Asp Pro Arg His Trp Gly Pro Ser Leu Phe Ser Thr
145                 150                 155                 160

Ile Ser Gln Ala Phe Trp Asn Leu Val Arg Asp Asp Leu Pro Ala Leu
                165                 170                 175
```

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser
340

<210> 2
<211> 225
<212> PRT
<213> JC-Polyomavirus

<400> 2

Met Ala Leu Gln Leu Phe Asn Pro Glu Asp Tyr Tyr Asp Ile Leu Phe
1 5 10 15

Pro Gly Val Asn Ala Phe Val Asn Asn Ile His Tyr Leu Asp Pro Arg
20 25 30

His Trp Gly Pro Ser Leu Phe Ser Thr Ile Ser Gln Ala Phe Trp Asn
35 40 45

Leu Val Arg Asp Asp Leu Pro Ala Leu Thr Ser Gln Glu Ile Gln Arg
50 55 60

Arg Thr Gln Lys Leu Phe Val Glu Ser Leu Ala Arg Phe Leu Glu Glu
65 70 75 80

Thr Thr Trp Ala Ile Val Asn Ser Pro Ala Asn Leu Tyr Asn Tyr Ile
85 90 95

Ser Asp Tyr Tyr Ser Arg Leu Ser Pro Val Arg Pro Ser Met Val Arg
100 105 110

Gln Val Ala Gln Arg Glu Gly Thr Tyr Ile Ser Phe Gly His Ser Tyr
115 120 125

Thr Gln Ser Ile Asp Asp Ala Asp Ser Ile Gln Glu Val Thr Gln Arg
130 135 140

Leu Asp Leu Lys Thr Pro Asn Val Gln Ser Gly Glu Phe Ile Glu Arg
145 150 155 160

Ser Ile Ala Pro Gly Gly Ala Asn Gln Arg Ser Ala Pro Gln Trp Met
165 170 175

Leu Pro Leu Leu Leu Gly Leu Tyr Gly Thr Val Thr Pro Ala Leu Glu
180 185 190

Ala Tyr Glu Asp Gly Pro Asn Lys Lys Lys Arg Arg Lys Glu Gly Pro
195 200 205

Arg Ala Ser Ser Lys Thr Ser Tyr Lys Arg Arg Ser Arg Ser Ser Arg
210 215 220

Ser
225

<210> 3
<211> 36
<212> PRT
<213> JC-Polyomavirus

<400> 3

Ser Gly Glu Phe Ile Glu Arg Ser Ile Ala Pro Gly Gly Ala Asn Gln
1 5 10 15

Arg Ser Ala Pro Gln Trp Met Leu Pro Leu Leu Leu Gly Leu Tyr Gly
20 25 30

Thr Val Thr Pro
        35

<210> 4
<211> 51
<212> PRT
<213> Homo sapiens (human)

<400> 4

Met Ala Arg Tyr Arg Cys Cys Arg Ser Gln Ser Arg Ser Arg Tyr Tyr
1               5                   10                  15

Arg Gln Arg Gln Arg Ser Arg Arg Arg Arg Arg Arg Ser Cys Gln Thr
            20                  25                  30

Arg Arg Arg Ala Met Arg Cys Cys Arg Pro Arg Tyr Arg Pro Arg Cys
        35                  40                  45

Arg Arg His
    50

<210> 5
<211> 21
<212> PRT
<213> Homo sapiens (human)

<400> 5

Ser Gln Ser Arg Ser Arg Tyr Tyr Arg Gln Arg Gln Arg Ser Arg Arg
1               5                   10                  15

Arg Arg Arg Arg Ser
            20

<210> 6
<211> 17
<212> PRT
<213> Human immunodeficiency virus (HIV)

<400> 6

Gly Arg Lys Lys Arg Arg Gln Arg Arg Arg Pro Pro Gln Lys Arg Lys
1               5                   10                  15

Gly

<210> 7
<211> 16
<212> PRT
<213> Antennapedia spec.

<400> 7

Arg Gln Ile Lys Ile Trp Phe Gln Asn Arg Arg Met Lys Trp Lys Lys
1               5                   10                  15

<210>  8
<211>  21
<212>  PRT
<213>  Human papillomavirus type 33 (HPV33)

<400>  8

Phe Ile Leu Arg Arg Arg Arg Lys Arg Phe Pro Tyr Phe Phe Thr Asp
1               5                   10                  15

Val Arg Val Ala Ala
            20

<210>  9
<211>  21
<212>  PRT
<213>  Human papillomavirus type 33 (HPV33)

<400>  9

Asp Asp Leu Arg Arg Arg Arg Lys Arg Phe Pro Tyr Phe Phe Thr Asp
1               5                   10                  15

Val Arg Val Ala Ala
            20

<210>  10
<211>  405
<212>  PRT
<213>  synthetic

<400>  10

Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln

85 90 95

Leu Gly Tyr Arg Phe Phe Ala Asp Trp Asp His Lys Val Ser Thr Val
100 105 110

Gly Leu Phe Gln Gln Pro Ala Met Ala Leu Gln Leu Phe Asn Pro Glu
115 120 125

Asp Tyr Tyr Asp Ile Leu Phe Pro Gly Val Asn Ala Phe Val Asn Asn
130 135 140

Ile His Tyr Leu Asp Pro Arg His Trp Gly Pro Ser Leu Phe Ser Thr
145 150 155 160

Ile Ser Gln Ala Phe Trp Asn Leu Val Arg Asp Asp Leu Pro Ala Leu
165 170 175

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

```
Arg Arg Ser Arg Ser Ser Arg Ser Tyr Pro Tyr Asp Val Pro Asp Tyr
            340                 345                 350

Ala Leu Glu Ala Arg Tyr Arg Cys Cys Arg Ser Gln Ser Arg Ser Arg
        355                 360                 365

Tyr Tyr Arg Gln Arg Gln Arg Ser Arg Arg Arg Arg Arg Arg Ser Cys
    370                 375                 380

Gln Thr Arg Arg Arg Ala Met Arg Cys Cys Arg Pro Arg Tyr Arg Pro
385                 390                 395                 400

Arg Cys Arg Arg His
                405
```

<210> 11
<211> 376
<212> PRT
<213> synthetic

<400> 11

```
Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140
```

Ile His Tyr Leu Asp Pro Arg His Trp Gly Pro Ser Leu Phe Ser Thr
145 150 155 160

Ile Ser Gln Ala Phe Trp Asn Leu Val Arg Asp Asp Leu Pro Ala Leu
165 170 175

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser Tyr Pro Tyr Asp Val Pro Asp Tyr
340 345 350

Ala Leu Glu Ser Gln Ser Arg Ser Arg Tyr Tyr Arg Gln Arg Gln Arg
355 360 365

Ser Arg Arg Arg Arg Arg Arg Ser
370 375

<210> 12
<211> 372
<212> PRT

<213> synthetic

<400> 12

```
Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140

Ile His Tyr Leu Asp Pro Arg His Trp Gly Pro Ser Leu Phe Ser Thr
145                 150                 155                 160

Ile Ser Gln Ala Phe Trp Asn Leu Val Arg Asp Asp Leu Pro Ala Leu
                165                 170                 175

Thr Ser Gln Glu Ile Gln Arg Arg Thr Gln Lys Leu Phe Val Glu Ser
            180                 185                 190

Leu Ala Arg Phe Leu Glu Glu Thr Thr Trp Ala Ile Val Asn Ser Pro
        195                 200                 205

Ala Asn Leu Tyr Asn Tyr Ile Ser Asp Tyr Tyr Ser Arg Leu Ser Pro
    210                 215                 220

Val Arg Pro Ser Met Val Arg Gln Val Ala Gln Arg Glu Gly Thr Tyr
225                 230                 235                 240
```

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser Tyr Pro Tyr Asp Val Pro Asp Tyr
340 345 350

Ala Val Asp Gly Arg Lys Lys Arg Arg Gln Arg Arg Arg Pro Pro Gln
355 360 365

Lys Arg Lys Gly
370

<210> 13
<211> 371
<212> PRT
<213> synthetic

<400> 13

Met Gly Ala Ala Leu Ala Leu Leu Gly Asp Leu Val Ala Thr Val Ser
1 5 10 15

Glu Ala Ala Ala Ala Thr Gly Phe Ser Val Ala Glu Ile Ala Ala Gly
20 25 30

Glu Ala Ala Ala Thr Ile Glu Val Glu Ile Ala Ser Leu Ala Thr Val
35 40 45

Glu Gly Ile Thr Ser Thr Ser Glu Ala Ile Ala Ala Ile Gly Leu Thr
50 55 60

Pro Glu Thr Tyr Ala Val Ile Thr Gly Ala Pro Gly Ala Val Ala Gly
65 70 75 80

```
Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140

Ile His Tyr Leu Asp Pro Arg His Trp Gly Pro Ser Leu Phe Ser Thr
145                 150                 155                 160

Ile Ser Gln Ala Phe Trp Asn Leu Val Arg Asp Asp Leu Pro Ala Leu
                165                 170                 175

Thr Ser Gln Glu Ile Gln Arg Arg Thr Gln Lys Leu Phe Val Glu Ser
            180                 185                 190

Leu Ala Arg Phe Leu Glu Glu Thr Thr Trp Ala Ile Val Asn Ser Pro
        195                 200                 205

Ala Asn Leu Tyr Asn Tyr Ile Ser Asp Tyr Tyr Ser Arg Leu Ser Pro
    210                 215                 220

Val Arg Pro Ser Met Val Arg Gln Val Ala Gln Arg Glu Gly Thr Tyr
225                 230                 235                 240

Ile Ser Phe Gly His Ser Tyr Thr Gln Ser Ile Asp Asp Ala Asp Ser
                245                 250                 255

Ile Gln Glu Val Thr Gln Arg Leu Asp Leu Lys Thr Pro Asn Val Gln
            260                 265                 270

Ser Gly Glu Phe Ile Glu Arg Ser Ile Ala Pro Gly Gly Ala Asn Gln
        275                 280                 285

Arg Ser Ala Pro Gln Trp Met Leu Pro Leu Leu Leu Gly Leu Tyr Gly
    290                 295                 300

Thr Val Thr Pro Ala Leu Glu Ala Tyr Glu Asp Gly Pro Asn Lys Lys
305                 310                 315                 320

Lys Arg Arg Lys Glu Gly Pro Arg Ala Ser Ser Lys Thr Ser Tyr Lys
                325                 330                 335
```

Arg Arg Ser Arg Ser Ser Arg Ser Tyr Pro Tyr Asp Val Pro Asp Tyr
340 345 350

Ala Val Asp Arg Gln Ile Lys Ile Trp Phe Gln Asn Arg Arg Met Lys
355 360 365

Trp Lys Lys
370

<210> 14
<211> 376
<212> PRT
<213> synthetic

<400> 14

Met Gly Ala Ala Leu Ala Leu Leu Gly Asp Leu Val Ala Thr Val Ser
1 5 10 15

Glu Ala Ala Ala Ala Thr Gly Phe Ser Val Ala Glu Ile Ala Ala Gly
20 25 30

Glu Ala Ala Ala Thr Ile Glu Val Glu Ile Ala Ser Leu Ala Thr Val
35 40 45

Glu Gly Ile Thr Ser Thr Ser Glu Ala Ile Ala Ala Ile Gly Leu Thr
50 55 60

Pro Glu Thr Tyr Ala Val Ile Thr Gly Ala Pro Gly Ala Val Ala Gly
65 70 75 80

Phe Ala Ala Leu Val Gln Thr Val Thr Gly Gly Ser Ala Ile Ala Gln
85 90 95

Leu Gly Tyr Arg Phe Phe Ala Asp Trp Asp His Lys Val Ser Thr Val
100 105 110

Gly Leu Phe Gln Gln Pro Ala Met Ala Leu Gln Leu Phe Asn Pro Glu
115 120 125

Asp Tyr Tyr Asp Ile Leu Phe Pro Gly Val Asn Ala Phe Val Asn Asn
130 135 140

Ile His Tyr Leu Asp Pro Arg His Trp Gly Pro Ser Leu Phe Ser Thr
145 150 155 160

Ile Ser Gln Ala Phe Trp Asn Leu Val Arg Asp Asp Leu Pro Ala Leu
165 170 175

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser Tyr Pro Tyr Asp Val Pro Asp Tyr
340 345 350

Ala Val Asp Phe Ile Leu Arg Arg Arg Arg Lys Arg Phe Pro Tyr Phe
355 360 365

Phe Thr Asp Val Arg Val Ala Ala
370 375

<210> 15
<211> 376
<212> PRT
<213> synthetic

<400> 15

Met Gly Ala Ala Leu Ala Leu Leu Gly Asp Leu Val Ala Thr Val Ser
1 5 10 15

Glu Ala Ala Ala Ala Thr Gly Phe Ser Val Ala Glu Ile Ala Ala Gly
20 25 30

Glu Ala Ala Ala Thr Ile Glu Val Glu Ile Ala Ser Leu Ala Thr Val
35 40 45

Glu Gly Ile Thr Ser Thr Ser Glu Ala Ile Ala Ala Ile Gly Leu Thr
50 55 60

Pro Glu Thr Tyr Ala Val Ile Thr Gly Ala Pro Gly Ala Val Ala Gly
65 70 75 80

Phe Ala Ala Leu Val Gln Thr Val Thr Gly Gly Ser Ala Ile Ala Gln
85 90 95

Leu Gly Tyr Arg Phe Phe Ala Asp Trp Asp His Lys Val Ser Thr Val
100 105 110

Gly Leu Phe Gln Gln Pro Ala Met Ala Leu Gln Leu Phe Asn Pro Glu
115 120 125

Asp Tyr Tyr Asp Ile Leu Phe Pro Gly Val Asn Ala Phe Val Asn Asn
130 135 140

Ile His Tyr Leu Asp Pro Arg His Trp Gly Pro Ser Leu Phe Ser Thr
145 150 155 160

Ile Ser Gln Ala Phe Trp Asn Leu Val Arg Asp Asp Leu Pro Ala Leu
165 170 175

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln

260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser Tyr Pro Tyr Asp Val Pro Asp Tyr
340 345 350

Ala Val Asp Asp Asp Leu Arg Arg Arg Arg Lys Arg Phe Pro Tyr Phe
355 360 365

Phe Thr Asp Val Arg Val Ala Ala
370 375

<210> 16
<211> 361
<212> PRT
<213> synthetic

<400> 16

Met Gly Ala Ala Leu Ala Leu Leu Gly Asp Leu Val Ala Thr Val Ser
1 5 10 15

Glu Ala Ala Ala Ala Thr Gly Phe Ser Val Ala Glu Ile Ala Ala Gly
20 25 30

Glu Ala Ala Ala Thr Ile Glu Val Glu Ile Ala Ser Leu Ala Thr Val
35 40 45

Glu Gly Ile Thr Ser Thr Ser Glu Ala Ile Ala Ala Ile Gly Leu Thr
50 55 60

Pro Glu Thr Tyr Ala Val Ile Thr Gly Ala Pro Gly Ala Val Ala Gly
65 70 75 80

Phe Ala Ala Leu Val Gln Thr Val Thr Gly Gly Ser Ala Ile Ala Gln
85 90 95

Leu Gly Tyr Arg Phe Phe Ala Asp Trp Asp His Lys Val Ser Thr Val

100 105 110

Gly Leu Phe Gln Gln Pro Ala Met Ala Leu Gln Leu Phe Asn Pro Glu
115 120 125

Asp Tyr Tyr Asp Ile Leu Phe Pro Gly Val Asn Ala Phe Val Asn Asn
130 135 140

Ile His Tyr Leu Asp Pro Arg His Trp Gly Pro Ser Leu Phe Ser Thr
145 150 155 160

Ile Ser Gln Ala Phe Trp Asn Leu Val Arg Asp Asp Leu Pro Ala Leu
165 170 175

Thr Ser Gln Glu Ile Gln Arg Arg Thr Gln Lys Leu Phe Val Glu Ser
180 185 190

Leu Ala Arg Phe Leu Glu Glu Thr Thr Trp Ala Ile Val Asn Ser Pro
195 200 205

Ala Asn Leu Tyr Asn Tyr Ile Ser Asp Tyr Tyr Ser Arg Leu Ser Pro
210 215 220

Val Arg Pro Ser Met Val Arg Gln Val Ala Gln Arg Glu Gly Thr Tyr
225 230 235 240

Ile Ser Phe Gly His Ser Tyr Thr Gln Ser Ile Asp Asp Ala Asp Ser
245 250 255

Ile Gln Glu Val Thr Gln Arg Leu Asp Leu Lys Thr Pro Asn Val Gln
260 265 270

Ser Gly Glu Phe Ile Glu Arg Ser Ile Ala Pro Gly Gly Ala Asn Gln
275 280 285

Arg Ser Ala Pro Gln Trp Met Leu Pro Leu Leu Leu Gly Leu Tyr Gly
290 295 300

Thr Val Thr Pro Ala Leu Glu Ala Tyr Glu Asp Gly Pro Asn Lys Lys
305 310 315 320

Lys Arg Arg Lys Glu Gly Pro Arg Ala Ser Ser Lys Thr Ser Tyr Lys
325 330 335

Arg Arg Ser Arg Ser Ser Arg Ser Tyr Pro Tyr Asp Val Pro Asp Tyr
340 345 350

```
Ala Val Asp His His His His His His
        355                 360


<210>  17
<211>  286
<212>  PRT
<213>  synthetic

<400>  17

Met Ala Leu Gln Leu Phe Asn Pro Glu Asp Tyr Tyr Asp Ile Leu Phe
1               5                   10                  15


Pro Gly Val Asn Ala Phe Val Asn Asn Ile His Tyr Leu Asp Pro Arg
            20                  25                  30


His Trp Gly Pro Ser Leu Phe Ser Thr Ile Ser Gln Ala Phe Trp Asn
        35                  40                  45


Leu Val Arg Asp Asp Leu Pro Ala Leu Thr Ser Gln Glu Ile Gln Arg
    50                  55                  60


Arg Thr Gln Lys Leu Phe Val Glu Ser Leu Ala Arg Phe Leu Glu Glu
65                  70                  75                  80


Thr Thr Trp Ala Ile Val Asn Ser Pro Ala Asn Leu Tyr Asn Tyr Ile
                85                  90                  95


Ser Asp Tyr Tyr Ser Arg Leu Ser Pro Val Arg Pro Ser Met Val Arg
            100                 105                 110


Gln Val Ala Gln Arg Glu Gly Thr Tyr Ile Ser Phe Gly His Ser Tyr
        115                 120                 125


Thr Gln Ser Ile Asp Asp Ala Asp Ser Ile Gln Glu Val Thr Gln Arg
    130                 135                 140


Leu Asp Leu Lys Thr Pro Asn Val Gln Ser Gly Glu Phe Ile Glu Arg
145                 150                 155                 160


Ser Ile Ala Pro Gly Gly Ala Asn Gln Arg Ser Ala Pro Gln Trp Met
                165                 170                 175


Leu Pro Leu Leu Leu Gly Leu Tyr Gly Thr Val Thr Pro Ala Leu Glu
            180                 185                 190


Ala Tyr Glu Asp Gly Pro Asn Lys Lys Lys Arg Arg Lys Glu Gly Pro
        195                 200                 205
```

Arg Ala Ser Ser Lys Thr Ser Tyr Lys Arg Arg Ser Arg Ser Ser Arg
    210                 215                 220

Ser Tyr Pro Tyr Asp Val Pro Asp Tyr Ala Leu Glu Ala Arg Tyr Arg
225                 230                 235                 240

Cys Cys Arg Ser Gln Ser Arg Ser Arg Tyr Tyr Arg Gln Arg Gln Arg
                245                 250                 255

Ser Arg Arg Arg Arg Arg Arg Ser Cys Gln Thr Arg Arg Arg Ala Met
            260                 265                 270

Arg Cys Cys Arg Pro Arg Tyr Arg Pro Arg Cys Arg Arg His
        275                 280                 285

<210> 18
<211> 257
<212> PRT
<213> synthetic

<400> 18

Met Ala Leu Gln Leu Phe Asn Pro Glu Asp Tyr Tyr Asp Ile Leu Phe
1               5                   10                  15

Pro Gly Val Asn Ala Phe Val Asn Asn Ile His Tyr Leu Asp Pro Arg
            20                  25                  30

His Trp Gly Pro Ser Leu Phe Ser Thr Ile Ser Gln Ala Phe Trp Asn
        35                  40                  45

Leu Val Arg Asp Asp Leu Pro Ala Leu Thr Ser Gln Glu Ile Gln Arg
    50                  55                  60

Arg Thr Gln Lys Leu Phe Val Glu Ser Leu Ala Arg Phe Leu Glu Glu
65                  70                  75                  80

Thr Thr Trp Ala Ile Val Asn Ser Pro Ala Asn Leu Tyr Asn Tyr Ile
                85                  90                  95

Ser Asp Tyr Tyr Ser Arg Leu Ser Pro Val Arg Pro Ser Met Val Arg
            100                 105                 110

Gln Val Ala Gln Arg Glu Gly Thr Tyr Ile Ser Phe Gly His Ser Tyr
        115                 120                 125

Thr Gln Ser Ile Asp Asp Ala Asp Ser Ile Gln Glu Val Thr Gln Arg
    130                 135                 140

Leu Asp Leu Lys Thr Pro Asn Val Gln Ser Gly Glu Phe Ile Glu Arg
145 150 155 160

Ser Ile Ala Pro Gly Gly Ala Asn Gln Arg Ser Ala Pro Gln Trp Met
165 170 175

Leu Pro Leu Leu Leu Gly Leu Tyr Gly Thr Val Thr Pro Ala Leu Glu
180 185 190

Ala Tyr Glu Asp Gly Pro Asn Lys Lys Lys Arg Arg Lys Glu Gly Pro
195 200 205

Arg Ala Ser Ser Lys Thr Ser Tyr Lys Arg Arg Ser Arg Ser Ser Arg
210 215 220

Ser Tyr Pro Tyr Asp Val Pro Asp Tyr Ala Leu Glu Ser Gln Ser Arg
225 230 235 240

Ser Arg Tyr Tyr Arg Gln Arg Gln Arg Ser Arg Arg Arg Arg Arg Arg
245 250 255

Ser

<210> 19
<211> 253
<212> PRT
<213> synthetic

<400> 19

Met Ala Leu Gln Leu Phe Asn Pro Glu Asp Tyr Tyr Asp Ile Leu Phe
1 5 10 15

Pro Gly Val Asn Ala Phe Val Asn Asn Ile His Tyr Leu Asp Pro Arg
20 25 30

His Trp Gly Pro Ser Leu Phe Ser Thr Ile Ser Gln Ala Phe Trp Asn
35 40 45

Leu Val Arg Asp Asp Leu Pro Ala Leu Thr Ser Gln Glu Ile Gln Arg
50 55 60

Arg Thr Gln Lys Leu Phe Val Glu Ser Leu Ala Arg Phe Leu Glu Glu
65 70 75 80

Thr Thr Trp Ala Ile Val Asn Ser Pro Ala Asn Leu Tyr Asn Tyr Ile
85 90 95

Ser Asp Tyr Tyr Ser Arg Leu Ser Pro Val Arg Pro Ser Met Val Arg
100 105 110

Gln Val Ala Gln Arg Glu Gly Thr Tyr Ile Ser Phe Gly His Ser Tyr
115 120 125

Thr Gln Ser Ile Asp Asp Ala Asp Ser Ile Gln Glu Val Thr Gln Arg
130 135 140

Leu Asp Leu Lys Thr Pro Asn Val Gln Ser Gly Glu Phe Ile Glu Arg
145 150 155 160

Ser Ile Ala Pro Gly Gly Ala Asn Gln Arg Ser Ala Pro Gln Trp Met
165 170 175

Leu Pro Leu Leu Leu Gly Leu Tyr Gly Thr Val Thr Pro Ala Leu Glu
180 185 190

Ala Tyr Glu Asp Gly Pro Asn Lys Lys Lys Arg Arg Lys Glu Gly Pro
195 200 205

Arg Ala Ser Ser Lys Thr Ser Tyr Lys Arg Arg Ser Arg Ser Ser Arg
210 215 220

Ser Tyr Pro Tyr Asp Val Pro Asp Tyr Ala Val Asp Gly Arg Lys Lys
225 230 235 240

Arg Arg Gln Arg Arg Arg Pro Pro Gln Lys Arg Lys Gly
245 250

<210> 20
<211> 252
<212> PRT
<213> synthetic

<400> 20

Met Ala Leu Gln Leu Phe Asn Pro Glu Asp Tyr Tyr Asp Ile Leu Phe
1 5 10 15

Pro Gly Val Asn Ala Phe Val Asn Asn Ile His Tyr Leu Asp Pro Arg
20 25 30

His Trp Gly Pro Ser Leu Phe Ser Thr Ile Ser Gln Ala Phe Trp Asn
35 40 45

Leu Val Arg Asp Asp Leu Pro Ala Leu Thr Ser Gln Glu Ile Gln Arg
50 55 60

Arg Thr Gln Lys Leu Phe Val Glu Ser Leu Ala Arg Phe Leu Glu Glu
65 70 75 80

Thr Thr Trp Ala Ile Val Asn Ser Pro Ala Asn Leu Tyr Asn Tyr Ile
85 90 95

Ser Asp Tyr Tyr Ser Arg Leu Ser Pro Val Arg Pro Ser Met Val Arg
100 105 110

Gln Val Ala Gln Arg Glu Gly Thr Tyr Ile Ser Phe Gly His Ser Tyr
115 120 125

Thr Gln Ser Ile Asp Asp Ala Asp Ser Ile Gln Glu Val Thr Gln Arg
130 135 140

Leu Asp Leu Lys Thr Pro Asn Val Gln Ser Gly Glu Phe Ile Glu Arg
145 150 155 160

Ser Ile Ala Pro Gly Gly Ala Asn Gln Arg Ser Ala Pro Gln Trp Met
165 170 175

Leu Pro Leu Leu Leu Gly Leu Tyr Gly Thr Val Thr Pro Ala Leu Glu
180 185 190

Ala Tyr Glu Asp Gly Pro Asn Lys Lys Lys Arg Arg Lys Glu Gly Pro
195 200 205

Arg Ala Ser Ser Lys Thr Ser Tyr Lys Arg Arg Ser Arg Ser Ser Arg
210 215 220

Ser Tyr Pro Tyr Asp Val Pro Asp Tyr Ala Val Asp Arg Gln Ile Lys
225 230 235 240

Ile Trp Phe Gln Asn Arg Arg Met Lys Trp Lys Lys
245 250

<210> 21
<211> 257
<212> PRT
<213> synthetic

<400> 21

Met Ala Leu Gln Leu Phe Asn Pro Glu Asp Tyr Tyr Asp Ile Leu Phe
1 5 10 15

Pro Gly Val Asn Ala Phe Val Asn Asn Ile His Tyr Leu Asp Pro Arg
20 25 30

```
His Trp Gly Pro Ser Leu Phe Ser Thr Ile Ser Gln Ala Phe Trp Asn
        35                  40                  45

Leu Val Arg Asp Asp Leu Pro Ala Leu Thr Ser Gln Glu Ile Gln Arg
    50                  55                  60

Arg Thr Gln Lys Leu Phe Val Glu Ser Leu Ala Arg Phe Leu Glu Glu
65                  70                  75                  80

Thr Thr Trp Ala Ile Val Asn Ser Pro Ala Asn Leu Tyr Asn Tyr Ile
                85                  90                  95

Ser Asp Tyr Tyr Ser Arg Leu Ser Pro Val Arg Pro Ser Met Val Arg
            100                 105                 110

Gln Val Ala Gln Arg Glu Gly Thr Tyr Ile Ser Phe Gly His Ser Tyr
        115                 120                 125

Thr Gln Ser Ile Asp Asp Ala Asp Ser Ile Gln Glu Val Thr Gln Arg
    130                 135                 140

Leu Asp Leu Lys Thr Pro Asn Val Gln Ser Gly Glu Phe Ile Glu Arg
145                 150                 155                 160

Ser Ile Ala Pro Gly Gly Ala Asn Gln Arg Ser Ala Pro Gln Trp Met
                165                 170                 175

Leu Pro Leu Leu Leu Gly Leu Tyr Gly Thr Val Thr Pro Ala Leu Glu
            180                 185                 190

Ala Tyr Glu Asp Gly Pro Asn Lys Lys Lys Arg Arg Lys Glu Gly Pro
        195                 200                 205

Arg Ala Ser Ser Lys Thr Ser Tyr Lys Arg Arg Ser Arg Ser Ser Arg
    210                 215                 220

Ser Tyr Pro Tyr Asp Val Pro Asp Tyr Ala Val Asp Phe Ile Leu Arg
225                 230                 235                 240

Arg Arg Arg Lys Arg Phe Pro Tyr Phe Phe Thr Asp Val Arg Val Ala
                245                 250                 255

Ala


<210>  22
<211>  257
<212>  PRT
```

<213> synthetic

<400> 22

```
Met Ala Leu Gln Leu Phe Asn Pro Glu Asp Tyr Tyr Asp Ile Leu Phe
1               5                   10                  15

Pro Gly Val Asn Ala Phe Val Asn Asn Ile His Tyr Leu Asp Pro Arg
            20                  25                  30

His Trp Gly Pro Ser Leu Phe Ser Thr Ile Ser Gln Ala Phe Trp Asn
        35                  40                  45

Leu Val Arg Asp Asp Leu Pro Ala Leu Thr Ser Gln Glu Ile Gln Arg
    50                  55                  60

Arg Thr Gln Lys Leu Phe Val Glu Ser Leu Ala Arg Phe Leu Glu Glu
65                  70                  75                  80

Thr Thr Trp Ala Ile Val Asn Ser Pro Ala Asn Leu Tyr Asn Tyr Ile
                85                  90                  95

Ser Asp Tyr Tyr Ser Arg Leu Ser Pro Val Arg Pro Ser Met Val Arg
            100                 105                 110

Gln Val Ala Gln Arg Glu Gly Thr Tyr Ile Ser Phe Gly His Ser Tyr
        115                 120                 125

Thr Gln Ser Ile Asp Asp Ala Asp Ser Ile Gln Glu Val Thr Gln Arg
    130                 135                 140

Leu Asp Leu Lys Thr Pro Asn Val Gln Ser Gly Glu Phe Ile Glu Arg
145                 150                 155                 160

Ser Ile Ala Pro Gly Gly Ala Asn Gln Arg Ser Ala Pro Gln Trp Met
                165                 170                 175

Leu Pro Leu Leu Leu Gly Leu Tyr Gly Thr Val Thr Pro Ala Leu Glu
            180                 185                 190

Ala Tyr Glu Asp Gly Pro Asn Lys Lys Lys Arg Arg Lys Glu Gly Pro
        195                 200                 205

Arg Ala Ser Ser Lys Thr Ser Tyr Lys Arg Arg Ser Arg Ser Ser Arg
    210                 215                 220

Ser Tyr Pro Tyr Asp Val Pro Asp Tyr Ala Val Asp Asp Asp Leu Arg
225                 230                 235                 240
```

Arg Arg Arg Lys Arg Phe Pro Tyr Phe Phe Thr Asp Val Arg Val Ala
245 250 255

Ala

<210> 23
<211> 242
<212> PRT
<213> synthetic

<400> 23

Met Ala Leu Gln Leu Phe Asn Pro Glu Asp Tyr Tyr Asp Ile Leu Phe
1 5 10 15

Pro Gly Val Asn Ala Phe Val Asn Asn Ile His Tyr Leu Asp Pro Arg
20 25 30

His Trp Gly Pro Ser Leu Phe Ser Thr Ile Ser Gln Ala Phe Trp Asn
35 40 45

Leu Val Arg Asp Asp Leu Pro Ala Leu Thr Ser Gln Glu Ile Gln Arg
50 55 60

Arg Thr Gln Lys Leu Phe Val Glu Ser Leu Ala Arg Phe Leu Glu Glu
65 70 75 80

Thr Thr Trp Ala Ile Val Asn Ser Pro Ala Asn Leu Tyr Asn Tyr Ile
85 90 95

Ser Asp Tyr Tyr Ser Arg Leu Ser Pro Val Arg Pro Ser Met Val Arg
100 105 110

Gln Val Ala Gln Arg Glu Gly Thr Tyr Ile Ser Phe Gly His Ser Tyr
115 120 125

Thr Gln Ser Ile Asp Asp Ala Asp Ser Ile Gln Glu Val Thr Gln Arg
130 135 140

Leu Asp Leu Lys Thr Pro Asn Val Gln Ser Gly Glu Phe Ile Glu Arg
145 150 155 160

Ser Ile Ala Pro Gly Gly Ala Asn Gln Arg Ser Ala Pro Gln Trp Met
165 170 175

Leu Pro Leu Leu Leu Gly Leu Tyr Gly Thr Val Thr Pro Ala Leu Glu
180 185 190

Ala Tyr Glu Asp Gly Pro Asn Lys Lys Lys Arg Arg Lys Glu Gly Pro
195 200 205

Arg Ala Ser Ser Lys Thr Ser Tyr Lys Arg Arg Ser Arg Ser Ser Arg
210 215 220

Ser Tyr Pro Tyr Asp Val Pro Asp Tyr Ala Val Asp His His His His
225 230 235 240

His His

<210> 24
<211> 354
<212> PRT
<213> JC-Polyomavirus

<400> 24

Met Ala Pro Thr Lys Arg Lys Gly Glu Arg Lys Asp Pro Val Gln Val
1 5 10 15

Pro Lys Leu Leu Ile Arg Gly Gly Val Glu Val Leu Glu Val Lys Thr
20 25 30

Gly Val Asp Ser Ile Thr Glu Val Glu Cys Phe Leu Thr Pro Glu Met
35 40 45

Gly Asp Pro Asp Glu His Leu Arg Gly Phe Ser Lys Ser Ile Ser Ile
50 55 60

Ser Asp Thr Phe Glu Ser Asp Ser Pro Asn Arg Asp Met Leu Pro Cys
65 70 75 80

Tyr Ser Val Ala Arg Ile Pro Leu Pro Asn Leu Asn Glu Asp Leu Thr
85 90 95

Cys Gly Asn Ile Leu Met Trp Glu Ala Val Thr Leu Lys Thr Glu Val
100 105 110

Ile Gly Val Thr Ser Leu Met Asn Val His Ser Asn Gly Gln Ala Thr
115 120 125

His Asp Asn Gly Ala Gly Lys Pro Val Gln Gly Thr Ser Phe His Phe
130 135 140

Phe Ser Val Gly Gly Glu Ala Leu Glu Leu Gln Gly Val Leu Phe Asn
145 150 155 160

```
Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Ser Gly Ser Gln Gln Trp
            260                 265                 270

Arg Gly Leu Ser Arg Tyr Phe Lys Val Gln Leu Arg Lys Arg Arg Val
        275                 280                 285

Lys Asn Pro Tyr Pro Ile Ser Phe Leu Leu Thr Asp Leu Ile Asn Arg
    290                 295                 300

Arg Thr Pro Arg Val Asp Gly Gln Pro Met Tyr Gly Met Asp Ala Gln
305                 310                 315                 320

Val Glu Glu Val Arg Val Phe Glu Gly Thr Glu Glu Leu Pro Gly Asp
                325                 330                 335

Pro Asp Met Met Arg Tyr Val Asp Lys Tyr Gly Gln Leu Gln Thr Lys
            340                 345                 350

Met Leu


<210>  25
<211>  1062
<212>  DNA
<213>  JC-Polyomavirus

<400>  25
atgggagccg ccctggccct gctgggagat ctggtggcta cagtgtctga agccgccgct        60

gccaccggct tttctgtggc cgaaattgcc gctggcgagg ctgccgccac aatcgaggtg       120
```

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aacccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctt ga 1062

<210> 26
<211> 705
<212> DNA
<213> JC-Polyomavirus

<400> 26
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg cttga 705

<210> 27
<211> 108
<212> DNA
<213> JC-Polyomavirus

<400> 27
agcggcgagt tcatcgagag atccattgcc cctggcggag ccaaccagag atctgcccct 60

cagtggatgc tgcccctgct gctgggcctg tacggcacag tgacacca 108

<210> 28
<211> 252
<212> DNA
<213> Homo sapiens (human)

<400> 28
atggccaggt acagatgctg tcgcagccag agccggagca gatattaccg ccagagacaa 60

agaagtcgca gacgaaggag gcggagctgc cagacacgga ggagagccat gaggtgctgc 120

cgccccaggt acagaccgag atgtagaaga cactaattgc acaaaatagc acatccacca 180

aactcctgcc tgagaatgtt accagacttc aagatcctct tgccacatct tgaaaatgcc 240

accatccaat aa 252

<210> 29
<211> 67
<212> DNA
<213> Homo sapiens (human)

<400> 29
cgcagccaga gccggagcag atattaccgc cagagacaaa gaagtcgcag acgaaggagg 60

cggagct 67

<210> 30
<211> 51
<212> DNA
<213> Human immunodeficiency virus (HIV)

<400> 30
gggcgcaaga agcgtcgcca acgtcgccgt ccacccccaaa agcgcaaggg c 51

<210> 31
<211> 48
<212> DNA
<213> Antennapedia spec.

<400> 31
cgacaaatca aaatctggtt tcaaaatcga cgaatgaaat ggaaaaaa 48

<210> 32
<211> 63
<212> DNA
<213> Human papillomavirus type 33 (HPV33)

<400> 32
tttattttac gtcgcaggcg taaacgtttt ccatattttt ttacagatgt ccgtgtggcg 60

gcc 63

<210> 33
<211> 63
<212> DNA
<213> Human papillomavirus type 33 (HPV33)

<400> 33
gatgatttac gtcgcaggcg taaacgtttt ccatattttt ttacagatgt ccgtgtggcg 60

gcc 63

<210> 34
<211> 1218
<212> DNA
<213> synthetic

<400> 34
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aacccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctc tcgaggccag gtacagatgc 1080

tgtcgcagcc agagccggag cagatattac cgccagagac aaagaagtcg cagacgaagg 1140

aggcggagct gccagacacg gaggagagcc atgaggtgct gccgccccag gtacagaccg 1200

agatgtagaa gacactaa 1218

<210> 35
<211> 1135
<212> DNA
<213> synthetic

<400> 35
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aacccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctc tcgagcgcag ccagagccgg 1080

agcagatatt accgccagag acaaagaagt cgcagacgaa ggaggcggag cttga 1135

<210> 36
<211> 1119
<212> DNA
<213> synthetic

<400> 36
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aaccccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctg tcgacgggcg caagaagcgt 1080

cgccaacgtc gccgtccacc ccaaaagcgc aagggctga 1119

<210> 37
<211> 1116
<212> DNA
<213> synthetic

<400> 37
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aacccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctg tcgaccgaca aatcaaaatc 1080

tggtttcaaa atcgacgaat gaaatggaaa aaatga 1116

<210> 38
<211> 1131
<212> DNA
<213> synthetic

<400> 38
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aacccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctg tcgactttat tttacgtcgc 1080

aggcgtaaac gttttccata ttttttaca gatgtccgtg tggcggcctg a 1131

<210> 39
<211> 1131
<212> DNA
<213> synthetic

<400> 39

```
atgggagccg ccctggccct gctgggagat ctggtggcta cagtgtctga agccgccgct        60
gccaccggct tttctgtggc cgaaattgcc gctggcgagg ctgccgccac aatcgaggtg       120
gaaattgcca gcctggccac cgtggaaggc atcaccagca cctctgaagc cattgccgcc       180
atcggcctga cccccgagac atacgctgtg atcactggcg ctccaggcgc cgtggctgga       240
tttgctgctc tggtgcagac agtgaccggc ggctctgcca ttgctcagct gggctacaga       300
ttcttcgccg actgggacca caaggtgtcc accgtgggcc tgtttcagca gcctgccatg       360
gctctgcagc tgttcaaccc cgaggactac tacgacatcc tgttccccgg cgtgaacgcc       420
ttcgtgaaca acatccacta cctggacccc cggcactggg gccctagcct gttctctaca       480
atcagccagg ccttctggaa cctcgtgcgg gacgatctgc ctgccctgac cagccaggaa       540
atccagcggc ggacccagaa actgttcgtg gaaagcctgg cccggttcct ggaagagaca       600
acctgggcca tcgtgaacag ccccgccaac ctgtacaact acatcagcga ctactacagc       660
agactgagcc ccgtgcggcc cagcatggtg cgccaggtgg cacagagaga gggcacctat       720
atcagcttcg gccactccta cacccagagc atcgacgacg ccgacagcat ccaggaagtg       780
acccagagac tggacctgaa aacccccaac gtgcagagcg gcgagttcat cgagagatcc       840
attgcccctg gcggagccaa ccagagatct gcccctcagt ggatgctgcc cctgctgctg       900
ggcctgtacg gcacagtgac accagccctg gaagcctacg aggacggccc caacaagaag       960
aagcgccgga aagagggccc tagagccagc agcaagacca gctacaagcg gcggagcaga      1020
agcagcagat cctacccata cgatgttcca gattacgctg tcgacgatga tttacgtcgc      1080
aggcgtaaac gttttccata ttttttaca gatgtccgtg tggcggcctg a               1131
```

```
<210>  40
<211>  1086
<212>  DNA
<213>  synthetic

<400>  40
atgggagccg ccctggccct gctgggagat ctggtggcta cagtgtctga agccgccgct        60
gccaccggct tttctgtggc cgaaattgcc gctggcgagg ctgccgccac aatcgaggtg       120
gaaattgcca gcctggccac cgtggaaggc atcaccagca cctctgaagc cattgccgcc       180
atcggcctga cccccgagac atacgctgtg atcactggcg ctccaggcgc cgtggctgga       240
tttgctgctc tggtgcagac agtgaccggc ggctctgcca ttgctcagct gggctacaga       300
ttcttcgccg actgggacca caaggtgtcc accgtgggcc tgtttcagca gcctgccatg       360
gctctgcagc tgttcaaccc cgaggactac tacgacatcc tgttccccgg cgtgaacgcc       420
ttcgtgaaca acatccacta cctggacccc cggcactggg gccctagcct gttctctaca       480
atcagccagg ccttctggaa cctcgtgcgg gacgatctgc ctgccctgac cagccaggaa       540
```

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aaccccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctg tcgaccatca tcaccatcac 1080

cattga 1086

<210> 41
<211> 861
<212> DNA
<213> synthetic

<400> 41
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctctcgaggc caggtacaga 720

tgctgtcgca gccagagccg gagcagatat taccgccaga gacaaagaag tcgcagacga 780

aggaggcgga gctgccagac acggaggaga gccatgaggt gctgccgccc caggtacaga 840

ccgagatgta gaagacacta a 861

<210> 42
<211> 778
<212> DNA
<213> synthetic

<400> 42
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctctcgagcg cagccagagc 720

cggagcagat attaccgcca gagacaaaga agtcgcagac gaaggaggcg gagcttga 778

<210> 43
<211> 762
<212> DNA
<213> synthetic

<400> 43
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctgtcgacgg gcgcaagaag 720

cgtcgccaac gtcgccgtcc accccaaaag cgcaagggct ga 762

<210> 44
<211> 1116

<212> DNA
<213> synthetic

<400> 44
atgggagccg ccctggccct gctgggagat ctggtggcta cagtgtctga agccgccgct 60

gccaccggct tttctgtggc cgaaattgcc gctggcgagg ctgccgccac aatcgaggtg 120

gaaattgcca gcctggccac cgtggaaggc atcaccagca cctctgaagc cattgccgcc 180

atcggcctga cccccgagac atacgctgtg atcactggcg ctccaggcgc cgtggctgga 240

tttgctgctc tggtgcagac agtgaccggc ggctctgcca ttgctcagct gggctacaga 300

ttcttcgccg actgggacca caaggtgtcc accgtgggcc tgtttcagca gcctgccatg 360

gctctgcagc tgttcaaccc cgaggactac tacgacatcc tgttccccgg cgtgaacgcc 420

ttcgtgaaca acatccacta cctggacccc cggcactggg gccctagcct gttctctaca 480

atcagccagg ccttctggaa cctcgtgcgg gacgatctgc ctgccctgac cagccaggaa 540

atccagcggc ggacccagaa actgttcgtg gaaagcctgg cccggttcct ggaagagaca 600

acctgggcca tcgtgaacag ccccgccaac ctgtacaact acatcagcga ctactacagc 660

agactgagcc ccgtgcggcc cagcatggtg cgccaggtgg cacagagaga gggcacctat 720

atcagcttcg gccactccta cacccagagc atcgacgacg ccgacagcat ccaggaagtg 780

acccagagac tggacctgaa aaccccccaac gtgcagagcg gcgagttcat cgagagatcc 840

attgcccctg gcggagccaa ccagagatct gcccctcagt ggatgctgcc cctgctgctg 900

ggcctgtacg gcacagtgac accagccctg gaagcctacg aggacggccc caacaagaag 960

aagcgccgga aagagggccc tagagccagc agcaagacca gctacaagcg gcggagcaga 1020

agcagcagat cctacccata cgatgttcca gattacgctg tcgaccgaca aatcaaaatc 1080

tggtttcaaa atcgacgaat gaaatggaaa aaatga 1116

<210> 45
<211> 774
<212> DNA
<213> synthetic

<400> 45
atggctctgc agctgttcaa ccccgaggac tactacgaca tcctgttccc cggcgtgaac 60

gccttcgtga acaacatcca ctacctggac cccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctgtcgactt tattttacgt 720

cgcaggcgta aacgttttcc atattttttt acagatgtcc gtgtggcggc ctga 774

<210> 46
<211> 774
<212> DNA
<213> synthetic

<400> 46
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctgtcgacga tgatttacgt 720

cgcaggcgta aacgttttcc atattttttt acagatgtcc gtgtggcggc ctga 774

<210> 47
<211> 729
<212> DNA
<213> synthetic

<400> 47
atggctctgc agctgttcaa ccccgaggac tactacgaca tcctgttccc cggcgtgaac 60

gccttcgtga acaacatcca ctacctggac ccccggcact ggggccctag cctgttctct 120

acaatcagcc aggccttctg gaacctcgtg cgggacgatc tgcctgccct gaccagccag 180

gaaatccagc ggcggaccca gaaactgttc gtggaaagcc tggcccggtt cctggaagag 240

acaacctggg ccatcgtgaa cagccccgcc aacctgtaca actacatcag cgactactac 300

agcagactga gccccgtgcg gcccagcatg gtgcgccagg tggcacagag agagggcacc 360

tatatcagct tcggccactc ctacacccag agcatcgacg acgccgacag catccaggaa 420

gtgacccaga gactggacct gaaaaccccc aacgtgcaga gcggcgagtt catcgagaga 480

tccattgccc ctggcggagc caaccagaga tctgcccctc agtggatgct gcccctgctg 540

ctgggcctgt acggcacagt gacaccagcc ctggaagcct acgaggacgg ccccaacaag 600

aagaagcgcc ggaaagaggg ccctagagcc agcagcaaga ccagctacaa gcggcggagc 660

agaagcagca gatcctaccc atacgatgtt ccagattacg ctgtcgacca tcatcaccat 720

caccattga 729

<210> 48
<211> 1065
<212> DNA
<213> JC-Polyomavirus

<400> 48
atggctccca ccaagcgcaa gggcgagccc aaggaccccg tgcaagtgcc caagctgctg 60

atccgtggtg gtgtcgaggt gctggaagtc aagaccggcg tggactccat taccgaggtg 120

gagtgcttcc tcacccccga gatgggtgac cctgacgagc acctgagggg cttctccaag 180

tccatctcca tctccgacac cttcgagtcc gactccccca accgtgacat gctgccctgc 240

tactccgtgg ctcgtatccc cctgcccaac ctgaacgagg acctgacttg cggcaacatc 300

ctgatgtggg aggctgtgac cctcaagacc gaggtcatcg gcgtgacttc cctgatgaac 360

gtgcactcca acggccaggc tacccacgac aacggtgctg gcaagcccgt gcagggaacc 420

tccttccact tcttctccgt gggtggcgag gctctggaac tccagggcgt ggtgttcaac 480

taccgtacca agtaccccga cggcaccatc ttccccaaga acgctactgt gcagtcccaa 540

gtgatgaaca ccgagcacaa ggcttacctg gacaagaaca aggcctaccc cgtggagtgc 600

tgggtgcccg accccacccg taacgagaac acccgttact tcggcaccct gaccggtgga 660

gagaacgtgc ccccccgtgct gcacatcacc aacaccgcta ccaccgtgct gctggacgag 720

ttcggtgtcg gtcccctgtg caagggcgac aacctgtacc tgtccgctgt ggacgtgtgc 780

ggcatgttca ccaaccgttc cggttcccag cagtggcgtg gcctgtcccg ctacttcaag 840

gtgcagctgc gcaagcgtcg tgtgaagaac ccctacccta tctccttcct gctgaccgac 900

ctgatcaacc gtcgtacccc tcgtgtggac ggccagccca tgtacggcat ggacgctcag 960

gtggaagagg tccgcgtgtt cgagggcacc gaggaattgc ccggcgaccc cgacatgatg 1020

cgttacgtgg acaagtacgg ccagctccag accaagatgc tgtaa 1065

<210> 49
<211> 19
<212> RNA
<213> synthetic

<400> 49
cgggaagcug gaaauauaa 19

<210> 50
<211> 18
<212> PRT
<213> synthetic

<400> 50

Arg Gly Gly Arg Leu Ser Tyr Ser Arg Arg Arg Phe Ser Thr Ser Thr
1 5 10 15

Gly Arg

<210> 51
<211> 10
<212> PRT
<213> synthetic

<400> 51

Arg Arg Leu Ser Tyr Ser Arg Arg Arg Phe
1 5 10

<210> 52
<211> 12
<212> PRT
<213> synthetic

<400> 52

Pro Ile Arg Arg Arg Lys Lys Leu Arg Arg Leu Lys
1 5 10

<210> 53
<211> 12
<212> PRT
<213> synthetic

<400> 53

Arg Arg Gln Arg Arg Thr Ser Lys Leu Met Lys Arg
1 5 10

<210> 54
<211> 15
<212> PRT
<213> FHV

<400> 54

Arg Arg Arg Arg Asn Arg Thr Arg Arg Asn Arg Arg Arg Val Arg
1 5 10 15

<210> 55
<211> 19
<212> PRT
<213> BMV

<400> 55

Lys Met Thr Arg Ala Gln Arg Arg Ala Ala Ala Arg Arg Asn Arg Trp
1               5                   10                  15

Thr Ala Arg

<210> 56
<211> 13
<212> PRT
<213> HTLV

<400> 56

Thr Arg Arg Gln Arg Thr Arg Arg Ala Arg Arg Asn Arg
1               5                   10

<210> 57
<211> 13
<212> PRT
<213> Human immunodeficiency virus (HIV)

<400> 57

Gly Arg Lys Lys Arg Arg Gln Arg Arg Arg Pro Pro Gln
1               5                   10

<210> 58
<211> 13
<212> PRT
<213> Human immunodeficiency virus (HIV)

<400> 58

Gly Arg Arg Arg Arg Arg Arg Arg Arg Arg Pro Pro Gln
1               5                   10

<210> 59
<211> 48
<212> DNA
<213> synthetic

<400> 59
ccatattttt ttacagatgt ccgtgtggcg gcctgagcgg ccgctttc 48

<210> 60
<211> 48
<212> DNA
<213> synthetic

<400> 60
ccatattttt ttacagatgt ccgtgtggcg gcctgagcgg ccgctttc 48

<210> 61
<211> 51
<212> DNA
<213> synthetic

<400> 61
aaaacgttta cgcctgcgac gtaaatcatc gtcgacagcg taatctggaa c 51

<210> 62
<211> 45
<212> DNA
<213> synthetic

<400> 62
acgttggcga cgcttcttgc gcccgtcgac agcgtaatct ggaac 45

<210> 63
<211> 45
<212> DNA
<213> synthetic

<400> 63
acgttggcga cgcttcttgc gcccgtcgac agcgtaatct ggaac 45

<210> 64
<211> 39
<212> DNA
<213> synthetic

<400> 64
aatcgacgaa tgaaatggaa aaaatgagcg gccgctttc 39

<210> 65
<211> 45
<212> DNA
<213> synthetic

<400> 65
ttgaaaccag attttgattt gtcggtcgac agcgtaatct ggaac 45

<210> 66
<211> 24
<212> DNA
<213> synthetic

<400> 66
catcaccatt gagcggccgc tttc 24

<210> 67
<211> 30
<212> DNA
<213> synthetic

<400> 67
gtgatgatgg tcgacagcgt aatctggaac 30

<210> 68
<211> 7334
<212> DNA
<213> synthetic

<400> 68
ttctctgtca cagaatgaaa atttttctgt catctcttcg ttattaatgt ttgtaattga 60

ctgaatatca acgcttattt gcagcctgaa tggcgaatgg gacgcgccct gtagcggcgc 120

attaagcgcg gcgggtgtgg tggttacgcg cagcgtgacc gctacacttg ccagcgccct 180

agcgcccgct cctttcgctt tcttcccttc ctttctcgcc acgttcgccg gctttccccg 240

tcaagctcta aatcgggggc tccctttagg gttccgattt agtgctttac ggcacctcga 300

ccccaaaaaa cttgattagg gtgatggttc acgtagtggg ccatcgccct gatagacggt 360

ttttcgccct ttgacgttgg agtccacgtt ctttaatagt ggactcttgt tccaaactgg 420

aacaacactc aaccctatct cggtctattc ttttgattta taagggattt tgccgatttc 480

ggcctattgg ttaaaaaatg agctgattta acaaaaattt aacgcgaatt ttaacaaaat 540

attaacgttt acaatttcag gtggcacttt tcggggaaat gtgcgcggaa cccctatttg 600

tttatttttc taaatacatt caaatatgta tccgctcatg agacaataac cctgataaat 660

gcttcaataa tattgaaaaa ggaagagtat gagtattcaa catttccgtg tcgcccttat 720

tccctttttt gcggcatttt gccttcctgt ttttgctcac ccagaaacgc tggtgaaagt 780

aaaagatgct gaagatcagt tgggtgcacg agtgggttac atcgaactgg atctcaacag 840

cggtaagatc cttgagagtt ttcgccccga agaacgtttt ccaatgatga gcacttttaa 900

agttctgcta tgtggcgcgg tattatcccg tattgacgcc gggcaagagc aactcggtcg 960

ccgcatacac tattctcaga atgacttggt tgagtactca ccagtcacag aaaagcatct 1020

tacggatggc atgacagtaa gagaattatg cagtgctgcc ataaccatga gtgataacac 1080

tgcggccaac ttacttctga caacgatcgg aggaccgaag gagctaaccg cttttttgca 1140

caacatgggg gatcatgtaa ctcgccttga tcgttgggaa ccggagctga atgaagccat 1200

accaaacgac gagcgtgaca ccacgatgcc tgtagcaatg gcaacaacgt tgcgcaaact 1260

attaactggc gaactactta ctctagcttc ccggcaacaa ttaatagact ggatggaggc 1320

ggataaagtt gcaggaccac ttctgcgctc ggcccttccg gctggctggt ttattgctga 1380

taaatctgga gccggtgagc gtgggtctcg cggtatcatt gcagcactgg ggccagatgg 1440

taagccctcc cgtatcgtag ttatctacac gacggggagt caggcaacta tggatgaacg 1500

aaatagacag atcgctgaga taggtgcctc actgattaag cattggtaac tgtcagacca 1560

agtttactca tatatacttt agattgattt aaaacttcat ttttaattta aaaggatcta 1620

ggtgaagatc ctttttgata atctcatgac caaaatccct taacgtgagt tttcgttcca 1680
ctgagcgtca gaccccgtag aaaagatcaa aggatcttct tgagatcctt tttttctgcg 1740
cgtaatctgc tgcttgcaaa caaaaaaacc accgctacca gcggtggttt gtttgccgga 1800
tcaagagcta ccaactcttt ttccgaaggt aactggcttc agcagagcgc agataccaaa 1860
tactgtcctt ctagtgtagc cgtagttagg ccaccacttc aagaactctg tagcaccgcc 1920
tacatacctc gctctgctaa tcctgttacc agtggctgct gccagtggcg ataagtcgtg 1980
tcttaccggg ttggactcaa gacgatagtt accggataag gcgcagcggt cgggctgaac 2040
ggggggttcg tgcacacagc ccagcttgga gcgaacgacc tacaccgaac tgagatacct 2100
acagcgtgag cattgagaaa gcgccacgct tcccgaaggg agaaaggcgg acaggtatcc 2160
ggtaagcggc agggtcggaa caggagagcg cacgagggag cttccagggg gaaacgcctg 2220
gtatctttat agtcctgtcg ggtttcgcca cctctgactt gagcgtcgat ttttgtgatg 2280
ctcgtcaggg gggcggagcc tatggaaaaa cgccagcaac gcggcctttt tacggttcct 2340
ggccttttgc tggccttttg ctcacatgtt ctttcctgcg ttatcccctg attctgtgga 2400
taaccgtatt accgcctttg agtgagctga taccgctcgc cgcagccgaa cgaccgagcg 2460
cagcgagtca gtgagcgagg aagcggaaga gcgcctgatg cggtattttc tccttacgca 2520
tctgtgcggt atttcacacc gcagaccagc cgcgtaacct ggcaaaatcg gttacggttg 2580
agtaataaat ggatgccctg cgtaagcggg tgtgggcgga caataaagtc ttaaactgaa 2640
caaaatagat ctaaactatg acaataaagt cttaaactag acagaatagt tgtaaactga 2700
aatcagtcca gttatgctgt gaaaaagcat actggacttt tgttatggct aaagcaaact 2760
cttcattttc tgaagtgcaa attgcccgtc gtattaaaga ggggcgtggc caagggcatg 2820
gtaaagacta tattcgcggc gttgtgacaa tttaccgaac aactccgcgg ccgggaagcc 2880
gatctcggct tgaacgaatt gttaggtggc ggtacttggg tcgatatcaa agtgcatcac 2940
ttcttcccgt atgcccaact ttgtatagag agccactgcg ggatcgtcac cgtaatctgc 3000
ttgcacgtag atcacataag caccaagcgc gttggcctca tgcttgagga gattgatgag 3060
cgcggtggca atgccctgcc tccggtgctc gccggagact gcgagatcat agatatagat 3120
ctcactacgc ggctgctcaa acctgggcag aacgtaagcc gcgagagcgc caacaaccgc 3180
ttcttggtcg aaggcagcaa gcgcgatgaa tgtcttacta cggagcaagt tcccgaggta 3240
atcggagtcc ggctgatgtt gggagtaggt ggctacgtct ccgaactcac gaccgaaaag 3300
atcaagagca gcccgcatgg atttgacttg gtcagggccg agcctacatg tgcgaatgat 3360
gcccatactt gagccaccta actttgtttt agggcgactg ccctgctgcg taacatcgtt 3420
gctgctgcgt aacatcgttg ctgctccata acatcaaaca tcgacccacg gcgtaacgcg 3480

cttgctgctt ggatgcccga ggcatagact gtacaaaaaa acagtcataa caagccatga 3540

aaaccgccac tgcgccgtta ccaccgctgc gttcggtcaa ggttctggac cagttgcgtg 3600

agcgcatacg ctacttgcat tacagtttac gaaccgaaca ggcttatgtc aactgggttc 3660

gtgccttcat ccgtttccac ggtgtgcgtc acccggcaac cttgggcagc agcgaagtcg 3720

aggcatttct gtcctggctg gcgaacgagc gcaaggtttc ggtctccacg catcgtcagg 3780

cattggcggc cttgctgttc ttctacggca aggtgctgtg cacggatctg ccctggcttc 3840

aggagatcgg tagacctcgg ccgtcgcggc gcttgccggt ggtgctgacc ccggatgaag 3900

tggttcgcat cctcggtttt ctggaaggcg agcatcgttt gttcgcccag gactctagct 3960

atagttctag tggttggcct acgtacccgt agtggctatg gcagggcttg ccgccccgac 4020

gttggctgcg agccctgggc cttcacccga acttgggggt tggggtgggg aaaaggaaga 4080

aacgcgggcg tattggtccc aatggggtct cggtggggta tcgacagagt gccagccctg 4140

ggaccgaacc ccgcgtttat gaacaaacga cccaacaccc gtgcgtttta ttctgtcttt 4200

ttattgccgt catagcgcgg gttccttccg gtattgtctc cttccgtgtt tcagttagcc 4260

tcccccatct cccggtaccg catgcctcga gactgcagtt acagcatctt ggtctggagc 4320

tggccgtact tgtccacgta acgcatcatg tcggggtcgc cgggcaattc ctcggtgccc 4380

tcgaacacgc ggacctcttc cacctgagcg tccatgccgt acatgggctg gccgtccaca 4440

cgaggggtac gacggttgat caggtcggtc agcaggaagg agatagggta ggggttcttc 4500

acacgacgct tgcgcagctg caccttgaag tagcgggaca ggccacgcca ctgctgggaa 4560

ccggaacggt tggtgaacat gccgcacacg tccacagcgg acaggtacag gttgtcgccc 4620

ttgcacaggg gaccgacacc gaactcgtcc agcagcacgg tggtagcggt gttggtgatg 4680

tgcagcacgg ggggcacgtt ctctccaccg gtcagggtgc cgaagtaacg ggtgttctcg 4740

ttacgggtgg ggtcgggcac ccagcactcc acggggtagg ccttgttctt gtccaggtaa 4800

gccttgtgct cggtgttcat cacttgggac tgcacagtag cgttcttggg gaagatggtg 4860

ccgtcggggt acttggtacg gtagttgaac accacgccct ggagttccag agcctcgcca 4920

cccacggaga agaagtggaa ggaggttccc tgcacgggct tgccagcacc gttgtcgtgg 4980

gtagcctggc cgttggagtg cacgttcatc agggaagtca cgccgatgac ctcggtcttg 5040

agggtcacag cctcccacat caggatgttg ccgcaagtca ggtcctcgtt caggttgggc 5100

agggggatac gagccacgga gtagcagggc agcatgtcac ggttggggga gtcggactcg 5160

aaggtgtcgg agatggagat ggacttggag aagcccctca ggtgctcgtc agggtcaccc 5220

atctcggggg tgaggaagca ctccacctcg gtaatggagt ccacgccggt cttgacttcc 5280

agcacctcga caccaccacg gatcagcagc ttgggcactt gcacggggtc cttgggctcg 5340

cccttgcgct tggtgggagc catggctcga gatcccgggt gatcaagtct tcgtcgagtg 5400

attgtaaata aaatgtaatt tacagtatag tattttaatt aatatacaaa tgatttgata 5460
ataattctta tttaactata atatattgtg ttgggttgaa ttaaaggtcc gtatactccg 5520
gaatattaat agatcatgga gataattaaa atgataacca tctcgcaaat aaataagtat 5580
tttactgttt tcgtaacagt tttgtaataa aaaaacctat aaatattccg gattattcat 5640
accgtcccac catcgggcgc ggatcccggt ccgaagcgcg cggaattccc acaaccatgg 5700
gagccgccct ggccctgctg ggagatctgg tggctacagt gtctgaagcc gccgctgcca 5760
ccggcttttc tgtggccgaa attgccgctg gcgaggctgc cgccacaatc gaggtggaaa 5820
ttgccagcct ggccaccgtg gaaggcatca ccagcacctc tgaagccatt gccgccatcg 5880
gcctgacccc cgagacatac gctgtgatca ctggcgctcc aggcgccgtg gctggatttg 5940
ctgctctggt gcagacagtg accggcggct ctgccattgc tcagctgggc tacagattct 6000
tcgccgactg ggaccacaag gtgtccaccg tgggcctgtt tcagcagcct gccatggctc 6060
tgcagctgtt caaccccgag gactactacg acatcctgtt ccccggcgtg aacgccttcg 6120
tgaacaacat ccactacctg gacccccggc actggggccc tagcctgttc tctacaatca 6180
gccaggcctt ctggaacctc gtgcgggacg atctgcctgc cctgaccagc caggaaatcc 6240
agcggcggac ccagaaactg ttcgtggaaa gcctggcccg gttcctggaa gagacaacct 6300
gggccatcgt gaacagcccc gccaacctgt acaactacat cagcgactac tacagcagac 6360
tgagccccgt gcggcccagc atggtgcgcc aggtggcaca gagagagggc acctatatca 6420
gcttcggcca ctcctacacc cagagcatcg acgacgccga cagcatccag gaagtgaccc 6480
agagactgga cctgaaaacc cccaacgtgc agagcggcga gttcatcgag agatccattg 6540
cccctggcgg agccaaccag agatctgccc ctcagtggat gctgcccctg ctgctgggcc 6600
tgtacggcac agtgacacca gccctggaag cctacgagga cggccccaac aagaagaagc 6660
gccggaaaga gggccctaga gccagcagca agaccagcta caagcggcgg agcagaagca 6720
gcagatccta cccatacgat gttccagatt acgcttgagc ggccgctttc gaatctagag 6780
cctgcagtct cgacaagctt gtcgagaagt actagaggat cataatcagc cataccacat 6840
ttgtagaggt tttacttgct ttaaaaaacc tcccacacct ccccctgaac ctgaaacata 6900
aaatgaatgc aattgttgtt gttaacttgt ttattgcagc ttataatggt tacaaataaa 6960
gcaatagcat cacaaatttc acaaataaag catttttttc actgcattct agttgtggtt 7020
tgtccaaact catcaatgta tcttatcatg tctggatctg atcactgctt gagcctagga 7080
gatccgaacc agataagtga aatctagttc caaactattt tgtcattttt aattttcgta 7140
ttagcttacg acgctacacc cagttcccat ctattttgtc actcttccct aaataatcct 7200
taaaaactcc atttccaccc ctcccagttc ccaactattt tgtccgccca cagcggggca 7260

tttttcttcc tgttatgttt ttaatcaaac atcctgccaa ctccatgtga caaaccgtca 7320

tcttcggcta cttt 7334

<210> 69
<211> 23
<212> DNA
<213> synthetic

<400> 69
cccagtcacg acgttgtaaa acg 23

<210> 70
<211> 23
<212> DNA
<213> synthetic

<400> 70
agcggataac aatttcacac agg 23

<210> 71
<211> 20
<212> DNA
<213> synthetic

<400> 71
tgacatgctg ccctgctact 20

<210> 72
<211> 21
<212> DNA
<213> synthetic

<400> 72
gcaagtcagg tcctcgttca g 21

<210> 73
<211> 20
<212> DNA
<213> synthetic

<400> 73
cttggcaatc cggtactgtt 20

<210> 74
<211> 20
<212> DNA
<213> synthetic

<400> 74
atatgtgcgt cggtaaaggc 20

<210> 75
<211> 18
<212> DNA
<213> synthetic

<400> 75
ctcgggaagc tggaaata 18

<210> 76
<211> 9
<212> PRT
<213> synthetic

<400> 76

Cys Gly Asn Lys Arg Thr Arg Gly Cys
1 5

<210> 77
<211> 6
<212> PRT
<213> synthetic

<400> 77

Lys Cys Cys Tyr Ser Leu
1 5

<210> 78
<211> 5
<212> PRT
<213> synthetic

<400> 78

Cys Arg Glu Lys Ala
1 5

<210> 79
<211> 80
<212> PRT
<213> Campylobacter spec.

<400> 79

Ile Asp Phe Asn Thr Trp Ala Ser Lys Asn Asn Lys His Phe Thr Ala
1 5 10 15

Ile Glu Lys Leu Arg Arg Arg Ala Ile Phe Asn Met Asn Ala Lys Phe
20 25 30

Val Asp Ser Phe Asn Lys Ile Gly Ser Phe Lys Leu Ser Val Asp Gly
35 40 45

Pro Phe Ala Ala Met Thr Asn Glu Glu Tyr Arg Thr Leu Leu Lys Ser
50 55 60

Lys Arg Thr Thr Glu Glu Asn Gly Gln Val Lys Tyr Leu Asn Ile Gln
65 70 75 80

<210> 80
<211> 145
<212> PRT
<213> Campylobacter spec.

<400> 80

```
Met Ile Lys Lys Ser Leu Asp Asn Leu Val Ile Met Ala Leu Val Phe
1               5                   10                  15

Ile Met Val Gly Cys Ser Thr Ala Pro Lys Pro Lys Glu Leu Asp Asp
            20                  25                  30

Asn Ser Ala Leu Ser Ile Asn Asn Ser Ile Leu Glu Lys Lys Tyr Ser
        35                  40                  45

Phe Val Pro Lys Asp Pro Tyr Leu Ser Gly Phe Asn Trp Thr Tyr His
    50                  55                  60

Ile Val Val Glu Lys Lys Thr Ile Asp Asp Asp Phe Ile Lys Asn Asp
65                  70                  75                  80

Leu Ile Thr Lys Thr Phe Leu Leu Ala His Asn Ser Thr Lys Ile Ile
                85                  90                  95

Leu Val Gly Arg Lys Asp Leu Ile Glu Gln Tyr Lys Gln Tyr Phe Glu
            100                 105                 110

Lys Asn Gln Val Leu Ala Pro Ile Glu Leu Gln Pro Val Asn Pro Ile
        115                 120                 125

Glu Arg Asp Phe Asn Lys Val Asn Ile Leu Phe Phe Asn Lys Thr Asn
    130                 135                 140

Phe
145
```

<210> 81
<211> 31
<212> PRT
<213> Homo sapiens (human)

<400> 81

```
Lys Asp Glu Pro Gln Arg Arg Ser Ala Arg Leu Ser Ala Lys Pro Ala
1               5                   10                  15

Pro Pro Lys Pro Glu Pro Lys Pro Lys Lys Ala Pro Ala Lys Lys
            20                  25                  30
```

<210> 82
<211> 12
<212> PRT
<213> synthetic

<400> 82

Cys Met Gly Thr Ile Asn Thr Arg Thr Lys Lys Cys
1 5 10

<210> 83
<211> 12
<212> PRT
<213> synthetic

<400> 83

Trp His Ser Asp Met Glu Trp Trp Tyr Leu Leu Gly
1 5 10

<210> 84
<211> 7
<212> PRT
<213> synthetic

<400> 84

Leu Thr Val Ser Pro Trp Tyr
1 5

<210> 85
<211> 12
<212> PRT
<213> synthetic

<400> 85

Trp Asn Leu Pro Trp Tyr Tyr Ser Val Ser Pro Thr
1 5 10

<210> 86
<211> 193
<212> PRT
<213> Homo sapiens (human)

<400> 86

Met Asp Lys Asp Cys Glu Met Lys Arg Thr Thr Leu Asp Ser Pro Leu
1 5 10 15

Gly Lys Leu Glu Leu Ser Gly Cys Glu Gln Gly Leu His Glu Ile Lys
20 25 30

Leu Leu Gly Lys Gly Thr Ser Ala Ala Asp Ala Val Glu Val Pro Ala
35 40 45

Pro Ala Ala Val Leu Gly Gly Pro Glu Pro Leu Met Gln Ala Thr Ala
    50                  55                  60

Trp Leu Asn Ala Tyr Phe His Gln Pro Glu Ala Ile Glu Glu Phe Pro
65                  70                  75                  80

Val Pro Ala Leu His His Pro Val Phe Gln Gln Glu Ser Phe Thr Arg
                85                  90                  95

Gln Val Leu Trp Lys Leu Leu Lys Val Val Lys Phe Gly Glu Val Ile
            100                 105                 110

Ser Tyr Gln Gln Leu Ala Ala Leu Ala Gly Asn Pro Ala Ala Thr Ala
        115                 120                 125

Ala Val Lys Thr Ala Leu Ser Gly Asn Pro Val Pro Ile Leu Ile Pro
    130                 135                 140

Cys His Arg Val Val Ser Ser Ser Gly Ala Val Gly Gly Tyr Glu Gly
145                 150                 155                 160

Gly Leu Ala Val Lys Glu Trp Leu Leu Ala His Glu Gly His Arg Leu
                165                 170                 175

Gly Lys Pro Gly Leu Gly Pro Ala Gly Gly Ser Pro Gly Leu Glu Val
            180                 185                 190

Asn

<210> 87
<211> 8
<212> PRT
<213> Homo sapiens (human)

<400> 87

Ser Leu Val Ser Phe Leu Thr Gly
1               5

<210> 88
<211> 106
<212> PRT
<213> Homo sapiens (human)

<400> 88

Glu Lys Pro Ser Pro Cys Gln Cys Ser Arg Leu Ser Pro His Asn Arg
1               5                   10                  15

Thr Asn Cys Gly Phe Pro Gly Ile Thr Ser Asp Gln Cys Phe Asp Asn
20 25 30

Gly Cys Cys Phe Asp Ser Ser Val Thr Gly Val Pro Trp Cys Phe His
35 40 45

Pro Leu Pro Lys Gln Glu Ser Asp Gln Cys Val Met Glu Val Ser Asp
50 55 60

Arg Arg Asn Cys Gly Tyr Pro Gly Ile Ser Pro Glu Glu Cys Ala Ser
65 70 75 80

Arg Lys Cys Cys Phe Ser Asn Phe Ile Phe Glu Val Pro Trp Cys Phe
85 90 95

Phe Pro Lys Ser Val Glu Asp Cys His Tyr
100 105

<210> 89
<211> 231
<212> PRT
<213> Coccidioides posadasii

<400> 89

Met His Leu Ala Ser Ile Leu Thr Cys Leu Leu Ala Ala Thr Val Ser
1 5 10 15

Val Ser Ala Ser Pro Gly Ser Glu Arg Thr Gln Lys Arg Pro Ser Leu
20 25 30

Ala Val Pro Arg Cys Pro Arg Lys Ala Thr Ala Ser Phe Asp Lys Ser
35 40 45

Val Pro Glu Met Lys Ala Phe Pro Asn Thr Gln Val Asp Leu Cys Trp
50 55 60

Glu Pro Thr Ala Phe Gln Phe Thr Phe Lys Ala Phe Asp Glu Thr Asn
65 70 75 80

Phe Tyr Phe Asp Pro Lys His Arg Thr Asn Asp Asp Ile Trp Lys Tyr
85 90 95

Glu Val Met Glu Ala Phe Ile Tyr His Gly Thr Asn Asp Pro Gln Thr
100 105 110

Tyr Phe Glu Phe Glu Val Ser Pro Asn Asn Val Thr Tyr Gln Thr Phe
115 120 125

```
Val Tyr Asn Pro Ser Lys Val Arg Lys Glu Gly Ala Pro Phe Asp His
    130                 135                 140

Phe Phe Val Ser Asp Pro Ala Ala Asp Gly Phe Thr Ser Ile Thr Thr
145                 150                 155                 160

Leu Asp Arg Lys Ala Gln Thr Trp Val Ser Glu Val Lys Ile Pro Leu
                165                 170                 175

Ala Leu Phe Asn Val Asp Arg Pro Arg Leu Ser Arg Trp Arg Met Asn
            180                 185                 190

Phe Phe Arg Thr Val Thr Ser Pro Ala Thr Tyr Pro Asn Gln Glu Leu
        195                 200                 205

Gly Ala Trp Asn Ser Pro Asp Val Ala Ser Phe His Val Thr Pro Phe
    210                 215                 220

Phe Gly Asp Val Ile Leu Val
225                 230
```